(19) Europäisches Patentamt
European Patent Office
Office européen des brevets

(11) **EP 2 594 588 B1**

(12) **EUROPEAN PATENT SPECIFICATION**

(45) Date of publication and mention
of the grant of the patent:
**21.05.2014 Bulletin 2014/21**

(51) Int Cl.:
*C07K 16/26* (2006.01)     *A61K 39/395* (2006.01)
*A61P 31/00* (2006.01)     *A61P 9/00* (2006.01)
*A61P 13/12* (2006.01)

(21) Application number: **12193121.6**

(22) Date of filing: **16.11.2012**

(54) **Anti-Adrenomedullin (ADM) antibody or anti-ADM antibody fragment or an anti-ADM non-Ig protein scaffold for use in therapy**

Anti-Adrenomedullin-Antikörper (ADM) oder Anti-ADM-Antikörperfragment oder Anti-ADM-non-Ig Proteingerüst zur Verwendung bei der Behandlung

Anticorps anti-adrénomédulline (ADM) ou fragment d'anticorps anti-ADM ou échafaudage protéique non-Ig anti-ADM destinés à être utilisés en thérapie

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.11.2011 EP 11189448**
**16.03.2012 EP 12160018**

(43) Date of publication of application:
**22.05.2013 Bulletin 2013/21**

(73) Proprietor: **AdrenoMed AG**
**16761 Hennigsdorf (DE)**

(72) Inventor: **Bergmann, Andreas**
**13465 Berlin (DE)**

(74) Representative: **Kilger, Ute**
**Boehmert & Boehmert**
**Pettenkoferstrasse 20-22**
**80336 München (DE)**

(56) References cited:
**EP-A2- 0 622 458**

• **PING WANG ET AL: "The Pivotal role of adrenomedullin in producing hyperdynamic circulation during early stage of sepsis", ARCHIVES OF SURGERY, AMERICAN MEDICAL ASSOCIATION, CHICAGO, IL, US, vol. 133, 1 December 1998 (1998-12-01), pages 1298-1304, XP002599345, ISSN: 0004-0010**

• **DUC QUYEN CHU ET AL: "The calcitonin gene-related peptide (CGRP) antagonist CGRP8-37 blocks vasodilatation in inflamed rat skin: involvement of adrenomedullin in addition to CGRP", NEUROSCIENCE LETTERS, vol. 310, no. 2-3, 1 September 2001 (2001-09-01), pages 169-172, XP055051751, ISSN: 0304-3940, DOI: 10.1016/S0304-3940(01)02132-2**

• **OUAFIK L'HOUCINE ET AL: "Neutralization of adrenomedullin inhibits the growth of human glioblastoma cell lines in vitro and suppresses tumor xenograft growth in vivo", AMERICAN JOURNAL OF PATHOLOGY; [10640], AMERICAN SOCIETY FOR INVESTIGATIVE PATHOLOGY, US, vol. 160, no. 4, 1 April 2002 (2002-04-01) , pages 1279-1292, XP002421261, ISSN: 0002-9440**

• **MARTINEZ A ET AL: "IS ADRENOMEDULLIN A CAUSAL AGENT IN SOME CASES OF TYPE 2 DIABETES?", PEPTIDES, ELSEVIER, AMSTERDAM, vol. 20, no. 12, 1 December 1999 (1999-12-01), pages 1471-1478, XP000982202, ISSN: 0196-9781, DOI: 10.1016/S0196-9781(99) 00158-8**

• **HYVELIN J ET AL: "Adrenomedullin: A Cardiac Depressant Factor in Septic Shock", JOURNAL OF CARDIAC SURGERY, FUTURA PUBL., MOUNT KISCO, NY, US, vol. 17, no. 4, 1 January 2002 (2002-01-01), pages 328-335, XP007915796, ISSN: 0886-0440 [retrieved on 2010-07-12]**

• **PING WANG: "Andrenomedullin and cardiovascular responses in sepsis", PEPTIDES, vol. 22, no. 11, 1 November 2001 (2001-11-01), pages 1835-1840, XP055022163, ISSN: 0196-9781, DOI: 10.1016/S0196-9781(01)00534-4**

- **PIO R ET AL:** "Complement Factor H Is a Serumbinding Protein for Adrenomedullin, and the Resulting Complex Modulates the Bioactivities of Both Partners", JOURNAL OF BIOLOGICAL CHEMISTRY, AMERICAN SOCIETY FOR BIOCHEMISTRY AND MOLECULAR BIOLOGY, US, vol. 276, no. 15, 1 April 2001 (2001-04-01), pages 12292-12300, XP009119203, ISSN: 0021-9258, DOI: 10.1074/JBC.M007822200 [retrieved on 2000-12-14]
- **STRUCK JOACHIM ET AL:** "Method for the Selective Measurement of Amino-Terminal Variants of Procalcitonin", CLINICAL CHEMISTRY, vol. 55, no. 9, September 2009 (2009-09), pages 1672-1679, ISSN: 0009-9147
- **MOODY T W ET AL:** 'Adrenomedullin binds with high affinity, elevates cyclic AMP, and stimulates c-fos mRNA in C6 glioma cells' PEPTIDES 1997 vol. 18, no. 8, 1997, pages 1111 - 1115 ISSN: 0196-9781
- **GEBAUER M ET AL:** "Engineered protein scaffolds as next-generation antibody therapeutics", CURRENT OPINION IN CHEMICAL BIOLOGY, CURRENT BIOLOGY LTD, LONDON, GB, vol. 13, no. 3, 1 June 2009 (2009-06-01), pages 245-255, XP026285197, ISSN: 1367-5931, DOI: 10.1016/J.CBPA.2009.04.627 [retrieved on 2009-06-06]
- **MILLER MAE JEAN ET AL:** "Adrenomedullin expression in human tumor cell lines: Its potential role as an autocrine growth factor", JOURNAL OF BIOLOGICAL CHEMISTRY, AMERICAN SOCIETY FOR BIOCHEMISTRY AND MOLECULAR BIOLOGY, US, vol. 271, no. 38, 1 September 1996 (1996-09-01), pages 23345-23351, XP002184272, ISSN: 0021-9258, DOI: 10.1074/JBC.271.38.23345
- **WURCH THIERRY ET AL:** "Novel protein scaffolds as emerging therapeutic proteins: from discovery to clinical proof-of-concept", TRENDS IN BIOTECHNOLOGY, vol. 30, no. 11, November 2012 (2012-11), pages 575-582, ISSN: 0167-7799

## Description

### Field of the invention

Subject matter of the present disclosure is an anti-adrenomedullin antibody or an anti-adrenomedullin antibody fragment or an anti-ADM non-Ig protein scaffold, wherein said antibody or fragment or scaffold is a non-neutralizing antibody.

Subject matter of the present disclosure is an anti-adrenomedullin antibody or an anti-adrenomedullin antibody fragment or an anti-ADM non-Ig protein scaffold, wherein said antibody or fragment or scaffold is

- an ADM stabilizing antibody or an ADM stabilizing antibody fragment or an ADM stabilizing non-Ig protein scaffold that enhances the half life ($t_{1/2}$ half retention time) of adrenomedullin in serum, blood, plasma at least 10 %, preferably at least, 50 %, more preferably >50 %, most preferably 100 % and/or

- wherein said ADM stabilizing antibody or an adrenomedullin stabilizing antibody fragment or an ADM stabilizing non-Ig protein scaffold blocks the bioactivity of ADM to less than 80 %, preferably to less than 50 %.

The stated above is in the sense of blocking the ADM bioactivity of not more than 80 % or not more than 50 %, respectively, and thus is to be understood as limited blocking of ADM bioactivity or reduction of ADM bioactivity by the respective ADM binder, being it an ADM stabilizing antibody or antibody fragment or non-Ig protein scaffold. By implication, this means that ADM bioactivity is remaining. For instance, in case of blocking the ADM bioactivity of not more than 80 %, *i.e.* approximately 20 % residual ADM bioactivity present. In case of blocking the ADM bioactivity of not more than 50 %, *i.e.* approximately 50 % residual ADM bioactivity present.

Subject matter of the present invention is an anti-adrenomedullin antibody or an anti-adrenomedullin antibody fragment or an anti-ADM non-Ig protein scaffold, wherein said antibody or fragment or scaffold binds to the N-terminal part (aa 1-21) of human adrenomedullin.

In a preferred embodiment the subject matter of the present invention is an anti-adrenomedullin antibody or an anti-adrenomedullin antibody fragment or an anti-ADM non-Ig protein scaffold, wherein said antibody or fragment or scaffold binds to the N-terminal end (aa 1) of human adrenomedullin.

### Background

The peptide adrenomedullin (ADM) was described for the first time in 1993 (Kitamura, K., et al., "Adrenomedullin: A Novel Hypotensive Peptide Isolated From Human Pheochromocytoma", Biochemical and Biophysical Research Communications, Vol. 192 (2), pp. 553-560 (1993)) as a novel hypotensive peptide comprising 52 amino acids, which had been isolated from a human pheochromocytome; SEQ ID No.: 21. In the same year, cDNA coding for a precursor peptide comprising 185 amino acids and the complete amino acid sequence of this precursor peptide were also described. The precursor peptide, which comprises, inter alia, a signal sequence of 21 amino acids at the N-terminus, is referred to as "preproadrenomedullin" (pre-proADM). In the present description, all amino acid positions specified usually relate to the pre-proADM which comprises the 185 amino acids. The peptide adrenomedullin (ADM) is a peptide which comprises 52 amino acids (SEQ ID NO: 21) and which comprises the amino acids 95 to 146 of pre-proADM, from which it is formed by proteolytic cleavage. To date, substantially only a few fragments of the peptide fragments formed in the cleavage of the pre-proADM have been more exactly characterized, in particular the physiologically active peptides adrenomedullin (ADM) and "PAMP", a peptide comprising 20 amino acids (22-41) which follows the 21 amino acids of the signal peptide in pre-proADM. The discovery and characterization of ADM in 1993 triggered intensive research activity, the results of which have been summarized in various review articles, in the context of the present description, reference being made in particular to the articles to be found in an issue of "Peptides" devoted to ADM in particular (Editorial, Takahashi, K., "Adrenomedullin: from a pheochromocytoma to the eyes", Peptides, Vol. 22, p. 1691 (2001)) and (Eto, T., "A review of the biological properties and clinical implications of adrenomedullin and proadrenomedullin N-terminal 20 peptide (PAMP), hypotensive and vasodilating peptides", Peptides, Vol. 22, pp. 1693-1711 (2001)). A further review is (Hinson, et al., "Adrenomedullin, a Multifunctional Regulatory Peptide", Endocrine Reviews, Vol. 21(2), pp. 138-167 (2000)). In the scientific investigations to date, it has been found, inter alia, that ADM may be regarded as a polyfunctional regulatory peptide. It is released into the circulation in an inactive form extended by glycine (Kitamura, K., et al., "The intermediate form of glycine-extended adrenomedullin is the major circulating molecular form in human plasma", Biochem. Biophys. Res. Commun., Vol. 244(2), pp. 551-555 (1998). Abstract Only). There is also a binding protein (Pio, R., et al., "Complement Factor H is a Serum-binding Protein for adrenomedullin, and the Resulting Complex Modulates the Bioactivities of Both Partners", The Journal of Biological Chemistry, Vol. 276(15), pp. 12292-12300 (2001)) which is specific for ADM and probably likewise modulates the effect of ADM. Those physiological effects of ADM as well as of PAMP which are of primary importance in the investigations to date were the effects influencing blood pressure.

Hence, ADM is an effective vasodilator, and thus it is possible to associate the hypotensive effect with the particular peptide segments in the C-terminal part of ADM. It has furthermore been found that the above-mentioned further physiologically active peptide PAMP formed from pre-proADM likewise exhibits a hypotensive effect, even if it appears to have an action mechanism differing from that of ADM (cf. in addition to the abovementioned review articles (Eto, T., "A review of the biological properties and clinical implications of adrenomedullin and proadrenomedullin N-terminal 20 peptide (PAMP), hypotensive and vasodilating peptides", Peptides, Vol. 22, pp. 1693-1711 (2001)) and (Hinson, et al., "Adrenomedullin, a Mul-tifunctional Regulatory Peptide", Endocrine Reviews, Vol. 21(2), pp. 138-167 (2000)) also (Kuwasako, K., et al., "Purification and characterization of PAMP-12 (PAMP-20) in porcine adrenal medulla as a major endogenous biologically active peptide", FEBS Lett, Vol. 414(1), pp. 105-110 (1997). Abstract only), (Kuwasaki, K., et al., "Increased plasma proadrenomedullin N-terminal 20 peptide in patients with essential hypertension", Ann. Clin. Biochem., Vol. 36 (Pt. 5), pp. 622-628 (1999). Abstract only) or (Tsuruda, T., et al., "Secretion of proadrenomedullin N-terminal20 peptide from cultured neonatal rat cardiac cells", Life Sci., Vol. 69(2), pp. 239-245 (2001). Abstract only) and EP-A2 0 622 458). It has furthermore been found that the concentrations of ADM which can be measured in the circulation and other biological liquids are, in a number of pathological states, significantly above the concentrations to be found in healthy control persons. Thus, the ADM level in patients with congestive heart failure, myocardial infarction, kidney diseases, hypertensive disorders, Diabetes mellitus, in the acute phase of shock and in sepsis and septic shock are significantly increased, although to different extents. The PAMP concentrations are also increased in some of said pathological states, but the plasma levels are lower relative to ADM ((Eto, T., "A review of the biological properties and clinical implications of adrenomedullin and proadrenomedullin N-terminal 20 peptide (PAMP), hypotensive and vasodilating peptides", Peptides, Vol. 22, pp. 1693-1711 (2001)); page 1702). It is furthermore known that unusually high concentrations of ADM are to be observed in sepsis, and the highest concentrations in septic shock (cf. (Eto, T., "A review of the biological properties and clinical implications of adrenomedullin and proadrenomedullin N-terminal 20 peptide (PAMP), hypotensive and vasodilating peptides", Peptides, Vol. 22, pp. 1693-1711 (2001)) and (Hirata, et al., "Increased Circulating Adreno-medullin, a Novel Vasodilatory Peptide, in Sepsis", Journal of Clinical Endocrinology and Metabolism, Vol. 81(4), pp. 1449-1453 (1996)), (Ehlenz, K., et al., "High levels of circulating adrenomedullin in severe illness: Correlation with C-reactive protein and evidence against the adrenal medulla as site of origin", Exp Clin Endocrinol Diabetes, Vol. 105, pp. 156-162 (1997)), (Tomoda, Y., et al., "Regulation of adrenomedullin secretion from cultured cells", Peptides, Vol. 22, pp. 1783-1794 (2001)), (Ueda, S., et al., "Increased Plasma Levels of Adrenomedullin in Patients with Systemic Inflammatory Response Syndrome", Am. J. Respir. Crit. Care Med., Vol. 160, pp. 132-136 (1999)) and (Wang, P., "Adrenomedullin and cardiovascular responses in sepsis", Peptides, Vol. 22, pp. 1835-1840 (2001)).

Known in the art is further a method for identifying adrenomedullin immunoreactivity in biological liquids for diagnostic purposes and, in particular within the scope of sepsis diagnosis, cardiac diagnosis and cancer diagnosis. According to the invention, the midregional partial peptide of the proadrenomedullin, which contains amino acids (45-92) of the entire preproadrenomedullin, is measured, in particular, with an immunoassay which works with at least one labeled antibody that specifically recognizes a sequence of the mid-proADM (WO2004/090546).

WO-A1 2004/097423 describes the use of an antibody against adrenomedullin for diagnosis, prognosis, and treatment of cardiovascular disorders. Treatment of diseases by blocking the ADM receptor are also described in the art, (e.g. WO-A1 2006/027147, WO/2007/062676 ) said diseases may be sepsis, septic shock, cardiovascular diseases, infections, dermatological diseases, endocrinological diseases, metabolic diseases, gastroenterological diseases, cancer, inflammation, hematological diseases, respiratory diseases, muscle skeleton diseases, neurological diseases, urological diseases.

It is reported for the early phase of sepsis that ADM improves heart function and the blood supply in liver, spleen, kidney and small intestine. ADM-neutralizing antibodies neutralize the before mentioned effects during the early phase of sepsis (Wang, P., "Adrenomedullin and cardiovascular responses in sepsis", Peptides, Vol. 22, pp. 1835-1840 (2001).

In the later phase of sepsis, the hypodynamical phase of sepsis, ADM constitutes a risk factor that is strongly associated with the mortality of patients in septic shock. (Schütz et al., "Circulating Precursor levels of endothelin-1 and adrenomedullin, two endothelium-derived, counteracting substances, in sepsis", Endothelium, 14:345-351, (2007)). Methods for the diagnosis and treatment of critically ill patients, *e.g.* in the very late phases of sepsis, and the use of endothelin and endothelin agonists with vasoconstrictor activity for the preparation of medicaments for the treatment of critically ill patients have been described in WO-A1 2007/062676. It is further described in WO-A1 2007/062676 to use, in place of endothelin and/or endothelin agonists, or in combination therewith, adrenomedullin antagonists, *i.e.* molecules which prevent or attenuate the vasodilating action of adrenomedulin, *e.g.* by blocking its relevant receptors, or substances preventing the binding of adrenomedullin to its receptor (*e.g.* specific binders as *e.g.* antibodies binding to adrenomedullin and blocking its receptor bindings sites; "immunological neutralization"). Such use, or combined use, including a subsequent or preceding separate use, has been described in certain cases to be desirable for example to improve the therapeutic success, or to avoid undesirable physiological stress or side effects. Thus, it is reported that neutralizing ADM antibodies may be used for the treatment of sepsis in the late stage of sepsis.

Administration of ADM in combination with ADM-binding-Protein-1 is described for treatment of sepsis and septic shock in the art. It is assumed that treatment of septic animals with ADM and ADM-binding-Protein-1 prevents transition to the late phase of sepsis. It has to be noted that in a living organism ADM binding protein (complement factor H) is present in the circulation of said organism in high concentrations (Pio et al.: Identification, characterization, and physiological actions of factor H as an Adrenomedullin binding Protein present in Human Plasma; Microscopy Res. and Technique, 55:23-27 (2002) and Martinez et al.; Mapping of the Adrenomedullin-Binding domains in Human Complement factor H; Hypertens Res Vol. 26, Suppl (2003), S56-59).

In accordance with the invention the ADM-binding-protein-1 may also be referred to as ADM-binding-protein-1 (complement factor H).

Administration of ADM may thus be beneficial in an early stage of a disease like e.g. sepsis but may be detrimental in a later stage of sepsis. The opposite may be the case with the administration of anti-ADM antibodies. Further, dosing may be critical when applying ADM or anti-ADM antibodies.

For other diseases blocking of ADM may be beneficial to a certain extent. However, it might also be detrimental if ADM is totally neutralized as a certain amount of ADM may be required for several physiological functions. In many reports it was emphasized that the administration of ADM may be beneficial in certain diseases. In contrast thereto in other reports ADM was reported as being life threatening when administered in certain conditions. The disadvantage of the prior art may be overcome with the anti-ADM antibodies or anti-ADM antibody fragments or anti-ADM non-Ig protein scaffolds provided by the present invention.

**Description of the invention**

Subject matter of the invention is an anti-adrenomedullin antibody or an anti-ADM antibody fragment binding to adrenomedullin or an anti-ADM non-Ig protein scaffold binding to adrenomedullin for use as a medicament, wherein said antibody or said fragment or said scaffold is a non-neutralizing antibody or fragment or scaffold that is said antibody or fragment or scaffold binds to the N-terminal part, aa-21, of adrenomedullin:

YRQSMNNFQGLRSFGCRFGTC; SEQ ID No. 23.

Throughout the specification the "antibodies", or "antibody fragments" or "non-Ig protein scaffolds" in accordance with the invention are capable to bind ADM, and thus are directed against ADM, and thus can be referred to as "anti-ADM antibodies", "anti-ADM antibody fragments", or "anti-ADM non-Ig protein scaffolds".

In another embodiment of the invention the anti-ADM antibodies, anti-ADM antibody fragments, or anti-ADM non-Ig protein scaffolds provided by the invention are capable to bind circulating ADM, and thus are directed against circulating ADM.

In a specific embodiment said anti-ADM antibody, anti-ADM antibody fragment or anti-ADM non-Ig protein scaffold is a non-neutralizing antibody, fragment or non-Ig protein scaffold. A neutralizing anti-ADM antibody, anti-ADM antibody fragment or anti-ADM non-Ig protein scaffold would block the bioactivity of ADM to nearly 100%, to at least more than 90%, preferably to at least more than 95%.

In contrast, a non-neutralizing anti-ADM antibody, or anti-ADM antibody fragment or anti-ADM non-Ig protein scaffold blocks the bioactivity of ADM less than 100%, preferably to less than 95%, preferably to less than 90%, more preferred to less than 80% and even more preferred to less than 50 % (baseline value). This means that the residual bioactivity of ADM bound to the non-neutralizing anti-ADM antibody, or anti-ADM antibody fragment or anti-ADM non-Ig protein scaffold would be more than 0%, preferably more than 5 %, preferably more than 10 % , more preferred more than 20 %, more preferred more than 50 %. This is in the sense of blocking the ADM bioactivity of not more than 80 % or not more than 50 %, respectively, and thus is to be understood as limited blocking of ADM bioactivity by the respective ADM binder, being it an anti-ADM antibody or anti-ADM antibody fragment or anti-ADM non-Ig protein scaffold.

It has been understood that said limited blocking of the bioactivity of ADM occurs even at excess concentration of the antibody, antibody fragment or non-Ig protein scaffold, meaning an excess of the antibody, fragment or scaffold in relation to ADM. Said limited blocking is an intrinsic property of the ADM binder itself. This means that said antibody, fragment or scaffold has a maximal inhibition of 80% or 50%, respectively. In a preferred embodiment said anti-ADM antibody, anti-ADM antibody fragment or anti-ADM non-Ig protein scaffold would block the bioactivity of ADM to at least 5 %. This means - by implication - approximately 95% residual ADM bioactivity is present.

Subject matter of the present disclosure is an adrenomedullin antibody or an adrenomedullin antibody fragment for use in a treatment of a chronic or acute disease wherein said antibody or fragment is

- an ADM stabilizing antibody or an adrenomedullin antibody fragment that enhances the $t_{1/2}$ half retention time of adrenomedullin in serum, blood, plasma at least 10 %, preferably at least, 50 %, more preferably >50 %, most preferably 100 % and/or

• wherein said antibody or said adrenomedullin antibody fragment blocks the bioactivity of ADM to less than 80 %, preferably to less than 50 %.

Subject matter of the present invention is an adrenomedullin antibody or an adrenomedullin antibody fragment for use in a treatment of a chronic or acute disease wherein said antibody or fragment binds to the N-terminal part (aa 1-21) of human adrenomedullin.

In a preferred embodiment the subject matter of the present invention is an adrenomedullin antibody or an adrenomedullin antibody fragment for use in a treatment of a chronic or acute disease wherein said antibody or fragment binds to the N-terminal end of human adrenomedullin.

In this context (a) molecule(s), being it an antibody, or an antibody fragment or a non-Ig protein scaffold with "non-neutralizing anti-ADM activity", collectively termed here for simplicity as "non-neutralizing" anti-ADM antibody, antibody fragment, or non-Ig protein scaffold, that e.g. blocks the bioactivity of ADM to less than 80 %, is defined as

- a molecule or molecules binding to ADM, which upon addition to a culture of an eukaryotic cell line, which expresses functional human recombinant ADM receptor composed of CRLR (calcitonin receptor like receptor) and RAMP3 (receptor-activity modifying protein 3), reduces the amount of cAMP produced by the cell line through the action of parallel added human synthetic ADM peptide, wherein said added human synthetic ADM is added in an amount that in the absence of the non-neutralizing antibody to be analyzed, leads to half-maximal stimulation of cAMP synthesis, wherein the reduction of cAMP by said molecule(s) binding to ADM takes place to an extent, which is not more than 80%, even when the non-neutralizing molecule(s) binding to ADM to be analyzed is added in an amount, which is 10-fold more than the amount, which is needed to obtain the maximal reduction of cAMP synthesis obtainable with the non-neutralizing antibody to be analyzed.

The same definition applies to the other ranges; 95%, 90%, 50% etc.

The bioactivity is defined as the effect that a substance takes on a living organism or tissue or organ or functional unit in vivo or in vitro (e.g. in an assay) after its interaction. In case of ADM bioactivity this may be the effect of ADM in a human recombinant Adrenomedullin receptor cAMP functional assay. Thus, according to the present invention bioactivity is defined via an Adrenomedullin receptor cAMP functional assay. The following steps may be performed in order to determine the bioactivity of ADM in such an assay:

- Dose response curves are performed with ADM in said human recombinant Adrenomedullin receptor cAMP functional assay.
- The ADM-concentration of half-maximal cAMP stimulation may be calculated.
- At constant half-maximal cAMP-stimulating ADM-concentrations dose response curves (up to 100μg/ml final concentration) are performed by an ADM stabilizing antibody or an adrenomedullin stabilizing antibody fragment or an adrenomedullin stabilizing non-Ig protein scaffold, respectively.

A maximal inhibition in said ADM bioassay of 50% means that said anti-ADM antibody or said anti-adrenomedullin antibody fragment or said anti-adrenomedullin non-Ig protein scaffold, respectively, blocks the bioactivity to 50% of baseline values. A maximal inhibition in said ADM bioassay of 80% means that said anti-ADM antibody or said anti-adrenomedullin antibody fragment or said anti-adrenomedullin non-Ig protein scaffold, respectively, blocks the bioactivity of ADM to 80%. This is in the sense of blocking the ADM bioactivity to not more than 80%. This means approximately 20% residual ADM bioactivity remains present.

However, by the present specification and in the above context the expression "blocks the bioactivity of ADM" in relation to the herein disclosed anti-ADM antibodies, anti-ADM antibody fragments, and anti-ADM non-Ig protein scaffolds should be understood as mere decreasing the bioactivity of ADM, preferably decreasing circulating ADM bioactivity from 100% to 20% remaining ADM bioactivity at maximum, preferably decreasing the ADM bioactivity from 100% to 50% remaining ADM bioactivity; but in any case there is ADM bioactivity remaining that can be determined as detailed above.

The bioactivity of ADM may be determined in a human recombinant Adrenomedullin receptor cAMP functional assay (Adrenomedullin Bioassay) according to Example 2.

Anti-adrenomedullin (ADM) antibody is an antibody that binds specifically to ADM, anti-adrenomedullin antibody fragment is a fragment of an ADM antibody wherein said fragment binds specifically to ADM. An anti-ADM non-Ig protein scaffold is a non-Ig protein scaffold that binds specifically to ADM. Specifically binding to ADM allows binding to other antigens as well. This means, this specificity would not exclude that the antibody may cross-react with other polypeptides than that against it has been raised. This also pertains the specificity of the anti-ADM antibody fragment, or anti-ADM non-Ig protein scaffold in accordance with the invention.

Surprisingly it has turned out that non-neutralizing anti-ADM antibodies or non-neutralizing anti-ADM antibody fragments or a non-neutralizing anti-ADM non-Ig protein scaffolds offer a significant therapeutic advantage over the neutralizing ones.

In one specific embodiment it is preferred to use an anti-ADM antibody or an anti-adrenomedullin antibody fragment or anti-ADM non-Ig protein scaffold according to the present invention, wherein said anti-adrenomedullin antibody or said anti-adrenomedullin antibody fragment or anti-ADM non-Ig protein scaffold is an ADM stabilizing antibody or an adrenomedullin stabilizing antibody fragment or an adrenomedullin stabilizing non-Ig protein scaffold that enhances the half life ($t_{1/2}$; half retention time) of adrenomedullin in serum, blood, plasma at least 10 %, preferably at least 50 %, more preferably >50 %, most preferably >100%.

The half life (half retention time) of ADM may be determined in human plasma in absence and presence of an ADM stabilizing antibody or an adrenomedullin stabilizing antibody fragment or an adrenomedullin stabilizing non-Ig protein scaffold, respectively, using an immunoassay for the quantification of ADM.

The following steps may be conducted:

- ADM may be diluted in human citrate plasma in absence and presence of an ADM stabilizing antibody or an adrenomedullin stabilizing antibody fragment or an adrenomedullin stabilizing non-Ig protein scaffold, respectively, and may be incubated at 24 °C.
- Aliquots are taken at selected time points (e.g. within 24 hours) and degradation of ADM may be stopped in said aliquots by freezing at -20 °C.
- The quantity of ADM may be determined by an hADM immunoassay directly, if the selected assay is not influenced by the stabilizing antibody. Alternatively, the aliquot may be treated with denaturing agents (like HCl) and, after clearing the sample (e.g. by centrifugation) the pH can be neutralized and the ADM-quantified by an ADM immunoassay. Alternatively, non-immunoassay technologies (e.g. rpHPLC) can be used for ADM-quantification.
- The half life of ADM is calculated for ADM incubated in absence and presence of an ADM stabilizing antibody or an adrenomedullin stabilizing antibody fragment or an adrenomedullin stabilizing non-Ig protein scaffold, respectively,
- The enhancement of half life is calculated for the stabilized ADM in comparison to ADM that has been incubated in absence of an ADM stabilizing antibody or an adrenomedullin stabilizing antibody fragment or an adrenomedullin stabilizing non-Ig protein scaffold.

A two-fold increase of the half life of ADM is an enhancement of half life of 100%.

Half Life (half retention time) is defined as the period over which the concentration of a specified chemical or drug takes to fall to half its baseline concentration in the specified fluid or blood.

An assay that may be used for the determination of the Half life (half retention time) of adrenomedullin in serum, blood, plasma is described in Example 3.

An antibody according to the present invention is a protein including one or more polypeptides substantially encoded by immunoglobulin genes that specifically binds an antigen. The recognized immunoglobulin genes include the kappa, lambda, alpha (IgA), gamma ($IgG_1$, $IgG_2$, $IgG_3$, $IgG_4$), delta (IgD), epsilon (IgE) and mu (IgM) constant region genes, as well as the myriad immunoglobulin variable region genes. Full-length immunoglobulin light chains are generally about 25 Kd or 214 amino acids in length. Full-length immunoglobulin heavy chains are generally about 50 Kd or 446 amino acid in length. Light chains are encoded by a variable region gene at the NH2-terminus (about 110 amino acids in length) and a kappa or lambda constant region gene at the COOH-terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and F(ab')$_2$, as well as bifunctional hybrid antibodies and single chains (*e.g.*, Lanzavecchia et al., Eur. J. Immunol. 17:105,1987; Huston et al., Proc. Natl. Acad. Sci. U.S.A., 85:5879-5883, 1988; Bird et al., Science 242:423-426, 1988; Hood et al., Immunology, Benjamin, N.Y., 2nd ed., 1984; Hunkapiller and Hood, Nature 323:15-16,1986). An immunoglobulin light or heavy chain variable region includes a framework region interrupted by three hypervariable regions, also called complementarity determining regions (CDR's) (see, Sequences of Proteins of Immunological Interest, E. Kabat et al., U.S. Department of Health and Human Services, 1983). As noted above, the CDRs are primarily responsible for binding to an epitope of an antigen. An immune complex is an antibody, such as a monoclonal antibody, chimeric antibody, humanized antibody or human antibody, or functional antibody fragment, specifically bound to the antigen.

Chimeric antibodies are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. In one example, a therapeutic chimeric antibody is thus a hybrid protein composed of the variable or antigen-binding domain from a mouse antibody and the constant or effector domain from a human antibody, although other mammalian species can be used, or the variable region can be produced by molecular techniques. Methods of making chimeric antibodies are well known in the art, *e.g.*, see U.S. Patent No. 5,807,715. A "humanized" immunoglobulin is an immunoglobulin including a human framework region and one or more CDRs from a non-human (such as a mouse, rat, or synthetic) immunoglobulin. The non-human immunoglobulin providing the CDRs is termed a "donor" and the human immunoglobulin providing the framework is termed an "acceptor." In one embodiment, all the CDRs are from the donor immunoglobulin in a humanized immunoglobulin. Constant regions need not be present, but if they are, they must be substantially identical to human immunoglobulin constant regions, *i.e.*, at least about 85-90%, such as about 95% or more identical. Hence, all parts of a humanized immunoglobulin, except possibly the CDRs, are substantially identical to corresponding parts of natural human immunoglobulin sequences. A "humanized antibody" is an antibody comprising a humanized light chain and a humanized heavy chain immunoglobulin. A humanized antibody binds to the same antigen as the donor antibody that provides the CDRs. The acceptor framework of a humanized immunoglobulin or antibody may have a limited number of substitutions by amino acids taken from the donor framework. Humanized or other monoclonal antibodies can have additional conservative amino acid substitutions which have substantially no effect on antigen binding or other immunoglobulin functions. Exemplary conservative substitutions are those such as gly, ala; val, ile, leu; asp, glu; asn, gln; ser, thr; lys, arg; and phe, tyr. Humanized immunoglobulins can be constructed by means of genetic engineering (*e.g.*, see U.S. Patent No. 5,585,089). A human antibody is an antibody wherein the light and heavy chain genes are of human origin. Human antibodies can be generated using methods known in the art. Human antibodies can be produced by immortalizing a human B cell secreting the antibody of interest. Immortalization can be accomplished, for example, by EBV infection or by fusing a human B cell with a myeloma or hybridoma cell to produce a trioma cell. Human antibodies can also be produced by phage display methods (see, *e.g.*, Dower et al., PCT Publication No. WO91/17271; McCafferty et al., PCT Publication No. WO92/001047; and Winter, PCT Publication No. WO92/20791), or selected from a human combinatorial monoclonal antibody library (see the Morphosys website). Human antibodies can also be prepared by using transgenic animals carrying a human immunoglobulin gene (for example, see Lonberg et al., PCT Publication No. WO93/12227; and Kucherlapati, PCT Publication No. WO91/10741).

Thus, the anti-ADM antibody may have the formats known in the art. Examples are human antibodies, monoclonal antibodies, humanized antibodies, chimeric antibodies, CDR-grafted antibodies. In a preferred embodiment antibodies according to the present invention are recombinantly produced antibodies as *e.g.* IgG, a typical full-length immunoglobulin, or antibody fragments containing at least the F-variable domain of heavy and/or light chain as *e.g.* chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (miniantibody) dimerized with the CH3 domain; bivalent Fab or multivalent Fab, *e.g.* formed via multimerization with the aid of a heterologous domain, *e.g.* via dimerization of dHLX domains, *e.g.* Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, *e.g.* from a different class than G; single-domain antibodies, *e.g.* nanobodies derived from camelid or fish immunoglobulines and numerous others.

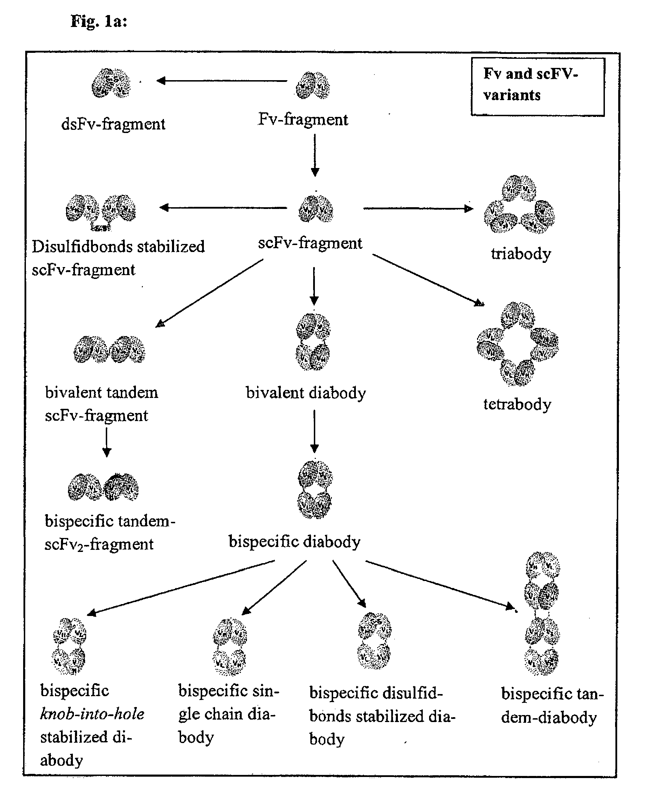

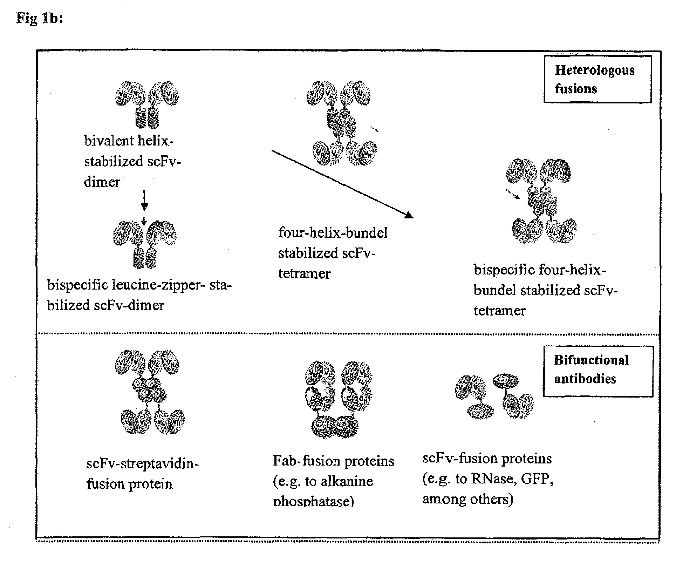

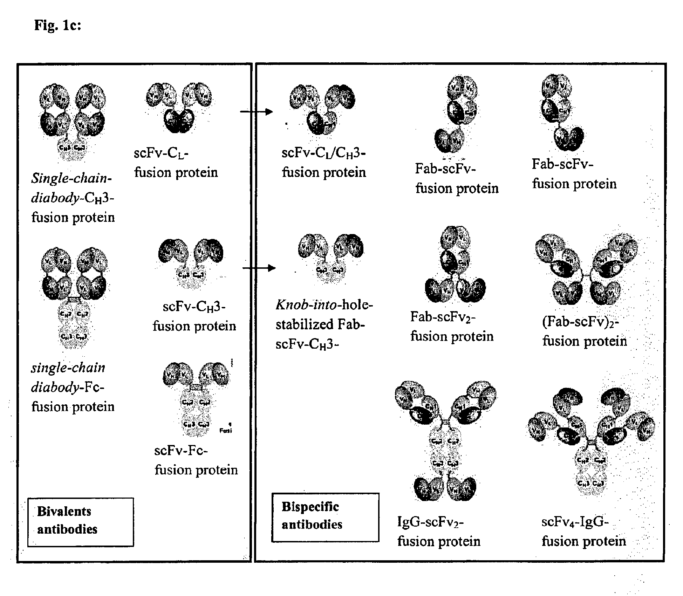

In addition to anti-ADM antibodies other biopolymer scaffolds are well known in the art to complex a target molecule and have been used for the generation of highly target specific biopolymers. Examples are aptamers, spiegelmers, anticalins and conotoxins. For illustration of antibody formats please see Fig. 1a, 1b and 1c.

In a preferred embodiment the anti-ADM antibody format is selected from the group comprising Fv fragment, scFv fragment, Fab fragment, scFab fragment, F(ab)$_2$ fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One of the most preferred formats is the scFab format.

Non-Ig protein scaffolds may be protein scaffolds and may be used as antibody mimics as they are capable to bind to ligands or antigenes. Non-Ig protein scaffolds may be selected from the group comprising tetranectin-based non-Ig protein scaffolds (*e.g.* described in US 2010/0028995), fibronectin scaffolds (*e.g.* described in EP 1266 025; lipocalin-based scaffolds ((e.g. described in WO 2011/154420); ubiquitin scaffolds (e.g. described in WO 2011/073214), transferring scaffolds (e.g. described in US 2004/0023334), protein A scaffolds (e.g. described in EP 2231860), ankyrin repeat based scaffolds (e.g. described in WO 2010/060748), microproteins preferably microproteins forming a cystine knot) scaffolds (e.g. described in EP 2314308), Fyn SH3 domain based scaffolds (e.g. described in WO 2011/023685) EGFR-A-domain based scaffolds (e.g. described in WO 2005/040229) and Kunitz domain based scaffolds (e.g. described in EP 1941867).

In one preferred embodiment of the invention antibodies according to the present invention may be produced as follows:

A Balb/c mouse was immunized with 100μg ADM-Peptide-BSA-Conjugate at day 0 and 14 (emulsified in 100μl complete

Freund's adjuvant) and 50μg at day 21 and 28 (in 100μl incomplete Freund's adjuvant). Three days before the fusion experiment was performed, the animal received 50μg of the conjugate dissolved in 100μl saline, given as one intraperitoneal and one intra-venous injection.

Splenocytes from the immunized mouse and cells of the myeloma cell line SP2/0 were fused with 1ml 50% polyethylene glycol for 30s at 37°C. After washing, the cells were seeded in 96-well cell culture plates. Hybrid clones were selected by growing in HAT medium [RPMI 1640 culture medium supplemented with 20% fetal calf serum and HAT-Supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting, the selected cultures were cloned and recloned using the limiting-dilution technique and the isotypes were determined (see also Lane, R.D. (1985). A short-duration polyethylene glycol fusion technique for increasing production of monoclonal antibody-secreting hybridomas. J. Immunol. Meth. 81: 223-228; Ziegler, B. et al. (1996) Glutamate decarboxylase (GAD) is not detectable on the surface of rat islet cells examined by cytofluorometry and complement-dependent antibody-mediated cytotoxicity of monoclonal GAD antibodies, Horm. Metab. Res. 28: 11-15).

Antibodies may be produced by means of phage display according to the following procedure:

The human naive antibody gene libraries HAL7/8 were used for the isolation of recombinant single chain F-Variable domains (scFv) against adrenomedullin peptide. The antibody gene libraries were screened with a panning strategy comprising the use of peptides containing a biotin tag linked via two different spacers to the adrenomedullin peptide sequence. A mix of panning rounds using non-specifically bound antigen and streptavidin bound antigen were used to minimize background of non-specific binders. The eluted phages from the third round of panning have been used for the generation of monoclonal scFv expressing E.coli strains. Supernatant from the cultivation of these clonal strains has been directly used for an antigen ELISA testing. (see also Hust, M., Meyer, T., Voedisch, B., Rülker, T., Thie, H., El-Ghezal, A., Kirsch, M.I., Schütte, M., Helmsing, S., Meier, D., Schirrmann, T., Dübel, S., 2011. A human scFv antibody generation pipeline for proteome research. Journal of Biotechnology 152, 159-170; Schütte, M., Thullier, P., Pelat, T., Wezler, X., Rosenstock, P., Hinz, D., Kirsch, M.I.,Hasenberg, M., Frank, R., Schirrmann, T., Gunzer, M., Hust, M., Dübel, S., 2009. Identification of a putative Crf splice variant and generation of recombinant antibodies for the specific detection of Aspergillus fumigatus. PLoS One 4, e6625)

Humanization of murine antibodies may be conducted according to the following procedure:

For humanization of an antibody of murine origin the antibody sequence is analyzed for the structural interaction of framework regions (FR) with the complementary determining regions (CDR) and the antigen. Based on structural modeling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modeling. (Almagro JC, Fransson J., 2008. Humanization of antibodies. Front Biosci. 2008 Jan 1;13:1619-33.)

In a preferred embodiment the ADM antibody format is selected from the group comprising Fv fragment, scFv fragment, Fab fragment, scFab fragment, F(ab)$_2$ fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One of the most preferred formats is scFab format.

In another preferred embodiment, the anti-ADM antibody, anti-ADM antibody fragment, or anti-ADM non-Ig protein scaffold is a full length antibody, antibody fragment, or non-Ig protein scaffold.

In a preferred embodiment the anti-adrenomedullin antibody or an anti-adrenomedullin antibody fragment or anti-adrenomedullin non-Ig protein scaffold is directed to and can bind to an epitope of at least 5 amino acids in length contained in ADM.

In a specific embodiment the anti-adrenomedullin antibody or an anti-adrenomedullin antibody fragment or anti-adrenomedullin non-Ig protein scaffold is directed to and can bind to an epitope of at least 4 amino acids in length contained in ADM.

In one specific embodiment of the invention the anti-Adrenomedullin (ADM) antibody or anti-ADM antibody fragment binding to adrenomedullin or anti-ADM non-Ig protein scaffold binding to adrenomedullin is provided for use as a medicament wherein said antibody or fragment or scaffold is not ADM-binding-Protein-1 (complement factor H).

In othe embodiment of the invention the anti-Adrenomedullin (ADM) antibody or anti-ADM antibody fragment binding to adrenomedullin or anti-ADM non-Ig protein scaffold binding to adrenomedullin is provided for use as a medicament wherein said antibody or fragment or scaffold binds to a region of preferably at least 4, or at least 5 amino acids within the sequence of aa 1-21 of mature human ADM:

SEQ ID No.: 23
YRQSMNNFQGLRSFGCRFGTC.

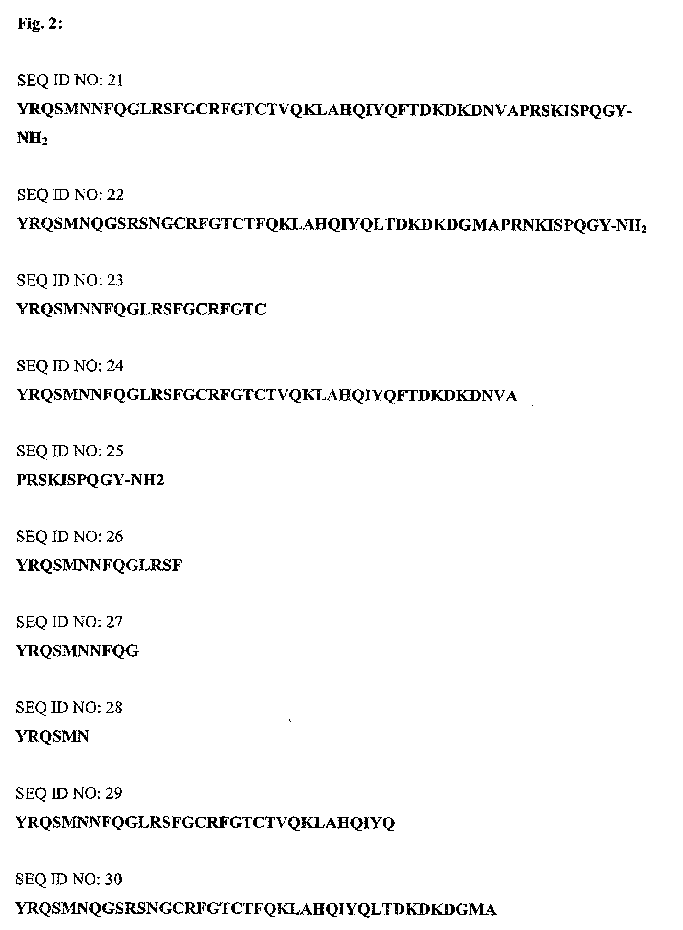

Thus, in the embodiment of the present invention said anti-ADM antibody or an anti-adrenomedullin antibody fragment or anti-ADM non-Ig protein scaffold binds to a region of ADM that is located in the N-terminal part (aa 1-21) of adrenomedullin, (see Fig. 2).

In another preferred embodiment said anti-antibody or an anti-adrenomedullin antibody fragment or anti-ADM non-Ig protein scaffold recognizes and binds to the N-terminal end (aa 1) of adrenomedullin. N-terminal end means that the amino acid 1, that is "Y" of SEQ ID No. 21 or 23; is mandatory for antibody binding. Said anti-ADM antibody or anti-ADM antibody fragment or anti-ADM non-Ig protein scaffold would neither bind N-terminal extended nor N-terminal modified Adrenomedullin nor N-terminal degraded adrenomedullin.

It also preferred to use an ADM antibody or an adrenomedullin antibody fragment according to the present invention, wherein said antibody or an adrenomedullin antibody fragment is an ADM stabilizing antibody or an ADM stabilizing adrenomedullin antibody fragment that enhances the $t_{1/2}$; half retention time of adrenomedullin in serum, blood, plasma at least 10 %, preferably at least 50 %, more preferably >50 %, most preferably >100. An assay that may be used for the determination of the half retention time of adrenomedullin in serum, blood, plasma is described in Example 3.

It is also preferred to use an ADM antibody or an adrenomedullin antibody fragment according to the present invention, wherein said antibody or said adrenomedullin antibody fragment blocks the bioactivity of ADM to less than 80 %, preferably less than 50%.

In a preferred embodiment a modulating antibody or a modulating adrenomedullin antibody fragment is used in the treatment of sepsis. A modulating antibody or an adrenomedullin anti-body fragment enhances the bioactivity of ADM in the early phase of sepsis and reduces the damaging effects of ADM in the late phase of sepsis. A "modulating" antibody or a modulating adrenomedullin antibody fragment is an antibody or an adrenomedullin antibody fragment that enhances the $t_{1/2}$ half retention time of adrenomedullin in serum, blood, plasma at least 10 %, preferably at least, 50 % more preferably >50 %, most preferably >100% and blocks the bioactivity of ADM to less than 80 %, preferably less than 50 %.

In another specific embodiment pursuant to the invention the herein provided anti-ADM antibody or anti-ADM antibody fragment or anti-ADM non-Ig protein scaffold does not bind to the C-terminal portion of ADM, *i.e.* the aa 43 - 52 of ADM
PRSKISPQGY-NH2
(SEQ ID NO: 25).

In a specific embodiment a modulating anti-ADM antibody or a modulating anti-adrenomedullin antibody fragment or a modulating anti-adrenomedullin non-Ig protein scaffold according to the present invention is used for treatment of subjects for preventing or treating diseases or conditions, e.g. for use in prevention or therapy or prevention of a chronic or acute disease or acute condition of a subject.

Such a modulating anti-ADM antibody or a modulating anti-adrenomedullin antibody fragment or a modulating anti-adrenomedullin non-Ig protein scaffold may be especially useful in the treatment of sepsis. A modulating anti-ADM antibody or a modulating anti-adrenomedullin antibody fragment or a modulating anti-adrenomeduliin non-Ig protein scaffold enhances the bioactivity of ADM in the early phase of sepsis and reduces the damaging effects of ADM in the late phase of sepsis.

A "modulating" antibody or a modulating adrenomedullin antibody fragment or a modulating adrenomedullin non-Ig protein scaffold is an antibody or antibody fragment or non-Ig protein scaffold that enhances the half life ($t_{1/2}$ half retention time) of adrenomedullin in serum, blood, plasma at least 10 %, preferably at least, 50 %, more preferably >50 %, most preferably >100% and blocks the bioactivity of ADM to less than 80 %, preferably less than 50 % and wherein the bioactivity of ADM is blocked to at least 5%. These values related to half-life and blocking of bioactivity have to be understood in relation to the before-mentioned assays in order to determine these values. It has to be understood that said modulating antibody or a modulating adrenomedullin antibody fragment or a modulating adrenomedullin non-Ig protein scaffold is an antibody or antibody fragment or non-Ig protein scaffold that is binding to ADM.

It should be emphasized that blocking the ADM bioactivity is in the sense of no more than 80%, and thus 20% residual ADM bioactivity. The same applies to blocking the ADM bioactivity to no more than 50%, and thus residual 50% ADM bioactivity.

Subject matter of the present disclosure is an anti-adrenomedullin antibody or an anti-adrenomedullin antibody fragment or an anti-ADM non-Ig protein scaffold, wherein said antibody or fragment or scaffold is

• an ADM stabilizing antibody or an adrenomedullin stabilizing antibody fragment or ADM stabilizing non-Ig protein scaffold that enhances the $t_{1/2}$ half retention time of adrenomedullin in serum, blood, plasma at least 10 %, preferably at least, 50 %, more preferably >50 %, most preferably 100 % and/or

- wherein said anti-adrenomedullin antibody or said anti-adrenomeduliin antibody fragment or said anti-ADM non-Ig protein scaffold blocks the bioactivity of ADM to less than 80 %, preferably to less than 50 %.

The stated above is in the sense of blocking the ADM bioactivity of not more than 80 % or not more than 50 %, respectively, and thus is to be understood as limited blocking of ADM bioactivity by the respective ADM binder, being it an ADM stabilizing antibody or antibody fragment or non-Ig protein scaffold, respectively.

Such a modulating antibody or a modulating adrenomedullin antibody fragment or a modulating adrenomedullin non-Ig protein scaffold offers the advantage that the dosing of the administration is facilitated. The combination of partially blocking or partially reducing Adrenomedullin bioactivity and increase of the in vivo half life (increasing the Adrenomedullin bioactivity) leads to beneficial simplicity of anti-Adrenomedullin antibody or an anti-adrenomedullin antibody fragment or anti-adrenomedullin non-Ig protein scaffold dosing. In a situation of excess of endogenous Adrenomedullin (maximal stimulation, late sepsis phase, shock, hypodynamic phase) the activity lowering effect is the major impact of the antibody or fragment or scaffold, limiting the (negative) effect of Adrenomedullin. In case of low or normal endogenous Adrenomedullin concentrations, the biological effect of anti-Adrenomedullin antibody or an anti-adrenomedullin antibody fragment or anti-ADM non-Ig protein scaffold is a combination of lowering (by partially blocking) and increase by increasing the Adrenomedullin half life. If the half life effect is stronger than the blocking effect, the net biological activity of endogenous Adrenomedullin is beneficially increased in early phases of sepsis (low Adrenomedullin, hyperdynamic phase). Thus, the non-neutralizing and modulating anti-Adrenomedullin antibody or anti-adrenomedullin antibody fragment or anti-adrenomedullin non-Ig protein scaffold acts like an ADM bioactivity buffer in order to keep the bioactivity of ADM within a certain physiological range.

Thus, the dosing of the antibody/fragment/scaffold in e.g. sepsis may be selected from an excessive concentration, because both sepsis phases (early and late phase) benefit from excessive anti-ADM antibody or an anti-adrenomedullin antibody fragment or anti-ADM non-Ig protein scaffold treatment in case of a modulating effect. Excessive means: The anti-Adrenomedullin antibody or an anti-adrenomedullin antibody fragment or anti-ADM non-Ig protein scaffold concentration is higher than endogenous Adrenomedullin during late phase (shock) of e.g. sepsis. This means, in case of a modulating antibody or modulating antibody fragment or modulating non-Ig protein scaffold dosing in sepsis may be as follows:

The concentration of Adrenomedullin in septic shock is 226+/-66 fmol/ml (Nishio et al., "Increased plasma concentrations of adrenomedullin correlate with relaxation of vascular tone in patients with septic shock.", Crit Care Med. 1997, 25(6):953-7), an equimolar concentration of antibody or fragment or scaffold is 42.5$\mu$g/l blood, (based on 6 1 blood volume / 80kg body weight) 3.2$\mu$g/kg body weight. Excess means at least double (mean) septic shock Adrenomedullin concentration, at least > 3$\mu$g anti-Adrenomedullin antibody or an anti-adrenomedullin antibody fragment or anti-ADM non-Ig protein scaffold / kg body weight, preferred at least 6.4$\mu$g anti-Adrenomedullin antibody or an anti-adrenomedullin antibody fragment or anti-ADM non-Ig protein scaffold / kg body weight. Preferred > 10$\mu$g / kg, more preferred >20$\mu$g/kg, most preferred >100ug anti-Adrenomedullin antibody or an anti-adrenomedullin antibody fragment or anti-DM non-Ig protein scaffold / kg body weight. This may apply to other severe and acute conditions than septic shock as well.

Furthermore, in one embodiment of the invention an anti-Adrenomedullin (ADM) antibody or an anti-adrenomedullin antibody fragment or an anti-ADM non-Ig protein scaffold is monospecific.

Monospecific anti-Adrenomedullin (ADM) antibody or monospecific anti-adrenomedullin antibody fragment or monospecific anti-ADM non-Ig protein scaffold means that said antibody or antibody fragment or non-Ig protein scaffold binds to one specific region encompassing preferably at least 4, or at least 5 amino acids within the target ADM. Monospecific anti-Adrenomedullin (ADM) antibody or monospecific anti-adrenomedullin antibody fragment or monospecific anti-ADM non-Ig protein scaffold are anti-Adrenomedullin (ADM) antibodies or anti-adrenomedullin antibodiy fragments or anti-ADM non-Ig protein scaffolds that all have affinity for the same antigen

In another special embodiment the anti-ADM antibody or the antibody fragment binding to ADM is a monospecific antibody. Monospecific means that said antibody or antibody fragment binds to one specific region encompassing preferably at least 4, or preferably at least 5 amino acids within the target ADM. Monospecific antibodies or fragments are antibodies or fragments that all have affinity for the same antigen. Monoclonal antibodies are monospecific, but monospecific antibodies may also be produced by other means than producing them from a common germ cell.

In a specific embodiment of the invention the antibody is a monoclonal antibody or a fragment thereof. In one embodiment of the invention the anti-ADM antibody or the anti-ADM antibody fragment is a human or humanized antibody or derived therefrom. In one specific embodiment one or more (murine) CDR's are grafted into a human antibody or antibody fragment.

Subject matter of the present invention in one aspect is a human CDR-grafted antibody or antibody fragment thereof that binds to ADM, wherein the human CDR-grafted antibody or antibody fragment thereof comprises an antibody heavy chain (H chain) comprising:

SEQ ID NO: 1
GYTFSRYW

SEQ ID NO: 2
ILPGSGST
and/or

SEQ ID NO: 3
TEGYEYDGFDY
and/or further comprises an antibody light chain (L chain) comprising:

SEQ ID NO:4
QSIVYSNGNTY

SEQ ID NO: 5
RVS
and/or

SEQ ID NO: 6
FQGSHIPYT.

In one specific embodiment of the invention subject matter of the present invention is a human monoclonal antibody that binds to ADM or an antibody fragment thereof wherein the heavy chain comprises at least one CDR selected from the group comprising:

SEQ ID NO: 1
GYTFSRYW

SEQ ID NO: 2
ILPGSGST

SEQ ID NO: 3
TEGYEYDGFDY
and wherein the light chain comprises at least one CDR selected from the group comprising:

SEQ ID No: 4
QSIVYSNGNTY

SEQ ID NO: 5
RVS

SEQ ID NO: 6
FQGSHIPYT.

In a more specific embodiment of the invention subject matter of the invention is a human monoclonal antibody that binds to ADM or an antibody fragment thereof wherein the heavy chain comprises the sequences
SEQ ID NO: 1
GYTFSRYW
SEQ ID NO: 2
ILPGSGST
SEQ ID NO: 3
TEGYEYDGFDY
and wherein the light chain comprises the sequences
SEQ ID NO: 4
QSIVYSNGNTY
SEQ ID NO: 5

RVS
SEQ ID NO: 6
FQGSHIPYT.

In a very specific embodiment the ADM antibody has a sequence selected from the group comprising: SEQ ID NO 7, 8, 9, 10, 11, 12, 13 and 14:

SEQ ID NO: 7 ;AM-VH-C

QVQLQQSGAELMKPGASVKISCKATGYTFSRYWIEWVKQRPGHGLEWIGEILPGSGS
TNYNEKFKGKATITADTSSNTAYMQLSSLTSEDSAVYYCTEGYEYDGFDYWGQGTT
LTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKRVEPKHHHHHH

SEQ ID NO: 8 ;AM-VH1

QVQLVQSGAEVKKPGSSVKVSCKASGYTFSRYWISWVRQAPGQGLEWMGRILPGSG
STNYAQKFQGRVTITADESTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKHHHHHH

SEQ ID NO: 9 ;AM-VH2-E40

QVQLVQSGAEVKKPGSSVKVSCKASGYTFSRYWIEWVRQAPGQGLEWMGRILPGSG
STNYAQKFQGRVTITADESTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKHHHHHH

SEQ ID NO: 10 ;AM-VH3-T26-E55

QVQLVQSGAEVKKPGSSVKVSCKATGYTFSRYWISWVRQAPGQGLEWMGEILPGSG
STNYAQKFQGRVTITADESTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKHHHHHH

SEQ ID NO: 11 ;AM-VH4-T26-E40-E55

QVQLVQSGAEVKKPGSSVKVSCKATGYTFSRYWIEWVRQAPGQGLEWMGEILPGSG
STNYAQKFQGRVTITADESTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKHHHHHH

SEQ ID NO: 12 ;AM-VL-C

DVLLSQTPLSLPVSLGDQATISCRSSQSIVYSNGNTYLEWYLQKPGQSPKLLIYRVSNR

FSGVPDRFSGSGSGTDFTLKISRVEAEDLGVYYCFQGSHIPYTFGGGTKLEIKRTVAAP

SVFIFPPSDEQLKSGTASVVCLLNNFYPREAKVQWKVDNALQSGNSQESVTEQDSKD

STYSLSSTLTLSKADYEKHKVYACEVTHQGLSSPVTKSFNRGEC

SEQ ID NO: 13 ;AM-VL1

DVVMTQSPLSLPVTLGQPASISCRSSQSIVYSNGNTYLNWFQQRPGQSPRRLIYRVSN

RDSGVPDRFSGSGSGTDFTLKISRVEAEDVGVYYCFQGSHIPYTFGQGTKLEIKRTVA

APSVFIFPPSDEQLKSGTASVVCLLNNFYPREAKVQWKVDNALQSGNSQESVTEQDS

KDSTYSLSSTLTLSKADYEKHKVYACEVTHQGLSSPVTKSFNRGEC

SEQ ID NO: 14 ;AM-VL2-E40

DVVMTQSPLSLPVTLGQPASISCRSSQSIVYSNGNTYLEWFQQRPGQSPRRLIYRVSNR

DSGVPDRFSGSGSGTDFTLKISRVEAEDVGVYYCFQGSHIPYTFGQGTKLEIKRTVAA

PSVFIFPPSDEQLKSGTASVVCLLNNFYPREAKVQWKVDNALQSGNSQESVTEQDSK

DSTYSLSSTLTLSKADYEKHKVYACEVTHQGLSSPVTKSFNRGEC

The anti-ADM antibody or anti-adrenomedullin antibody fragment or anti-ADM non-Ig protein scaffold according to the present invention exhibits an affinity towards human ADM in such that affinity constant is greater than $10^{-7}$ M, preferred $10^{-8}$ M, preferred affinity is greater than $10^{-9}$ M, most preferred higher than $10^{-10}$ M. A person skilled in the art knows that it may be considered to compensate lower affinity by applying a higher dose of compounds and this measure would not lead out-of-the-scope of the invention. The affinity constants may be determined according to the method as described in Example 1.

The antibodies or fragments according to the present invention can be used in combinations with other agents, as e.g. with the so-called ADM-binding protein, for the uses as described therein.

It should be emphasized that the term "ADM binding protein" comprises ADM-binding-protein-1 (complement factor H), which however does not reflect a non-neutralizing and/or modulating anti-ADM antibody, antibody fragment, or non-Ig protein scaffold in accordance with the invention.

In a preferred embodiment the anti-ADM antibody or the anti-ADM antibody fragment or anti-ADM non-Ig protein scaffold is used for reducing the risk of mortality during a chronic or acute disease or acute condition of a patient.

Chronic or acute disease or acute condition according to the present invention may be a disease or condition selected from the group comprising severe infections as e.g. meningitis, Systemic inflammatory Response-Syndrom (SIRS) sepsis; other diseases as diabetes, cancer, acute and chronic vascular diseases as e.g. heart failure, myocardial infarction, stroke, atherosclerosis; shock as e.g. septic shock and organ dysfunction as e.g. kidney dysfunction, liver dysfunction, burnings, surgery, trauma poisoning, by chemotherapy damages. Especially useful is the antibody or fragment or scaffold according to the present invention for reducing the risk of mortality during sepsis and septic shock, *i.e.* late phases of sepsis.

In one embodiment the anti-ADM antibody or an anti-adrenomedullin antibody fragment or anti-ADM non-Ig protein scaffold is used in therapy or prevention of a chronic or acute disease or acute condition of a patient according to the present invention wherein said patient is an ICU patient. In another embodiment the anti-ADM antibody or an anti-adrenomedullin antibody fragment or anti-ADM non-Ig protein scaffold is used in therapy or prevention of a chronic or acute disease of a patient according to the present invention wherein said patient is critically ill. Critically ill means a patient having a disease or state in which death is possible or imminent.

The antibodies, antibody fragments and non-Ig protein scaffolds according to the present invention may be used for treatment of subjects for preventing or treating diseases or conditions, e.g. for use in prevention or therapy of a chronic or acute disease or acute condition of a subject. Such a disease may be selected from the group comprising severe infections as e.g. meningitis, Systemic inflammatory Response-Syndrome (SIRS,) sepsis; other diseases as diabetes, cancer, acute and chronic vascular diseases as e.g. heart failure, myocardial infarction, stroke, atherosclerosis; shock as e.g. septic shock and organ dysfunction as e.g. kidney dysfunction, liver dysfunction, burnings, surgery, traumata. Especially useful is the antibody or fragment or scaffold according to the present invention for reducing the risk of mortality during sepsis and septic shock, *i.e.* late phases of sepsis.

In the following clinical criteria for SIRS, sepsis, severe sepsis, septic shock will be defined.

1) Systemic inflammatory host response (SIRS) characterized by at least two of the following symptoms

- patients exhibit hypotension (mean arterial pressure is < 65 mm Hg)
- elevated serum lactate level being > 4 mmol/L
- blood glucose > 7.7 mmol/L (in absence of diabetes)
- central venous pressure is not within the range 8-12 mm Hg
- urine output is < 0.5 mL x kg$^{-1}$ x hr$^{-1}$
- central venous (superior vena cava) oxygen saturation is < 70% or mixed venous < 65%
- heart rate is > 90 beats/min
- temperature < 36°C or > 38°C
- respiratory rate > 20/min
- white cell count < 4 or > 12x10$^9$/L (leucocytes); > 10% immature neutrophils

2) Sepsis

Following at least two of the symptoms mentioned under 1), and additionally a clinical suspicion of new infection, being:

- cough/sputum/chest pain

- abdominal pain/distension/diarrhoea

- line infection

- endocarditis

- dysuria

- headache with neck stiffness

- cellulitis/wound/joint infection

- positive microbiology for any infection

3) Severe sepsis

Provided that sepsis is manifested in patient, and additionally a clinical suspicion of any organ dysfunction, being:

- blood pressure systolic < 90/mean; < 65mmHG

- lactate > 2 mmol/L

- Bilirubine > 34 $\mu$mol/L

- urine output < 0.5 mL/kg/h for 2h

- creatinine > 177 $\mu$mol/L

- platelets < 100x10$^9$/L

- SpO$_2$ > 90% unless O$_2$ given

4) Septic shock

At least one sign of end-organ dysfunction as mentioned under 3) is manifested. Septic shock is indicated, if there is refractory hypotension that does not respond to treatment and intravenous fluid administration alone is insufficient to maintain a patient's blood pressure from becoming hypotensive also provides for an administration of an anti-ADM antibody or an anti-ADM antibody fragment or an anti-ADM non-Ig protein scaffold in accordance with the present invention.

In one embodiment of the present invention the patient is not suffering from SIRS, a severe infection, sepsis, shock as *e.g.* septic shock. Said severe infection denotes *e.g.* meningitis, Systemic inflammatory Response-Syndrome (SIRS), sepsis, severe sepsis, and shock as *e.g.* septic shock. In this regard, a severe sepsis is characterized in that sepsis is manifested in said patient, and additionally a clinical suspicion of any organ dysfunction is present, being it:

- blood pressure systolic < 90/means; < 65mmHG

- lactate > 2 mmol/L

- Bilirubine > 34 $\mu$mol/L

- urine output < 0.5 mL/kg/h for 2h

- creatinine >177 $\mu$mol/L

- platelets <100x 10$^9$/L

- SpO$_2$ > 90% unless O$_2$ given

In another specific embodiment said acute disease or acute condition is not sepsis, severe sepsis or is not SIRS or is not shock, or septic shock.

In another embodiment said acute disease or acute condition is not sepsis.

The antibodies or fragments or scaffolds according to the present invention can be used in combinations with other agents, as e.g. with the so-called ADM binding protein, for the uses as described therein. ADM binding protein is also naturally present in the circulation of said patient.

It should be emphasized that the term "ADM binding protein" comprises ADM-binding-protein-1 (complement factor H), which however does not reflect a non-neutralizing and/or modulating anti-ADM antibody, antibody fragment, or non-Ig protein scaffold in accordance with the invention.

Subject of the present invention is further an anti-ADM antibody or an anti-adrenomedullin antibody fragment or anti-ADM non-Ig protein scaffold for use in therapy or prevention of a chronic or acute disease or acute condition of a patient according to the present invention, wherein said antibody or fragment or scaffold is to be used in combination with further active ingredients.

Subject matter of the invention is also an anti-Adrenomedullin (ADM) antibody or an anti-adrenomedullin antibody fragment or an anti-ADM non-Ig protein scaffold to be used in combination with another active drug, e.g. used as primary medicament, wherein said combination is for use in therapy or prevention of a chronic or acute disease or acute condition of a patient for stabilizing the circulation of said patient, in particular the systemic circulation of said patient.

Primary medicament means a medicament that acts against the primary cause of said disease or condition. Said primary medicament may be antibiotics in case of infections.

In a specific embodiment of the before mentioned combinations said combinations are to be used in combination with vasopressors e.g. catecholamine, wherein said further combination is for use in therapy or prevention of a chronic or acute disease or condition of a patient.

In one embodiment of the invention said patient having a chronic or acute disease or chronic condition is characterized by the need of the patient to get administration of vasopressors e.g. catecholamine administration.

Subject matter of the invention in one specific embodiment is, thus, an anti-Adrenomedullin (ADM) antibody or an anti-adrenomedullin antibody fragment or an anti-ADM non-Ig protein scaffold to be used in combination with ADM binding protein and/or further active ingredients for use in therapy or prevention of a patient in need of a treatment of vasopressors e.g. catecholamine treatment.

In a specific embodiment of the above-mentioned combinations said combinations are to be used in combination with fluids administered intravenously, wherein said combination is for use in therapy or prevention of a chronic or acute disease or condition of a patient for stabilizing the circulation, in particular the systemic circulation.

In one embodiment of the invention said patient having a chronic or acute disease or acute condition being in need for stabilizing the circulation is characterized by the need of the patient to get intravenous fluids.

Subject matter of the invention in one specific embodiment is, thus, an anti-Adrenomedullin (ADM) antibody or an anti-adrenomedullin antibody fragment or anti-ADM non-Ig protein scaffold in combination with ADM binding protein and/or further active ingredients for use in therapy or prevention of a patient in need of intravenous fluids. This is in the sense of the patient is in need of intravenous fluids for regulating the systemic fluid balance.

It should be emphasized that the term ADM binding protein also denotes ADM-binding-protein-1 (complement factor H), which however is not a non-neutralizing and modulating anti-ADM antibody, anti-ADM antibody fragment, or anti-ADM non-Ig protein scaffold as in accordance with the invention.

In accordance with the invention the ADM-binding-protein-1 may also be referred to as ADM-binding-protein-1 (complement factor H).

Said anti-ADM antibody or an anti-adrenomedullin antibody fragment or anti-ADM non-Ig protein scaffold or combinations thereof with ADM binding protein and/or further active ingredients may be used in combination with vasopressors e.g. catecholamine and/or with fluids administered intravenously for use in a of a chronic or acute disease or acute condition of a patient for stabilizing the circulation, in particular for stabilizing the systemic circulation.

Subject matter of the invention is also an anti-ADM antibody or an anti-adrenomedullin antibody fragment or anti-ADM non-Ig according to the present invention to be used in combination with TNF-alpha-antibodies. TNF-alpha-antibodies are commercially available for the treatment of patients.

In a preferred embodiment the anti-ADM antibody or the anti-ADM antibody fragment or anti-ADM non-Ig protein scaffold is used for reducing the risk of mortality during said chronic or acute disease of a patient wherein said disease is selected from the group comprising sepsis, diabetes, cancer, acute and chronic vascular diseases as e.g. heart failure, shock as e.g. septic shock and organ dysfunction as e.g. kidney dysfunction. Especially useful is the antibody or fragment or scaffold according to the present invention for reducing the risk of mortality during sepsis and septic shock, *i.e.* late phases of sepsis.

The antibodies, antibody fragments, scaffolds and combinations of the present invention may be used in therapy or prevention of a chronic or acute disease of a patient:

• for the prevention of organ dysfunction or organ failure, especially kidney dysfunction or kidney failure and / or,

• for stabilizing the circulation, e.g. for reducing the requirement of vasopressors e.g. catecholamine requirement of said patient and / or,

• for regulating the fluid balance in said patient.

• for reducing the mortality risk for said patient.

In one embodiment the anti-ADM antibody or an anti-adrenomedullin antibody fragment or anti-ADM non-Ig protein scaffold is used in therapy or prevention of a chronic or acute disease of a patient according to the present invention wherein said patient is an ICU patient. In another embodiment the anti-ADM antibody or an anti-adrenomedullin antibody fragment or anti-ADM non-Ig protein scaffold is used in therapy or prevention of a chronic or acute disease or acute condition of a patient according to the present invention, wherein said patient is critically ill. Critically ill means a patient is having a disease or state in which death is possible or imminent.

Subject of the present invention is further an anti-ADM antibody or an anti-adrenomedullin antibody fragment or anti-ADM non-Ig protein scaffold for use in therapy or prevention of a chronic or acute disease or acute condition of a patient according to the present invention wherein said antibody or fragment or scaffold is to be used in combination of ADM binding protein.

Subject of the present invention is further a pharmaceutical formulation comprising an anti-ADM antibody or anti-ADM antibody fragment or anti-ADM non-Ig protein scaffold according to the present invention.

Subject of the present invention is further a pharmaceutical formulation according to the present invention, wherein said pharmaceutical formulation is a solution, preferably a ready-to-use solution.

Said pharmaceutical formulation may be administered intra-muscular. Said pharmaceutical formulation may be administered intra-vascular. Said pharmaceutical formulation may be administered via infusion.

In another embodiment subject of the present invention is further a pharmaceutical formulation according to the present invention, wherein said pharmaceutical formulation is in a dried state to be reconstituted before use.

In another embodiment subject of the present invention is further a pharmaceutical formulation according to the present invention, wherein said pharmaceutical formulation is in a freeze-dried state.

In another embodiment of the invention the pharmaceutical formulation in accordance with the invention as may be administered intra-muscular, intra-vascular, or via infusion is preferably administered to a patient systemically. Therefore, in another embodiment of the present invention the pharmaceutical formulation according to the present invention is to be administered systemically to a patient.

## EXAMPLES

It should be emphasized that the antibodies, antibody fragments and non-Ig protein scaffolds of the example portion in accordance with the invention are binding to ADM, and thus should be considered as anti-ADM antibodies/antibody fragments/non-Ig protein scaffolds.

The following examples shall illustrate the invention.

## Example 1

### Generation of Antibodies and determination of their affinity constants

Several human and murine antibodies were produced and their affinity constants were determined (see tables 1 and 2).

Peptides/ conjugates for Immunization:

Peptides for immunization were synthesized, see Table 1, (JPT Technologies, Berlin, Gennany) with an additional N-terminal Cystein (if no Cystein is present within the selected ADM-sequence) residue for conjugation of the peptides to Bovine Serum Albumin (BSA). The peptides were covalently linked to BSA by using Sulfolink-coupling gel (Perbio-science, Bonn, Germany). The coupling procedure was performed according to the manual of Perbio.

The **murine antibodies** were generated according to the following method:

A Balb/c mouse was immunized with 100$\mu$g Peptide-BSA-Conjugate at day 0 and 14 (emulsified in 100$\mu$l complete Freund's adjuvant) and 50$\mu$g at day 21 and 28 (in 100$\mu$l incomplete Freund's adjuvant). Three days before the fusion experiment was performed, the animal received 50$\mu$g of the conjugate dissolved in 100$\mu$l saline, given as one intraperitoneal and one intra-venous injection.

Splenocytes from the immunized mouse and cells of the myeloma cell line SP2/0 were fused with 1ml 50% polyethylene glycol for 30s at 37°C. After washing, the cells were seeded in 96-well cell culture plates. Hybrid clones were selected by growing in HAT medium [RPMI 1640 culture medium supplemented with 20% fetal calf serum and HAT-Supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting, the selected cultures were cloned and recloned using the limiting-dilution technique and the isotypes were determined.

(see also Lane, R.D. "A short-duration polyethylene glycol fusion technique for increasing production of monoclonal antibody-secreting hybridomas", J. Immunol. Meth. 81: 223-228; (1985), Ziegler, B. et al. "Glutamate decarboxylase (GAD) is not detectable on the surface of rat islet cells examined by cytofluorometry and complement-dependent antibody-mediated cytotoxicity of monoclonal GAD antibodies", Horm. Metab. Res. 28: 11-15, (1996)).

### Mouse monoclonal antibody production:

Antibodies were produced via standard antibody production methods (Marx et al, Monoclonal Antibody Production, ATLA 25, 121, 1997,) and purified via Protein A. The antibody purities were > 95% based on SDS gel electrophoresis analysis.

### Human Antibodies

Human Antibodies were produced by means of phage display according to the following procedure:

The human naive antibody gene libraries HAL7/8 were used for the isolation of recombinant single chain F-Variable domains (scFv) against adrenomedullin peptide. The antibody gene libraries were screened with a panning strategy comprising the use of peptides containing a biotin tag linked via two different spacers to the adrenomedullin peptide sequence. A mix of panning rounds using non-specifically bound antigen and streptavidin bound antigen were used to minimize background of non-specific binders. The eluted phages from the third round of panning have been used for the generation of monoclonal scFv expressing E.coli strains. Supernatant from the cultivation of these clonal strains has been directly used for an antigen ELISA testing (see also Hust, M., Meyer, T., Voedisch, B., Rülker, T., Thie, H., El-Ghezal, A., Kirsch, M.I., Schütte, M., Helmsing, S., Meier, D., Schirrmann, T., Dübel, S., 2011. A human scFv antibody generation pipeline for proteome research. Journal of Biotechnology 152, 159-170; Schütte, M., Thullier, P., Pelat, T., Wezler, X., Rosenstock, P., Hinz, D., Kirsch, M.I.,Hasenberg, M., Frank, R., Schirrmann, T., Gunzer, M., Hust, M., Dübel, S., 2009. Identification of a putative Crf splice variant and generation of recombinant antibodies for the specific detection of Aspergillus fumigatus. PLoS One 4, e6625).

Positive clones have been selected based on positive ELISA signal for antigen and negative for streptavidin coated micro titer plates. For further characterizations the scFv open reading frame has been cloned into the expression plasmid pOPE107 (Hust et al., J. Biotechn. 2011), captured from the culture supernatant via immobilised metal ion affinity chromatography and purified by a size exclusion chromatography.

**Affinity Constants**

To determine the affinity of the antibodies to Adrenomedullin, the kinetics of binding of Adrenomedullin to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare). (Lorenz et al.," Functional Antibodies Targeting IsaA of Staphylococcus aureus Augment Host Immune Response and Open New Perspectives for Antibacterial Therapy"; Antimicrob Agents Chemother. 2011 January; 55(1): 165-173.)

The monoclonal antibodies were raised against the below depicted ADM regions of human and murine ADM, respectively. The following table represents a selection of obtained antibodies used in further experiments. Selection was based on target region:

Table 1:

| Sequence Number | Antigen/Immunogen | ADM Region | Designation | Affinity constants Kd (M) |
|---|---|---|---|---|
| SEQ ID: 15 | YRQSMNNFQGLRSFGCRFGTC | 1-21 | NT-H | $5.9 \times 10^{-9}$ |
| SEQ ID: 16 | CTVQKLAHQIYQ | 21-32 | MR-H | $2 \times 10^{-9}$ |
| SEQ ID: 17 | CAPRSKISPQGY-NH2 | C-42-52 | CT-H | $1.1 \times 10^{-9}$ |
| SEQ ID: 18 | YRQSMNQGSRSNGCRFGTC | 1-19 | NT-M | $3.9 \times 10^{-9}$ |
| SEQ ID: 19 | CTFQKLAHQIYQ | 19-31 | MR-M | $4.5 \times 10^{-10}$ |
| SEQ ID: 20 | CAPRNKISPQGY-NH2 | C-40-50 | CT-M | $9 \times 10^{-9}$ |

The following is a list of further obtained monoclonal antibodies:

List of anti-ADM-antibodies

**Table 2:**

| Target | Source | Clone number | Affinity (M) | max inhibition bioassay (%) (see example 2) |
|---|---|---|---|---|
| NT-M | Mouse | ADM/63 | $5.8 \times 10^{-9}$ | 45 |
| | Mouse | ADM/364 | $2.2 \times 10^{-8}$ | 48 |
| | Mouse | ADM/365 | $3.0 \times 10^{-8}$ | |
| | Mouse | ADM/366 | $1.7 \times 10^{-8}$ | |
| | Mouse | ADM/367 | $1.3 \times 10^{-8}$ | |
| | Mouse | ADM/368 | $1.9 \times 10^{-8}$ | |
| | Mouse | ADM/369 | $2.0 \times 10^{-8}$ | |

(continued)

| Target | Source | Clone number | Affinity (M) | max inhibition bioassay (%) (see example 2) |
|---|---|---|---|---|
| | Mouse | ADM/370 | $1.6 \times 10^{-8}$ | |
| | Mouse | ADM/371 | $2.0 \times 10^{-8}$ | |
| | Mouse | ADM/372 | $2.5 \times 10^{-8}$ | |
| | Mouse | ADM/373 | $1.8 \times 10^{-8}$ | |
| | Mouse | ADM/377 | $1.5 \times 10^{-8}$ | |
| | Mouse | ADM/378 | $2.2 \times 10^{-8}$ | |
| | Mouse | ADM/379 | $1.6 \times 10^{-8}$ | |
| | Mouse | ADM/380 | $1.8 \times 10^{-8}$ | |
| | Mouse | ADM/381 | $2.4 \times 10^{-8}$ | |
| | Mouse | ADM/382 | $1.6 \times 10^{-8}$ | |
| | Mouse | ADM/383 | $1.8 \times 10^{-8}$ | |
| | Mouse | ADM/384 | $1.7 \times 10^{-8}$ | |
| | Mouse | ADM/385 | $1.7 \times 10^{-8}$ | |
| | Mouse | ADM/403 | $1.2 \times 10^{-8}$ | |
| | Mouse | ADM/395 | $1.2 \times 10^{-8}$ | |
| | Mouse | ADM/396 | $3.0 \times 10^{-8}$ | |
| | Mouse | ADM/397 | $1.5 \times 10^{-8}$ | |
| MR-M | Mouse | ADM/38 | $4.5 \times 10^{-10}$ | 68 |
| MR-M | Mouse | ADM/39 | $5.9 \times 10^{-9}$ | 72 |
| CT-M | Mouse | ADM/65 | $9.0 \times 10^{-9}$ | 100 |
| CT-M | Mouse | ADM/66 | $1.6 \times 10^{-8}$ | 100 |
| NT-H | Mouse | ADM/33 | $5.9 \times 10^{-8}$ | 38 |
| NT-H | Mouse | ADM/34 | $1.6 \times 10^{-8}$ | 22 |
| MR-H | Mouse | ADM/41 | $1.2 \times 10^{-8}$ | 67 |
| MR-H | Mouse | ADM/42 | $<1 \times 10^{-8}$ | |
| MR-H | Mouse | ADM/43 | $2.0 \times 10^{-9}$ | 73 |
| MR-H | Mouse | ADM/44 | $<1 \times 10^{-8}$ | |
| CT-H | Mouse | ADM/15 | $<1 \times 10^{-8}$ | |
| CT-H | Mouse | ADM/16 | $1.1 \times 10^{-9}$ | 100 |
| CT-H | Mouse | ADM/17 | $3.7 \times 10^{-9}$ | 100 |
| CT-H | Mouse | ADM/18 | $<1 \times 10^{-8}$ | |
| hADM | Phage display | ADM/A7 | $<1 \times 10^{-8}$ | |
| | Phage display | ADM/B7 | $<1 \times 10^{-8}$ | |
| | Phage display | ADM/C7 | $<1 \times 10^{-8}$ | |
| | Phage display | ADM/G3 | $<1 \times 10^{-8}$ | |
| | Phage display | ADM/B6 | $<1 \times 10^{-8}$ | |
| | Phage display | ADM/B11 | $<1 \times 10^{-8}$ | |
| | Phage display | ADM/D8 | $<1 \times 10^{-8}$ | |

(continued)

| Target | Source | Clone number | Affinity (M) | max inhibition bioassay (%) (see example 2) |
|---|---|---|---|---|
| | Phage display | ADM/D11 | $<1\times10^{-8}$ | |
| | Phage display | ADM/G12 | $<1\times10^{-8}$ | |

Generation of antibody fragments by enzymatic digestion:

The generation of Fab and F(ab)$_2$ fragments was done by enzymatic digestion of the murine full length antibody NT-M. Antibody NT-M was digested using a) the pepsin-based F(ab)$_2$ Preparation Kit (Pierce 44988) and b) the papain-based Fab Preparation Kit (Pierce 44985). The fragmentation procedures were performed according to the instructions provided by the supplier. Digestion was carried out in case of F(ab)$_2$-fragmentation for 8h at 37°C. The Fab-fragmentation digestion was carried out for 16h, respectively.

Procedure for Fab Generation and Purification:

The immobilized papain was equilibrated by washing the resin with 0.5 ml of Digestion Buffer and centrifuging the column at 5000 x g for 1 minute. The buffer was discarded afterwards. The desalting column was prepared by removing the storage solution and washing it with digestion buffer, centrifuging it each time afterwards at 1000 x g for 2 minutes. 0.5ml of the prepared IgG sample where added to the spin column tube containing the equilibrated Immobilized Papain. Incubation time of the digestion reaction was done for 16h on a tabletop rocker at 37°C. The column was centrifuged at 5000 x g for 1 minute to separate digest from the Immobilized Papain. Afterwards the resin was washed with 0.5ml PBS and centrifuged at 5000 x g for 1 minute. The wash fraction was added to the digested antibody that the total sample volume was 1.0ml. The NAb Protein A Column was equilibrated with PBS and IgG Elution Buffer at room temperature. The column was centrifuged for 1 minute to remove storage solution (contains 0.02% sodium azide) and equilibrated by adding 2ml of PBS, centrifuge again for 1 minute and the flow-through discarded. The sample was applied to the column and resuspended by inversion. Incubation was done at room temperature with end-over-end mixing for 10 minutes. The column was centrifuged for 1 minute, saving the flow-through with the Fab fragments.

(References: Coulter, A. and Harris, R. (1983). J. Immunol. Meth. 59, 199-203.; Lindner I. et al. (2010) {alpha} 2-Macroglobulin inhibits the malignant properties of astrocytoma cells by impeding {beta}-catenin signaling. Cancer Res. 70, 277-87.; Kaufmann B. et al. (2010) Neutralization of West Nile virus by cross-linking of its surface proteins with Fab fragments of the human monoclonal antibody CR4354. PNAS. 107, 18950-5.; Chen X. et al. (2010) Requirement of open headpiece conformation for activation of leukocyte integrin $\alpha x\beta 2$. PNAS. 107, 14727-32.; Uysal H. et al. (2009) Structure and pathogenicity of antibodies specific for citrullinated collagen type II in experimental arthitis. J. Exp. Med. 206, 449-62.; Thomas G. M. et al. (2009) Cancer cell-derived microparticles bearing P-selectin glycoprotein ligand 1 accelerate thrombus formation in vivo. J. Exp. Med. 206, 1913-27.; Kong F. et al. (2009) Demonstration of catch bonds between an integrin and its ligand. J. Cell Biol. 185, 1275-84.)

Procedure for generation and purification of F(ab')$_2$ Fragments:

The immobilized Pepsin was equilibrated by washing the resin with 0.5 ml of Digestion Buffer and centrifuging the column at 5000 x g for 1 minute. The buffer was discarded afterwards. The desalting column was prepared by removing the storage solution and washing it with digestion buffer, centrifuging it each time afterwards at 1000 x g for 2 minutes. 0.5ml of the prepared IgG sample where added to the spin column tube containing the equilibrated Immobilized Pepsin. Incubation time of the digestion reaction was done for 16h on a tabletop rocker at 37°C. The column was centrifuged at 5000 x g for 1 minute to separate digest from the Immobilized Papain. Afterwards the resin was washed with 0.5mL PBS and centrifuged at 5000 x g for 1 minute. The wash fraction was added to the digested antibody that the total sample volume was 1.0ml. The NAb Protein A Column was equilibrated with PBS and IgG Elution Buffer at room temperature. The column was centrifuged for 1 minute to remove storage solution (contains 0.02% sodium azide) and equilibrated by adding 2mL of PBS, centrifuge again for 1 minute and the flow-through discarded. The sample was applied to the column and resuspended by inversion. Incubation was done at room temperature with end-over-end mixing for 10 minutes. The column was centrifuged for 1 minute, saving the flow-through with the Fab fragments.

(References: Mariani, M., et al. (1991). A new enzymatic method to obtain high-yield F(ab')2 suitable for clinical use from mouse IgG1. Mol.Immunol. 28: 69-77.;Beale, D. (1987). Molecular fragmentation: Some applications in immunology. Exp Comp Immunol 11:287-96.; Ellerson, J.R., et al. (1972). A fragment corresponding to the CH2 region of immunoglobulin G (IgG) with complement fixing activity. FEBS Letters 24(3):318-22.; Kerbel, R.S. and El-liot, B.E. (1983).

Detection of Fc receptors. Meth Enzymol 93:113-147.; Kulkarni, P.N., et al. (1985). Conjugation of methotrexate to IgG antibodies and their F(ab')2 fragments and the effect of conjugated methotrexate on tumor growth in vivo. Cancer Immunol Immunotherapy 19:211-4.; Lamoyi, E. (1986). Preparation of F(ab')2 Fragments from mouse IgG of various subclasses. Meth Enzymol 121:652-663.; Parham, P., et al. (1982). Monoclonal antibodies: purification, fragmentation and application to structural and functional studies of class I MHC antigens. J Immunol Meth 53:133-73.; Raychaudhuri, G., et al. (1985). Human IgG1 and its Fc fragment bind with different affinities to the Fc receptors on the human U937, HL-60 and ML-1 cell lines. Mol Immunol 22(9):1009-19.; Rousseaux, J., et al. (1980). The differential enzyme sensitivity of rat immunoglobulin G subclasses to papain an pepsin. Mol Immunol 17:469-82.; Rousseaux, J., et al. (1983). Optimal condition for the preparation of Fab and F(ab')2 fragments from monoclonal IgG of different rat IgG subclasses. J Immunol Meth 64:141-6.; Wilson, K.M., et al. (1991). Rapid whole blood assay for HIV-1 seropositivity using an Fab-peptide conjugate. J Immunol Meth 138:111-9.)

NT-H-Antibody Fragment Humanization

The antibody fragment was humanized by the CDR-grafting method (Jones, P. T., Dear, P. H., Foote, J., Neuberger, M. S., and Winter, G. (1986) Replacing the complementarity-determining regions in a human antibody with those from a mouse. Nature 321, 522-525).

The following steps where done to achieve the humanized sequence:

Total RNA extraction: Total RNA was extracted from NT-H hybridomas using the Qiagen kit.

First-round RT-PCR: QIAGEN® OneStep RT-PCR Kit (Cat No. 210210) was used. RT-PCR was performed with primer sets specific for the heavy and light chains. For each RNA sample, 12 individual heavy chain and 11 light chain RT-PCR reactions were set up using degenerate forward primer mixtures covering the leader sequences of variable regions. Reverse primers are located in the constant regions of heavy and light chains. No restriction sites were engineered into the primers.

Reaction Setup: 5x QIAGEN® OneStep RT-PCR Buffer 5.0 μl, dNTP Mix (containing 10 mM of each dNTP) 0.8 μl, Primer set 0.5 μl, QIAGEN® OneStep RT-PCR Enzyme Mix 0.8 μl, Template RNA 2.0 μl, RNase-free water to 20.0 μl, Total volume 20.0 μl

PCR condition: Reverse transcription: 50°C, 30 min; Initial PCR activation: 95°C, 15 min

Cycling: 20 cycles of 94°C, 25 sec; 54°C, 30 sec; 72°C, 30 sec; Final extension: 72°C, 10 min

Second-round semi-nested PCR: The RT-PCR products from the first-round reactions were further amplified in the second-round PCR. 12 individual heavy chain and 11 light chain RT-PCR reactions were set up using semi-nested primer sets specific for antibody variable regions.

Reaction Setup: 2x PCR mix 10 μl; Primer set 2 μl; First-round PCR product 8 μl; Total volume 20 μl; Hybridoma Antibody Cloning Report

PCR condition: Initial denaturing of 5 min at 95°C; 25 cycles of 95°C for 25 sec, 57°C for 30 sec, 68°C for 30 sec; Final extension is 10 min b8°C.

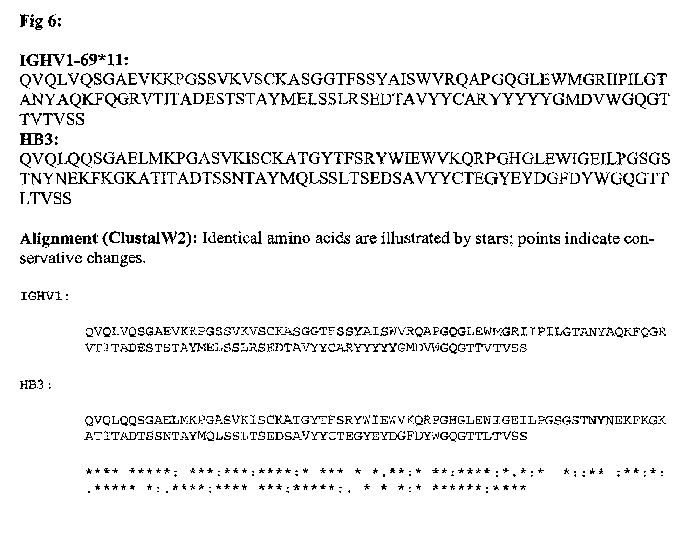

After PCR is finished, run PCR reaction samples onto agarose gel to visualize DNA fragments amplified.After sequencing more than 15 cloned DNA fragments amplified by nested RT-PCR, several mouse antibody heavy and light chains have been cloned and appear correct. Protein sequence alignment and CDR analysis identifies one heavy chain and one light chain. After alignment with homologous human framework sequences the resulting humanized sequence for the variable heavy chain is the following: see figure 6 (As the amino acids on positions 26, 40 and 55 in the variable heavy chain and amino acid on position 40 in the variable light are critical to the binding properties, they may be reverted to the murine original. The resulting candidates are depicted below) (Padlan, E. A. (1991) A possible procedure for reducing the immunogenicity of antibody variable domains while preserving their ligand-binding properties. Mol. Immunol. 28, 489-498.; Harris, L. and Bajorath, J. (1995) Profiles for the analysis of immunoglobulin sequences: Comparison of V gene subgroups. Protein Sci. 4, 306-310.).

Annotation for the antibody fragment sequences (SEQ ID NO: 7-14): bold and underline are the CDR 1, 2, 3 in chronologically arranged; italic are constant regions; hinge regions are highlighted with bold letters and the histidine tag with bold and italic letters; framework point mutation have a grey letter-background.

SEQ ID NO: 7 (AM-VH-C)

QVQLQQSGAELMKPGASVKISCKAT**GYTFSRYW**IEWVKQRPGHGLEWIGE**ILPGSG ST**NYNEKFKGKATITADTSS*NTAYMQLSSLTSEDSAVYYC**TEGYEYDGFDY**WGQGTTLT VSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKRV*EPK*HHHHHH*

SEQ ID NO: 8 (AM-VH1)

QVQLVQSGAEVKKPGSSVKVSCKAS**GYTFSRYW**ISWVRQAPGQGLEWMGR**ILPGS
GST**NYAQKFQGRVTITADES*TSTAYMELSSLRSEDTAVYYC**TEGYEYDGFD**YWGQGTTV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRV**EPK*HHHHHH*

SEQ ID NO: 9 (AM-VH2.-E40)

QVQLVQSGAEVKKPGSSVKVSCKAS**GYTFSRYW**IEWVRQAPGQGLEWMGR**ILPGS
GST**NYAQKFQGRVTITADES*TSTAYMELSSLRSEDTAVYYC**TEGYEYDGFD**YWGQGTTV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRV**EPK*HHHHHH*

SEQ ID NO: 10 (AM-VH3-T26-E55)

QVQLVQSGAEVKKPGSSVKVSCKA**TGYTFSRYW**ISWVRQAPGQGLEWMG**EILPGS
GST**NYAQKFQGRVTITADES*TSTAYMELSSLRSEDTAVYYC**TEGYEYDGFD**YWGQGTTV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRV**EPK*HHHHHH*

SEQ ID NO: 11 (AM-VH4-T26-E40-E55)

QVQLVQSGAEVKKPGSSVKVSCKA**TGYTFSRYW**IEWVRQAPGQGLEWMG**EILPGS
GST**NYAQKFQGRVTITADES*TSTAYMELSSLRSEDTAVYYC**TEGYEYDGFD**YWGQGTTV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRV**EPK*HHHHHH*

SEQ ID NO: 12 (AM-VL-C)

DVLLSQTPLSLPVSLGDQATISCRSS**QSIVYSNGNTY**LEWYLQKPGQSPKLLIY**RVSN**
RFSGVPDRFSGSGSGTDFTLKISRVEAEDLGVYYC**FQGSHIPYT**FGGGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

SEQ ID NO: 13 (AM-VL1)

DVVMTQSPLSLPVTLGQPASISCRSS**QSIVYSNGNTY**LNWFQQRPGQSPRRLIY**RVSN**
RDSGVPDRFSGSGSGTDFTLKISRVEAEDVGVYYC**FQGSHIPYT**FGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

SEQ ID NO: 14 (AM-VL2-E40)

DVVMTQSPLSLPVTLGQPASISCRSS**QSIVYSNGNTY**LEWFQQRPGQSPRRLIY**RVS**N
RDSGVPDRFSGSGSGTDFTLKISRVEAEDVGVYYC**FQGSHIPYT**FGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

**Example 2**

**Effect of selected anti-ADM-antibodies on anti-ADM-bioactivity**

The effect of selected ADM-antibodies on ADM-bioactivity was tested in an human recombinant Adrenomedullin receptor cAMP functional assay (Adrenomedullin Bioassay).

**Testing of antibodies targeting human or mouse adrenomedullin in human recombinant Adrenomedullin receptor cAMP functional assay (Adrenomedullin Bioassay)**

Materials:

    Cell line: CHO-K1
    Receptor: Adrenomedullin (CRLR + RAMP3)
    Receptor Accession Number Cell line: CRLR: U17473; RAMP3: AJ001016

CHO-K1 cells expressing human recombinant adrenomedullin receptor (FAST-027C) grown prior to the test in media without antibiotic were detached by gentle flushing with PBS-EDTA (5 mM EDTA), recovered by centrifugation and resuspended in assay buffer (KRH: 5 mM KCl, 1.25 mM MgSO4, 124 mM NaCl, 25 mM HEPES, 13.3 mM Glucose, 1.25 mM KH2PO4, 1.45 mM CaCl2, 0.5 g/l BSA).
Dose response curves were performed in parallel with the reference agonists (hADM or mADM).

Antagonist test (96well):

For antagonist testing, 6 μl of the reference agonist (human (5,63nM) or mouse (0,67nM) adrenomedullin) was mixed with 6 μl of the test samples at different antagonist dilutions; or with 6 μl buffer. After incubation for 60 min at room temperature, 12 μl of cells (2,500 cells/well) were added. The plates were incubated for 30 min at room temperature. After addition of the lysis buffer, percentage of DeltaF will be estimated, according to the manufacturer specification, with the HTRF kit from Cis-Bio International (cat n°62AM2 PEB). hADM 22-52 was used as reference antagonist.

**Antibodies testing cAMP-HTRF assay**

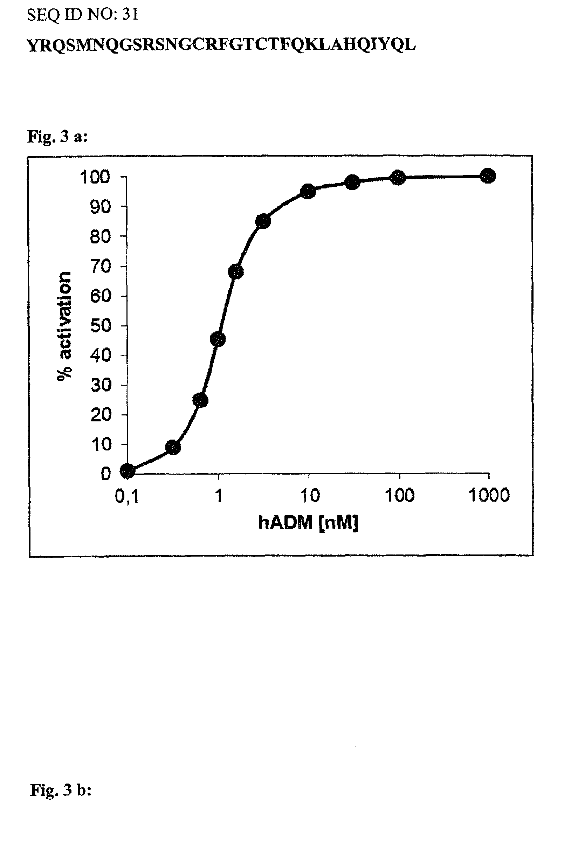

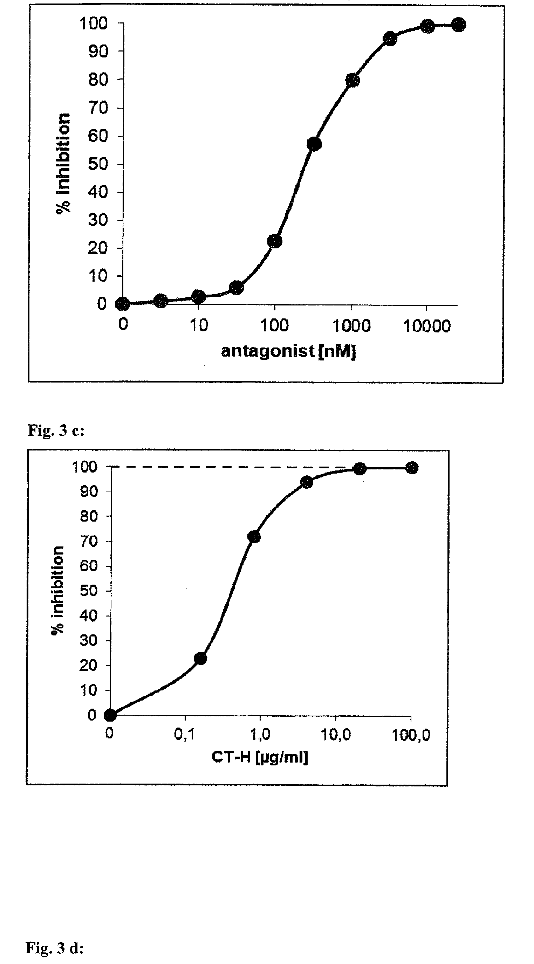

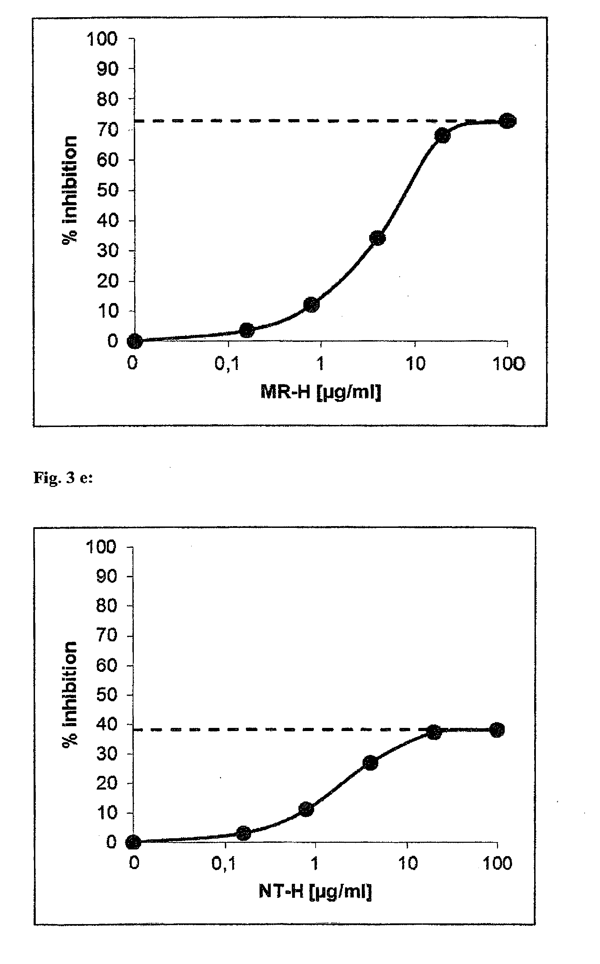

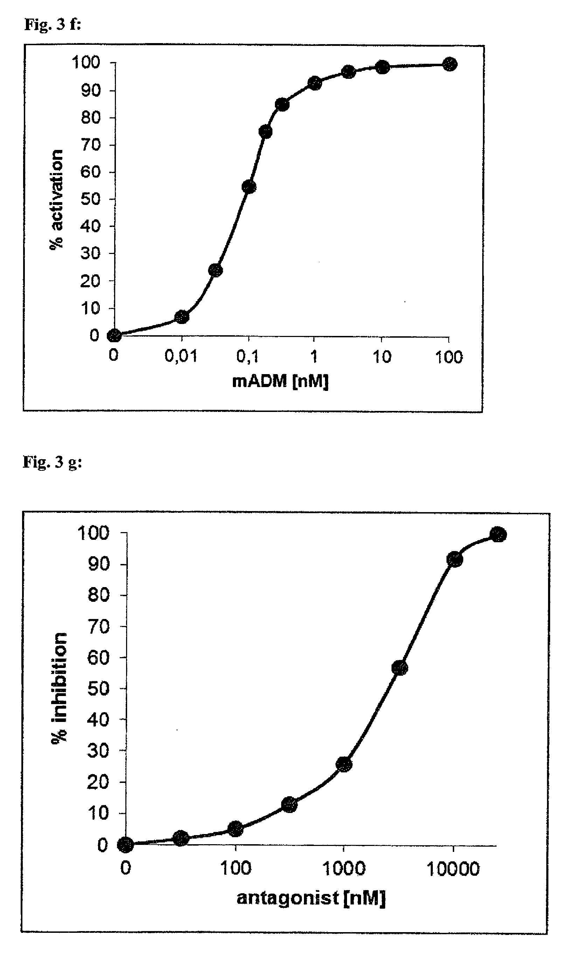

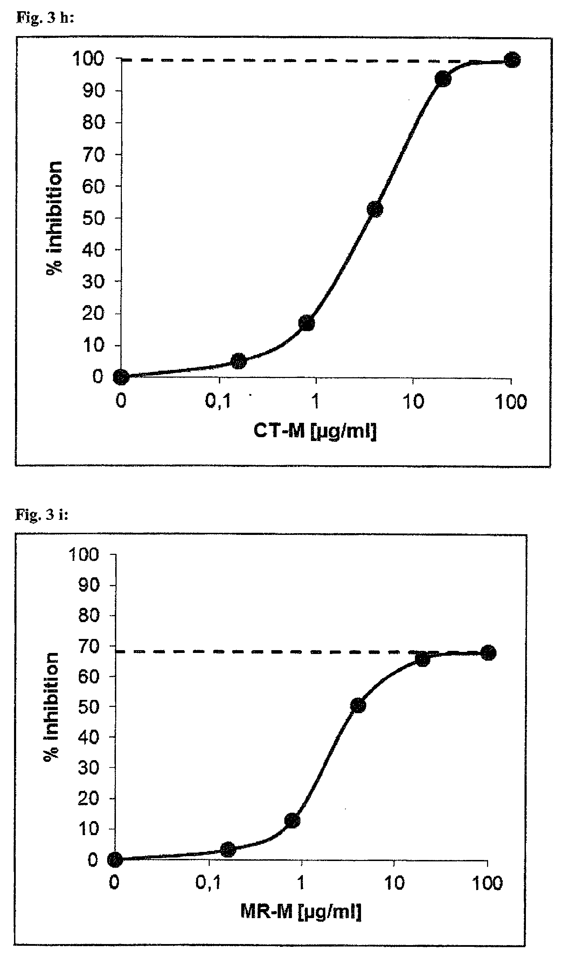

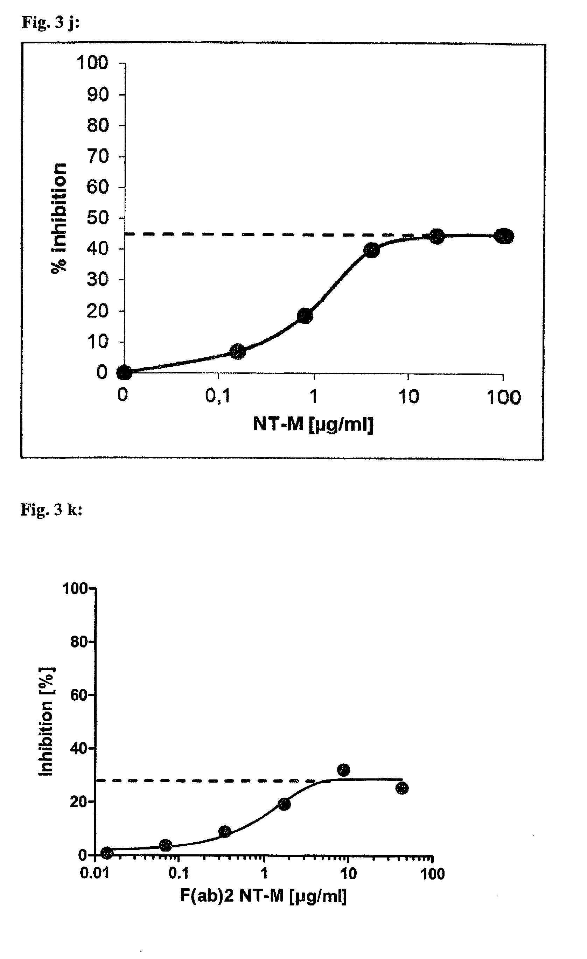

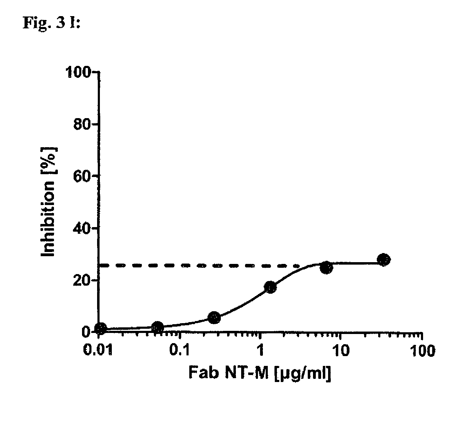

The anti-h-ADM antibodies (NT-H, MR-H, CT-H) were tested for antagonist activity in human recombinant adrenomedullin receptor (FAST-027C) cAMP functional assay in the presence of 5.63nM Human ADM 1-52, at the following final antibody concentrations: 100μg/ml, 20μg/ml, 4μg/ml, 0.8μg/ml, 0.16μg/ml.
The anti-m-ADM antibodies (NT-M, MR-M, CT-M) were tested for antagonist activity in human recombinant adrenomedullin receptor (FAST-027C) cAMP functional assay in the presence of 0.67nM Mouse ADM 1-50, at the following final antibody concentrations: 100μg/ml, 20μg/ml, 4μg/ml, 0.8μg/ml, 0.16μg/ml. Data were plotted relative inhibition vs. antagonist concentration (see figs. 3a to 3l). The maximal inhibition by the individual antibody is given in table 3.

**Table 3:**

| Antibody | Maximal inhibition of ADM bioactivity (ADM-Bioassay) (%) |
|----------|-----------------------------------------------------------|
| NT-H | 38 |
| MR-H | 73 |
| CT-H | 100 |
| NT-M FAB | 26 |

(continued)

| Antibody | Maximal inhibition of ADM bioactivity (ADM-Bioassay) (%) |
|---|---|
| NT-M FAB2 | 28 |
| NT-M | 45 |
| MR-M | 66 |
| CT-M | 100 |
| Non specific mouse IgG | 0 |

**Example 3**

**Data for stabilization of hADM by the anti-ADM antibody**

The stabilizing effect of human ADM by human ADM antibodies was tested using a hADM immunoassay.

**Immunoassay for the quantification of human Adrenomedullin**

The technology used was a sandwich coated tube luminescence immunoassay, based on Acridinium ester labelling.

**Labelled compound (tracer):** 100µg (100ul) CT-H (1mg/ ml in PBS, pH 7.4, AdrenoMed AGGermany) was mixed with 10µl Acridinium NHS-ester (1mg/ ml in acetonitrile, InVent GmbH, Germany) (EP 0353971) and incubated for 20min at room temperature. Labelled CT-H was purified by Gel-filtration HPLC on Bio-Sil® SEC 400-5 (Bio-Rad Laboratories, Inc., USA) The purified CT-H was diluted in (300 mmol/L potassiumphosphate, 100 mmol/L NaCl, 10 mmol/L Na-EDTA, 5 g/L Bovine Serum Albumin, pH 7.0). The final concentration was approx. 800.000 relative light units (RLU) of labelled compound (approx. 20ng labeled antibody) per 200 µL. Acridiniumester chemiluminescence was measured by using an AutoLumat LB 953 (Berthold Technologies GmbH & Co. KG).

**Solid phase:** Polystyrene tubes (Greiner Bio-One International AG, Austria) were coated (18h at room temperature) with MR-H (AdrenoMed AG, Germany) (1.5 µg MR-H/0.3 mL 100 mmol/L NaCl, 50 mmol/L TRIS/HCl, pH 7.8). After blocking with 5% bovine serum albumine, the tubes were washed with PBS, pH 7.4 and vacuum dried.

**Calibration:**

The assay was calibrated, using dilutions of hADM
(BACHEM AG, Switzerland) in 250 mmol/L NaCl, 2 g/L Triton X-100, 50 g/L Bovine Serum Albumin, 20 tabs/L Protease Inhibitor Cocktail (Roche Diagnostics AG, Switzerland))

**hADM Immunoassay:**

50 µl of sample (or calibrator) was pipetted into coated tubes, after adding labeleld CT-H (200µl), the tubes were incubated for 4h at 4°C. Unbound tracer was removed by washing 5 times (each 1ml) with washing solution (20mM PBS, pH 7.4, 0.1 % Triton X-100).

**Tube-bound chemiluminescence was measured by using the LB 953**

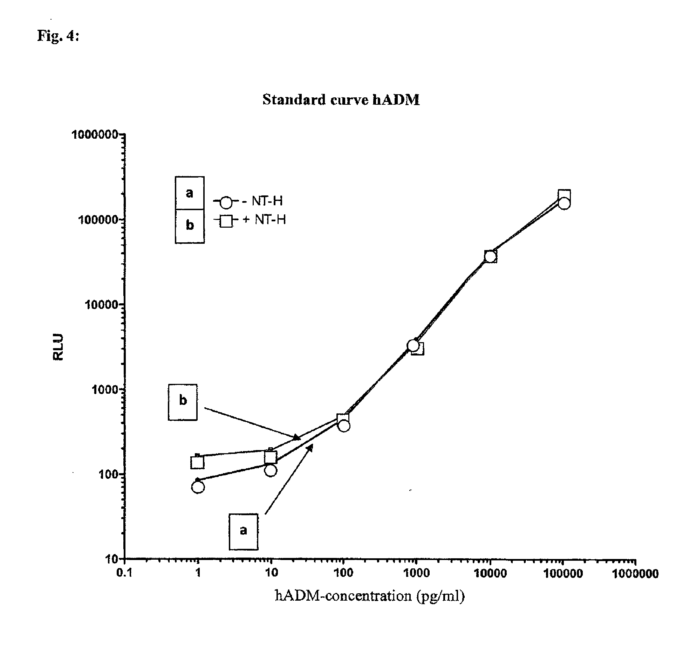

Figure 4 shows a typical hADM dose/ signal curve. And an hADM dose signal curve in the presence of 100 µg/mL antibody NT-H.
NT-H did not affect the described hADM immunoassay.

**Stability of human Adrenomedullin:**

Human ADM was diluted in human Citrate plasma (final concentration 10nM) and incubated at 24 °C. At selected time points, the degradation of hADM was stopped by freezing at -20 °C. The incubation was performed in absence and presence of NT-H (100µg/ml). The remaining hADM was quantified by using the hADM immunoassay described above.

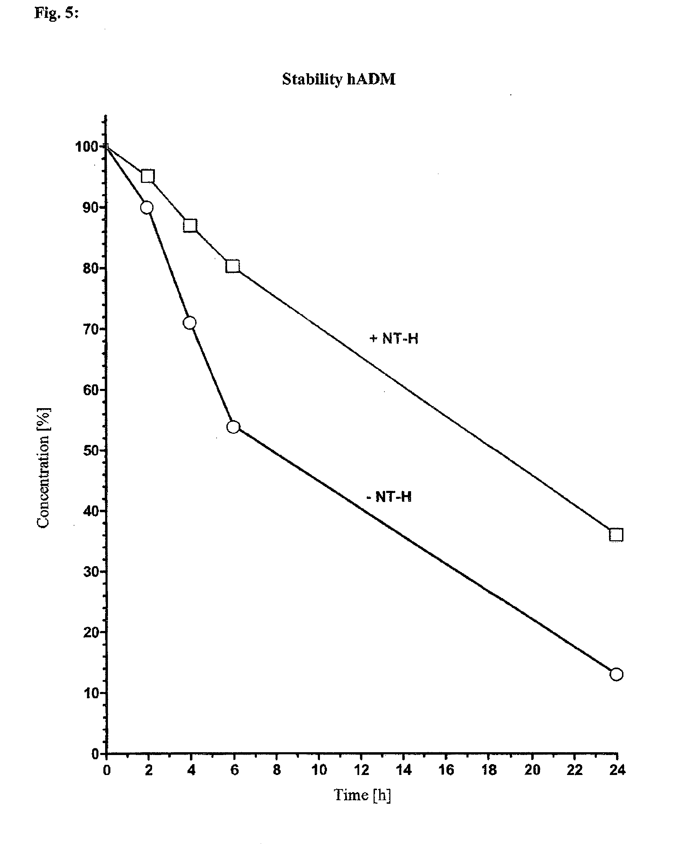

Figure 5 shows the stability of hADM in human plasma (citrate) in absence and in the presence of NT-H antibody. The half life of hADM alone was 7,8h and in the presence of NT-H, the half life was 18,3h. (2.3 times higher stability).

**Example 4**

**Sepsis Mortality (early treatment)**

**Animal model**

12-15 week old male C57B1/6 mice (Charles River Laboratories, Germany) were used for the study. Peritonitis had been surgically induced under light isofluran anesthesia. Incisions were made into the left upper quadrant of the peritoneal cavity (normal location of the cecum). The cecum was exposed and a tight ligature was placed around the cecum with sutures distal to the insertion of the small bowel. One puncture wound was made with a 24-gauge needle into the cecum and small amounts of cecal contents were expressed through the wound. The cecum was replaced into the peritoneal cavity and the laparotomy site was closed. Finally, animals were returned to their cages with free access to food and water. 500 $\mu$l saline were given s.c, as fluid replacement.

**Application and dosage of the compound (NT-M, MR-M, CT-M)**

Mice were treated immediately after CLP (early treatment). CLP is the abbreviation for cecal ligation and puncture (CLP).

**Study groups**

Three compounds were tested versus: vehicle and versus control compound treatment. Each group contained 5 mice for blood drawing after 1 day for BUN (serum blood urea nitrogen test) determination. Ten further mice per each group were followed over a period of 4 days. Group Treatment (10 $\mu$l/ g bodyweight) dose/ Follow-Up:

1 NT-M, 0.2 mg/ml survival over 4 days
2 MR-M, 0.2 mg/ml survival over 4 days
3 CT-M, 0.2 mg/ml survival over 4 days
4 non-specific mouse IgG, 0.2 mg/ml survival over 4 days
5 control - PBS 10 $\mu$l/g bodyweight survival over 4 days

**Clinical chemistry**

Blood urea nitrogen (BUN) concentrations for renal function were measured baseline and day 1 after CLP. Blood samples were obtained from the cavernous sinus with a capillary under light ether anaesthesia. Measurements were performed by using an AU 400 Olympus Multianalyser. The 4-day mortality is given in table 4. The average BUN concentrations are given in table 5.

**Table 4:**

| 4 day mortality | survival (%) |
|---|---|
| PBS | 0 |
| non-specific mouse IgG | 0 |
| CT-M | 10 |
| MR-M | 30 |
| NT-M | 70 |

**Table 5:**

| Average from 5 animals | BUN pre CLP (nM) | BUN day 1 (mM) |
|---|---|---|
| PBS | 8.0 | 23.2 |
| non-specific mouse IgG | 7.9 | 15.5 |
| CT-M | 7.8 | 13.5 |

(continued)

| Average from 5 animals | BUN pre CLP (nM) | BUN day 1 (mM) |
|---|---|---|
| MR-M | 8.1 | 24.9 |
| NT-M | 8.8 | 8.2 |

It can be seen from Table 4 that the NT-M antibody reduced mortality considerably. After 4 days 70 % of the mice survived when treated with NT-M antibody. When treated with MR-M antibody 30 % of the animals survived and when treated with CT-M antibody 10 % of the animals survived after 4 days. In contrast thereto all mice were dead after 4 days when treated with unspecific mouse IgG. The same result was obtained in the control group where PBS (phosphate buffered saline) was administered to mice.

The blood urea nitrogen or BUN test is used to evaluate kidney function, to help diagnose kidney disease, and to monitor patients with acute or chronic kidney dysfunction or failure. The results of the S-BUN Test revealed that the NT-M antibody was the most effective to protect the kidney.

**Sepsis Mortality (late treatment)**

**Animal model**

12-15 week old male C57B1/6 mice (Charles River Laboratories, Germany) were used for the study. Peritonitis had been surgically induced under light isofluran anesthesia. Incisions were made into the left upper quadrant of the peritoneal cavity (normal location of the cecum). The cecum was exposed and a tight ligature was placed around the cecum with sutures distal to the insertion of the small bowel. One puncture wound was made with a 24-gauge needle into the cecum and small amounts of cecal contents were expressed through the wound. The cecum was replaced into the peritoneal cavity and the laparotomy site was closed. Finally, animals were returned to their cages with free access to food and water. 500µl saline were given s.c. as fluid replacement.

**Application and dosage of the compound (NT-M FAB2)**

NT-M FAB2 was tested versus: vehicle and versus control compound treatment. Treatment was performed after full development of sepsis, 6 hours after CLP (late treatment). Each group contained 4 mice and were followed over a period of 4 days.

Group Treatment (10µl/ g bodyweight) dose/ Follow-Up:

**Study groups**

1 NT-M, FAB2 0.2 mg/ml survival over 4 days
2 control: non-specific mouse IgG, 0.2 mg/ml survival over 4 days
3 vehicle: - PBS 10µl/g bodyweight survival over 4 days

**Table 6:**

| 4 day mortality | survival (%) |
|---|---|
| PBS | 0 |
| Non-specific mouse IgG | 0 |
| NT-M FAB2 | 75 |

It can be seen from Table 6 that the NT-M FAB 2 antibody reduced mortality considerably. After 4 days 75 % of the mice survived when treated with NT-M FAB 2 antibody. In contrast thereto all mice were dead after 4 days when treated with non-specific mouse IgG. The same result was obtained in the control group where PBS (phosphate buffered saline) was administered to mice.

**Example 5**

**Incremental effect of anti-ADM antibody in CLP-animals on top of antibiotic** treatment **and circulation stabilization via catecholamines as well as regulation of fluid balance.**

**Animal model**

In this study male C57B1/6 mice (8-12 weeks, 22-30g) were utilized. A polymicrobial sepsis induced by cecal ligation and puncture (CLP) was used as the model for studying septic shock ((Albuszies G, et al: Effect of increased cardiac output on hepatic and intestinal microcirculatory blood flow, oxygenation, and metabolism in hyperdynamic murine septic shock. Crit Care Med 2005;33:2332-8), (Albuszies G, et al: The effect of iNOS deletion on hepatic gluconeogenesis in hyperdynamic murine septic shock. Intensive Care Med 2007;33:1094-101), (Barth E, et al: Role of iNOS in the reduced responsiveness of the myocardium to catecholamines in a hyperdynamic, murine model of septic shock. Crit Care Med 2006;34:307-13), (Baumgart K, et al: Effect of SOD-1 over-expression on myocardial function during resuscitated murine septic shock. Intensive Care Med 2009;35:344-9),
(Baumgart K, et al: Cardiac and metabolic effects of hypothermia and inhaled H2S in anesthetized and ventilated mice. Crit Care Med 2010;38:588-95), (Simkova V, et al: The effect of SOD-1 over-expression on hepatic gluconeogenesis and whole-body glucose oxidation during resuscitated, normotensive murine septic shock. Shock 2008;30:578-84), (Wagner F, et al.: Inflammatory effects of hypothermia and inhaled H2S during resuscitated, hyperdynamic murine septic shock. Shock, im Druck), (Wagner F, et al: Effects of intravenous H2S after murine blunt chest trauma: a prospective, randomized controlled trial. Crit Care 2011, submitted for publication)).

After weighing, mice were anesthetized by intraperitoneal injection of 120 $\mu$g/g Ketamin, 1.25 $\mu$g/g Midazolam and 0.25 $\mu$g/g Fentanyl. During the surgical procedure, body temperature was kept at 37-38°C. A 1 cm midline abdominal section was performed to get access to the cecum. The cecum then was ligated with 3-0 silk tie close to the basis and a single puncture with a 18-gauge needle was applied. The cecum was returned and the incision was closed again (4-0 tie). For the compensation of perioperative loss of liquids, 0.5 ml lacted Ringer's solution with 1 $\mu$g/g Buprenorphin as analgetic was injected subcutaneously in dorsal dermis. For antibiosis the mice received Ceftriaxon 30$\mu$g/g and Clindamycin 30$\mu$g/g subcutaneously via the lower extremities.

After CLP surgery the animal were kept in an adequately heated environment with water and food ad libitum.

The covering of liquid requirements were ensured by a dorsal subcutaneous injections with 0.5 ml lactated ringer's solution with 4 $\mu$g/g glucose and Buprenorphin 1$\mu$g/g, which were applied in an 8 hour cycle, after short term anesthesia by isofluran. In addition, antibiosis was maintained by subcutaneous injections of Ceftriaxon 30$\mu$g/g and Clindamycin 30$\mu$g/g via the lower extremities.

**Dosing of test substances**

**Early treatment**

Immediately after the CLP surgery and closing of the incision, the test substance antibody NT-M was applied in a concentration of 500 $\mu$g/ml in phosphate buffered saline (PBS) via injection into the penis vein for a dose of 2mg per kg body weight (dose volume 88-120 $\mu$l) (5 animals).

**Late treatment**

After full Sepsis development, 15.5h after CLP surgery, animals were anesthetized as described above and NT-M was applied in a concentration of 500 $\mu$g/ml in phosphate buffered saline (PBS) via injection into the penis vein for a dose of 2mg per kg body weight (dose volume 88-120 $\mu$l) (3 animals).

The control group (6 animals) received a corresponding amount of the vehicle PBS solution without antibody (4$\mu$l/g, 88-120 $\mu$l) immediately after CLP surgery.

**Study groups and experimental setting**

Murine septic shock model under intensive care monitoring:

Three groups with 3, 5 and 6 animals were monitored. Group 1 (5 animals) received the antibody NT-M 15.5h after CLP, group 2 received the antibody NT-M immediately after CLP surgery and group 3 received a comparable amount of PBS (4$\mu$l/g). 16 hour incubation post CLP (to allow the polymicrobial sepsis to progress), the experiment was continued with monitoring and interventions comparable to an intensive medical care regime. Therefore, after weighing the animals were anesthetized as described in the CLP surgery part (except the late treated animals, which were anesthized before treatment). Body temperature was maintained at 37-38°C for the rest of the experiment. After a tracheotomy and intubation, respiration was monitored and supported by laboratory animal lung ventilator Flexivent®, (Emka Technologies, FiO2 0,5, PEEP 10 H2O, VT 8µl/g, I:E 1: 1,5, AF 70-140 depending on temperature).

Anesthesia was maintained throughout the experiment via the cannulated vena jugularis externa dextra with a continuous infusion of Ketamin 30 µg/gxh and Fentanyl 0.3 µg/gxh. Furthermore, the right aorta carotis communis was cannulated for continuous monitoring of heart rate and the mean arterial pressure (MAP). The mean arterial pressure was maintained at MAP > 65 mmHg via intravenous (V. jugularis) infusion of colloids (80 µL/gxh, Hextend®) and, if needed, Noradrenalin dissolved in colloids as vasopressor. Blood samples (120 µl) were taken via the cannulated A. carotis at 0 and 4 hours for determination of creatinine. The bladder was punctured and urine was collected via a bladder catheter. The experiment was either terminated after 6 hours or prior to this, if the MAP > 65 mmHg (V. jugularis) could not be maintained with the vasorpressor dosing.

**Measured parameters**

The following parameters were measured and analyzed: Total consumption of noradrenalin (µg NA/g), consumption rate of noradrenalin (µg NA/g/h), total volume of urine collected during the experiment, creatinine concentration (µg/mL) at the end of the experiment and mean creatinine clearance (µL/min).

**Table 7:**

|  | Total consumption of Noradrenalin (µg NA/g) (Average) | consumption rate of Noradrenalin (µg NA/g/h) (Average) |
|---|---|---|
| Control (mouse IgG) (N=6) | 0.17 µg/g | 0.032 µg/h/g |
| NT-M (N=5) early treatment | 0.07 µg/g | 0,012µg/h/g |
| Relative change (early treatment, amelioration) | 59% (59%) | 62.5% (62.5%) |
| NT-M (N=3) late treatment | 0.04 µg/g | 0.0075 µg/h/g |
| Relative change (late treatment, amelioration) | 76,5% (76.5%) | 76,5% (76.5%) |

The catecholamine requirement was measured after administration of either non specific mouse IgG to a total of 6 mice as control group, NT-murine antibody to a group of 5 mice immediately after CLP (early treatment) or NT-murine antibody to a group of 3 mice 15.5h after CLP (late treatment).

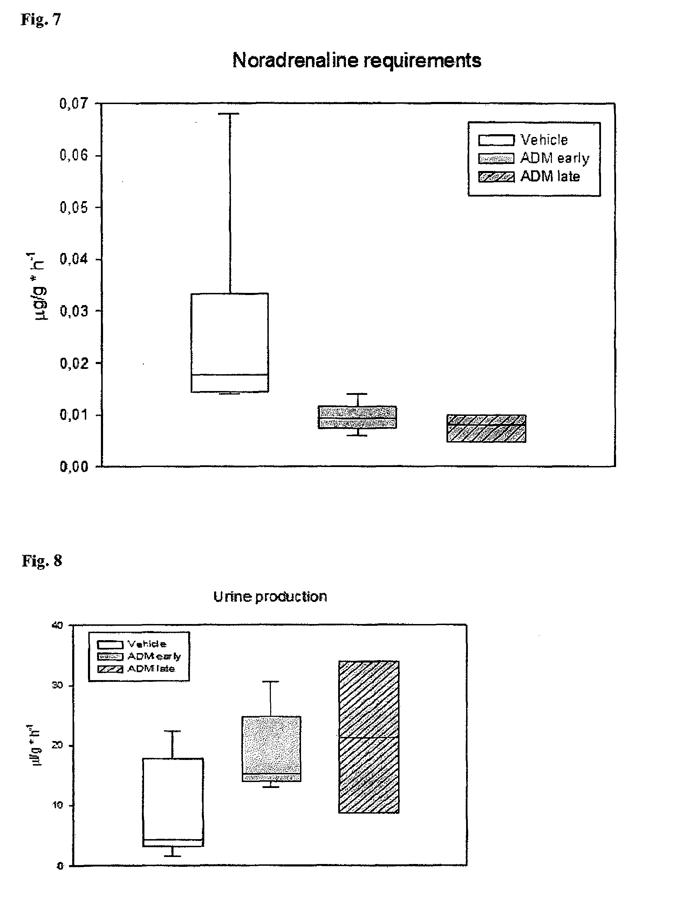

The reduction of the catecholamine requirement is a measure for the stabilization of the circulation. Thus, the data show that the ADM antibody, especially the NT-M antibody, leads to a considerable stabilization of the circulation and to a considerable reduction of the catecholamine requirement. The circulation-stabilizing effect was given in early treatment (immediately after CLP) and treatment after full sepsis development (late treatment) (see fig. 7).

**Regulation of Fluid Balance**

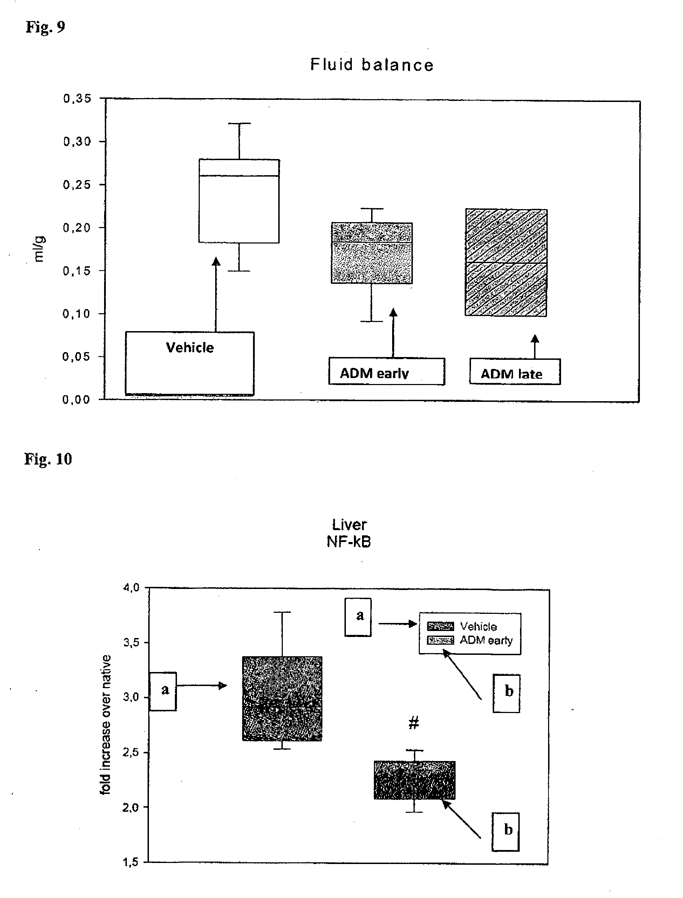

More positive fluid balance both early in resuscitation and cumulatively over 4 days is associated with an increased risk of mortality in septic shock. The control of the liquid balance is of utmost importance for the course of disease of patients having sepsis. (s. Boyd et al, 2011). Controlling the liquid balance of critical ill patients remains as a substantial challenge in intensive care medicine. As can be seen in table 8 treatment of mice after CLP (experimental procedures see "Animal Model") with NT-M antibody lead to an enhancement of the total volume of urine excreted. The urine secreted was approx, three times higher in NT-M-treated animals compared to non-treated mice. The positive treatment effect was given in early- and in late treatment. The fluid balance was improved by about 20-30%, also in both, early and late treatment. Thus, the data show that the use of ADM antibody, especially the use of NT ADM antibody, is favorable for regulating the fluid balance in patients. (see table 8 and figures 8 and 9).

**Table 8**

|  | **Urine** average volume/ g body weight | **Fluid balance** average volume/ g body weight |
|---|---|---|
| Control (mouse IgG) (N=6) | 0.042 ml/g | 0,23 ml/g |
| NT-M early (N=5) | 0.12 ml | 0,18ml/g |
| Relative change early treatment | + 186% | -21.7%% |
| NT-M late (N=3) | 0.125 ml | 0,16 ml/g |
| Relative change late treatment | + 198% | -30,5% |

**Improvement of kidney function**

The combination of acute renal failure and sepsis is associated with a 70 percent mortality, as compared with a 45 percent mortality among patients with acute renal failure alone. (Schrier and Wang, "Mechanisms of Disease Acute Renal Failure and Sepsis"; The New England Journal of Medicine; 351:159-69; 2004). Creatinine concentration and creatinine clearance are standard laboratory parameters for monitoring kidney (dys)function (Jacob, "Acute Renal Failure", Indian J. Anaesth.; 47 (5): 367-372; 2003). Creatinine and creatinine clearance data from above described animal experiment (early treatment) are given in Table 9.

**Table 9**

| Kidney function: | | |
|---|---|---|
|  | creatinine concentration ($\mu$g/mL) | mean creatinine clearance ($\mu$L/min) |
| control mouse IgG (MW) | 2.6 $\mu$g/ml | 174 $\mu$l/min |
| NT-M (MW) | 1.5 $\mu$g/ml | 373 $\mu$l/min |
| Relative change (amelioration) | -42%<br>(42%) | +114%<br>(114%) |

In comparision to control septic animals, the creatinine concentration was lowered by 42% and the creatinine clearance was improved by more than 100% as a result of NT-M treatment (Table 9). The data show that the administration of ADM-antibody, especially NT-M, leads to an improvement of kidney function.

**Improvement of liver inflammatory status**

Liver tissue for control and early treated animals was homogenized and lysed in lysing buffer. For cell extract preparation, cells were resuspended, lysed on ice, and centrifuged. The supernatant (protein extract) was stored at -80 °C. Activation of nuclear factor kappa-light-chain gene enhancer in B cells (NF-$\kappa$B) was determined as previously described using an electrophoretic mobility shift assay (EMSA)[1,2]. Cell extracts (10$\mu$g) were incubated on ice with poly-doxy-inosinic-deoxy-cytidylic acid (poly-dl-dC) and 32P-labeled double stranded oligonucleotide (Biomers, Ulm, Germany) containing the NF-$\kappa$B (HIV $\kappa$Bsite) (5'-GGATCCTCAACAGAGGGGACTTTCCGAGGCCA-3'). Complexes were separated in native polyacrylamide gels, dried and exposed to X-ray films. A phosphorimager and image analyzer software (AIDA Image Analyzer; Raytest) was used to quantify the radioactively labeled NF-$\kappa$B by densitometry. For comparison between individual gels, the intensity of each band was related to that of simultaneously loaded control animals which had not undergone surgical instrumentation and CLP. Therefore, the EMSA data are expressed as fold increase over control values. Statistics: All data are presented as median (range) unless otherwise stated differences between the two groups were analyzed with the Mann-Whitney rank sum test for unpaired samples. Results: The animals treated with NT-M presented with significantly attenuated liver tissue NF-$\kappa$B activation (2.27 (1.97-2.53)) compared to vehicle animals (2.92 (2.50-3.81)) ($p < 0.001$) (see figure 10).

**References:**

1. Wagner F, Wagner K, Weber S, Stahl B, Knöferl MW, Huber-Lang M, Seitz DH, Asfar P, Calzia E, Senftleben U, Gebhard F, Georgieff M, Radermacher P, Hysa V: Inflammatory effects of hypothermia and inhaled H2S during resuscitated, hyperdynamic murine septic shock. Shock 2011;35(4):396-402

2. Wagner F, Scheuerle A, Weber S, Stahl B, McCook O, Knöferl MW, Huber-Lang M, Seitz DH, Thomas J, Asfar P, Szabó C, Möller P, Gebhard F, Georgieff M, Calzia E, Radermacher P, Wagner K: Cardiopulmonary, histologic, and inflammatory effects of intravenous Na2S after blunt chest trauma-induced lung contusion in mice. J Trauma 2011;71(6):1659-67

## Example 6

### In vivo side effect determination of antibody NT-M

12-15 week old male C57B1/6 mice (Charles River Laboratories, Germany) were used for the study. 6 mice were treated with (10ul/g bodyweight) dose of NT-M, 0.2 mg/ml. As control, 6 mice were treated with (10 $\mu$l/g body weight) PBS. Survival and physical condition was monitored for 14 days. The mortality was 0 in both groups, there were no differences in physical condition between NT-M and control group.

## Example 7

### Gentamicin-induced nephrotoxicity

A non-septic acute kidney injury model has been established, which makes use of the nephrotoxicity induced by Gentamicin (Chiu PJS. Models used to assess renal functions. Drug Develop Res 32:247-255, 1994.). This model was used to assess whether treatment with anti-Adrenomedullin antibody can improve kidney function.

The experiment was performed as follows:

| Effect of a NT-M on Gentamicin-Induced Nephrotoxicity in Rats | | | | | | |
|---|---|---|---|---|---|---|
| **Study Design:** | | | | | | |
| | **Test** | | **Conc** | **Dosage** | | **Rats[d]** |
| Group | **Article** | **Route** | **mg/ml** | **ml/kg** | **mg/kg** | **(Male)** |
| 1 | Gentamicin[a] + vehicle[b] | IV | | | NA x 4[c] | 8 |
| 2 | Gentamicin[a] + NT-M | IV | | | X4[c] | 8 |
| [a]Gentamicin at 120 mg/kg intramuscularly for 7 days (days 0-6). | | | | | | |
| [b]Vehicle; injected intravenously (i.v.) 5 min before gentamicin on Day 0, followed by injections on Days 2, 4, and 6. [c]NT-M at 4 mg/kg was injected intravenously (i.v.) 5 min before gentamicin on Day 0, followed by 2 mg/kg i.v. on Days 2, 4, and 6. [d]Plasma samples were collected in EDTA tubes (Days 1 and 3 before Test and Control article: 100 $\mu$l; Day 7:120 $\mu$l. 24h urine collection on ice is initiated after gentamicin on Day 0, followed by Days 2 and 6; blood collection on days 1, 3, and 7. | | | | | | |

Groups of 8 male Sprague-Dawley rats weighing 250 $\pm$ 20 g were employed. Animals were challenged with gentamicin at 120 mg/kg i.m. for seven consecutive days (Groups 1 and 2). Test compound (anti-adrenomedullin antibody NT-M) and vehicle (phosphate buffered saline) were injected intravenously 5 min before gentamicin on day 0, followed by injection on days 2, 4, and 6. Body weights and clinical signs were monitored daily. Twenty-four (24) hour urine collections on ice were performed on Days 0, 2, and 6. Urine specimens were assayed for concentrations of Na+ and K+, and creatinine. Blood samples for clinical chemistry were collected on Days 1 (before gentamicin), 3 (before gentamicin), and 7. Serum electrolytes (Na+ and K+), creatinine, and BUN were the primary analytes that were monitored for assessing renal function. Plasma samples were collected in EDTA tubes (Days 1 and 3:100 $\mu$l; Day 7:120 $\mu$l). Creatinine clearance was calculated. Urine volume, urinary electrolytes, and creatinine are expressed as amount excreted per 100 g of animal body weight. All animals were sacrificed on Day 7. Kidneys were weighed.

Urine collection. The animals were placed in individual cages where urine was collected for 24 h on Day 0, Day 2, and Day 6. Urine volume, urinary Na+, K+, and creatinine were measured.

Endogenous creatinine clearance was calculated as follows:

CCr (ml/24 h) = [UCr (mg/ml) x V (ml/24 h)] / SCr (mg/ml)

24-hr urinary excretion of sodium (Na+) was calculated as follows:

UNaV ($\mu$Eq/24 h) = UNa ($\mu$Eq/ml) x V (ml/24 h)

24-hr urinary excretion of NAG and NGALwas similarly calculated.

The fractional excretion of Na$^+$ (FE$_{Na}$), or percentage of the filtered sodium that is excreted into the final urine, is a measure of tubular Na$^+$reabsorptive function. It was computed as follows:

$$FE_{Na}\ (\%) = 100 \times [U_{Na}\ (\mu Eq/ml) \times V\ (ml/24\ h)] / P_{Na}\ (\mu Eq/ml) \times C_{Cr}\ (ml/24\ h)$$

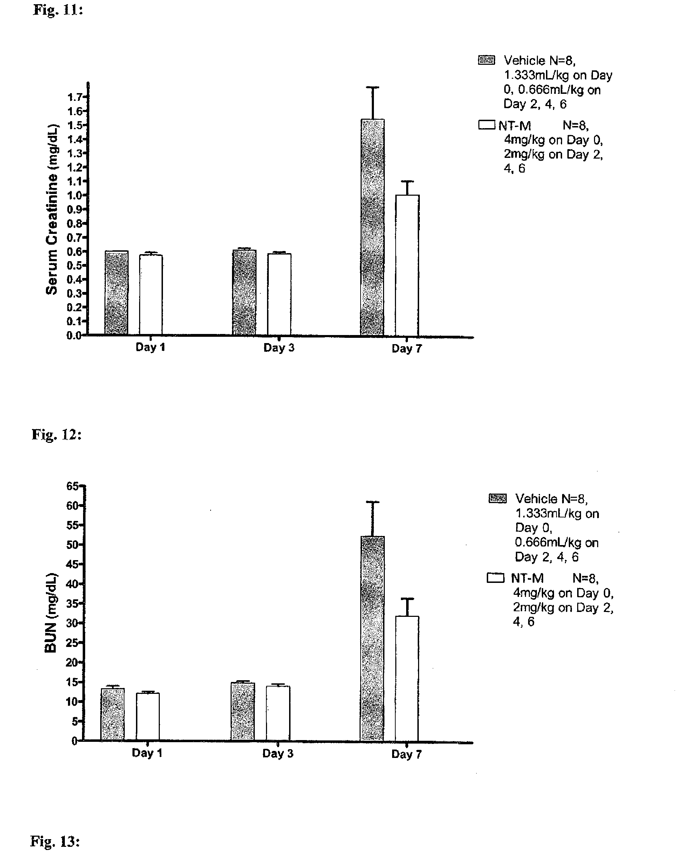

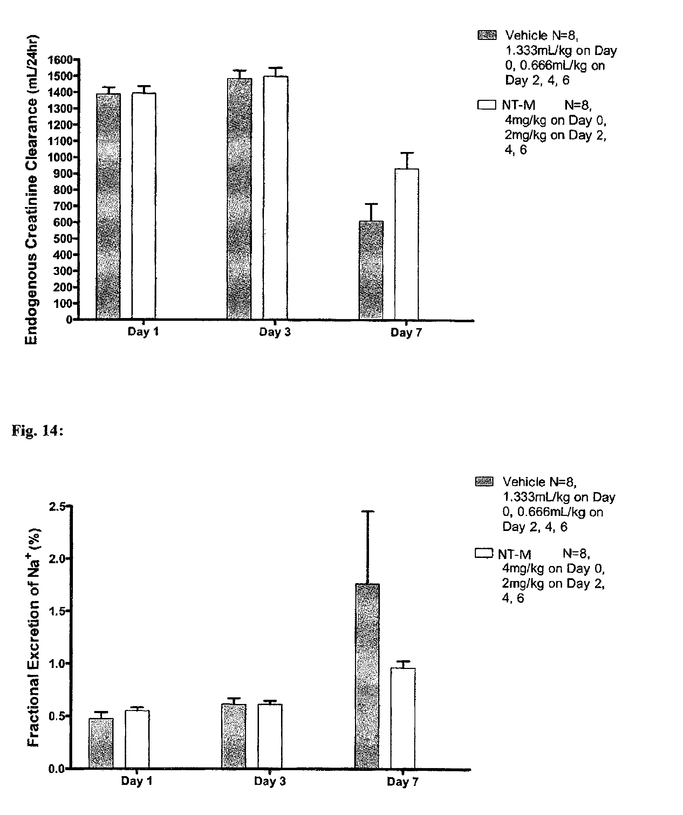

Treatment with anti-Adrenomedullin antibody improved several measures of kidney function on day 7 as compared to vehicle: serum creatinine 1.01 mg/dL (NT-M) vs 1.55 mg/dL (vehicle) (Fig. 11), BUN 32.08 mg/dL(NT-M) vs. 52.41 mg/dL (vehicle) (Fig. 12), endogenous creatinine clearance 934.43 mL/24 h (NT-M) vs. 613.34 mL/24 h (vehicle) (Fig. 13), fractional secretion of Na$^+$ 0.98 % (NT-M) vs. 1.75 % (vehicle) (Fig. 14).

## Example 8

In the mice CLP model described above, the effect of treatment with anti-adrenomedullin antibody NT-M on several parameters of kidney function was investigated.

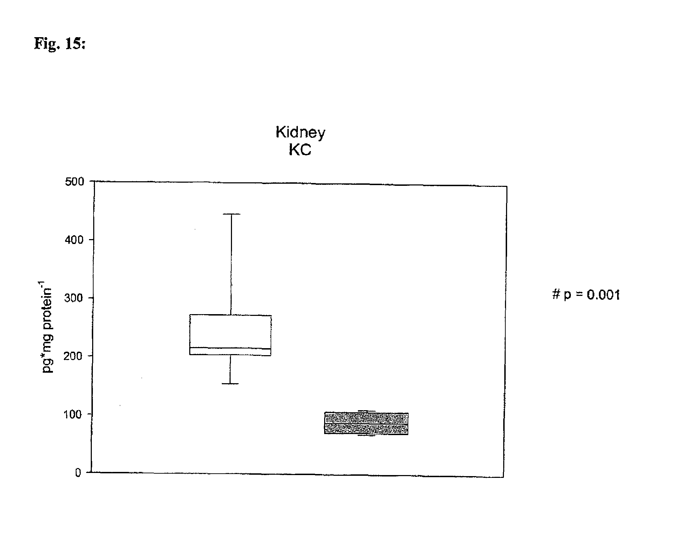

NT-M caused a three- and two-fold higher diuresis and creatinine clearance, respectively, ultimately resulting in lower creatinine, urea, and NGAL blood concentrations at the end of the experiment (see Table 10). Moreover, keratinocyte-derived chemokine (KC) concentrations in the kidney were significantly lowered by treatment with NT-M (Fig. 15).

**Table 10:** Parameters of kidney function in the vehicle- (n=11) and NT-M-treated (n=9) animals. Blood concentrations were measured in samples taken at the end of the experiment. NGAL = neutrophil gelatinase-associated lipocalin. All data are median (quartiles).

| | Vehicle | NT-M | p-Value |
|---|---|---|---|
| Urine output [$\mu$L·g$^{-1}$·h$^{-1}$] | 4.4 (3.5;16.5) | 15.2 (13.9;22.5) | 0.033 |
| Creatinine clearance [$\mu$L·min$^{-1}$] | 197 (110;301) | 400 (316;509) | 0.006 |
| Creatinine [$\mu$g·mL$^{-1}$] | 1.83 (1.52;3.04) | 1.28 (1.20;1.52) | 0.010 |
| Urea [$\mu$g·mL$^{-1}$] | 378 (268;513) | 175 (101;184) | 0.004 |
| NGAL [$\mu$g·mL$^{-1}$] | 16 (15;20) | 11 (10;13) | 0.008 |

The experiments were performed as follows:

*Creatinine, urea, and neutrophil gelatinase-associated lipocalin (NGAL)*

Blood NGAL concentrations were measured using a commercial ELISA (mouse NGAL, RUO 042, BioPorto Diagnostics A/S, Denmark, Gentofte). Urea and creatinine concentrations were measured with a capillary column (Optima-5MS, Macherey-Nagel, Düren, Germany) gas chromatography/mass spectrometry system (Agilent 5890/5970, Böblingen, Germany) using $^2$H$_3$-creatinine (CDN isotopes, Pointe-Claire, QU, Canada) and methyl-urea (FlukaChemikalien, Buchs, Switzerland) as internal standards. After deproteinization with acetonitrile, centrifugation and evaporation to dryness, the supernatant was reconstituted in formic acid, and extracted over a weak anion exchange column (WCX, Phenomenex, Aschaffenburg, Germany). Acetonitrile plus N,O-Bis(trimethylsilyl)trifluoroacetamide and N-(tertbutyldimethylsilyl)-N-methyltrifluoroacetamide allowed formation of the urea tert-butyldimethylsilyl- and the creatininetrimethylsilyl-derivatives, respectively. Ions m/z 231 and 245, and m/z 329 and 332 were monitored for urea and creatinine analytes and internal standards, respectively. From the urine output and the plasma and urine creatinine concentrations creatinine clearance was calculated using the standard formula.

**Sample preparation**

The kidney which was stored at -80°C was disrupted with a homogenizer in PBS and lysed with a 2-fold concentrated buffer for a whole cell lysate (100 mM Tris pH 7,6; 500 mM NaCl; 6 mM EDTA; 6 mM EGTA; 1 % Triton-X-100; 0,5 % NP 40; 10 % Glycerol; Protease-Inhibitors ($\beta$-Glycerolphosphate 2 mM; DTT 4 mM; Leupeptine 20 $\mu$M; Natriumorthovanadate 0,2 mM)) and subsequently centrifuged. The whole cell lysate was obtained out of the supernatant; the pellet consisting of cell remnants was discarded. The amount of protein was determined photometrically with a commercially available protein assay (Bio-Rad, Hercules, CA) and the specimens were adjusted in the way that the final protein concentration was 4 $\mu$g/$\mu$l. The samples for the Multiplex- and EMSA analysis were diluted 1:1 with EMSA buffer (10 mM Hepes; 50 mM KCl; 10 % Glycerol; 0,1 mM EDTA; 1 mM DTT), the samples for the immuno blots 1:1 with 2-fold Sample Buffer (2 % SDS; 125 mM Tris-HCL (pH 6,8 at 25°C); 10 % Glycerol; 50 mM DTT; 0,01 % Bromophenol blue).

Levels of keratinocyte-derived chemokine (KC) concentrations were determined using a mouse multiplex cytokine kit (Bio-Plex Pro Cytokine Assay, Bio-Rad, Hercules, CA), the assay was performed by using the Bio-plex suspension array system with the manufacturer's instructions (see also Wagner F, Wagner K, Weber S, Stahl B, Knöferl MW, Huber-Lang M, Seitz DH, Asfar P, Calzia E, Senftleben U, Gebhard F, Georgieff M, Radermacher P, Hysa V. Inflammatory effects of hypothermia and inhaled H2S during resuscitated, hyperdynamic murine septic shock. Shock 2011;35:396-402; and Wagner F, Scheuerle A, Weber S, Stahl B, McCook O, Knöferl MW, Huber-Lang M, Seitz DH, Thomas J, Asfar P, Szabó C, Möller P, Gebhard F, Georgieff M, Calzia E, Radermacher P, Wagner K. Cardiopulmonary, histologic, and inflammatory effects of intravenous Na2S after blunt chest trauma-induced lung contusion in mice. J Trauma 2011;71:1659-1667). In brief, the appropriate cytokine standards and samples were added to a filter plate. The samples were incubated with antibodies chemically attached to fluorescent-labeled micro beads. Thereafter, premixed detection antibodies were added to each well, and subsequently, streptavidin-phycoerythrin was added. Beads were then re-suspended, and the cytokines reaction mixture was quantified using the Bio-Plex protein array reader. Data were automatically processed and analyzed by Bio-Plex Manager Software 4.1 using the standard curve produced from recombinant cytokine standards. Levels below the detection limit of the assays were set to zero for statistical purposes.

**Example 9**

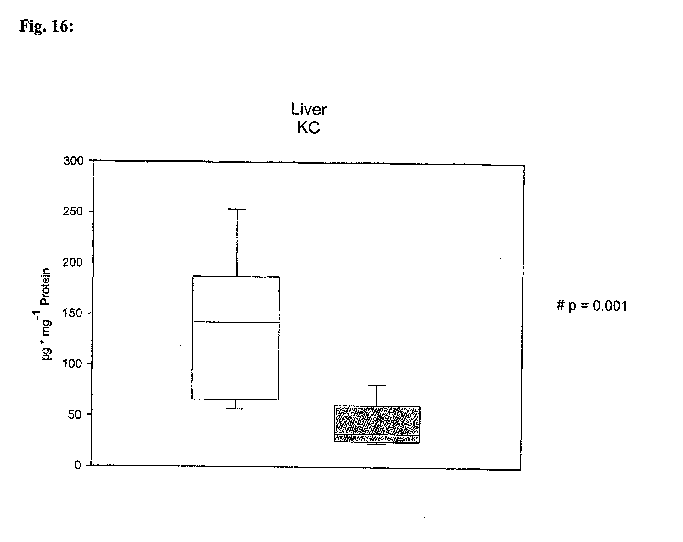

In the mice CLP model described above, the effect of treatment with anti-adrenomedullin antibody NT-M on the liver was investigated.
NT-M caused a significant lowering of keratinocyte-derived chemokine (KC) concentrations in the liver (Fig. 16).
Measurement of keratinocyte-derived chemokine (KC) was done analogous to example 8 (kidney).

**Example 10**

In the mice CLP model described above, the effect of treatment with anti-adrenomedullin antibody NT-M on several cytokines and chemokinesin the blood circulation (plasma) was investigated.

***Cytokine and chemokine concentrations***

Plasma levels of tumor necrosis factor (TNF)-$\alpha$, interleukin (IL)-6, monocyte chemoattractant protein (MCP)-1, and keratinocyte-derived chemokine (KC) concentrations were determined using a mouse multiplex cytokine kit (Bio-Plex Pro Cytokine Assay, Bio-Rad, Hercules, CA), the assay was performed by using the Bio-plex suspension array system with the manufacturer's instructions (see also Wagner F, Wagner K, Weber S, Stahl B, Knöferl MW, Huber-Lang M, Seitz DH, Asfar P, Calzia E, Senftleben U, Gebhard F, Georgieff M, Radermacher P, Hysa V. Inflammatory effects of hypothermia and inhaled H2S during resuscitated, hyperdynamic murine septic shock. Shock 2011;35:396-402; and Wagner F, Scheuerle A, Weber S, Stahl B, McCook O, Knöferl MW, Huber-Lang M, Seitz DH, Thomas J, Asfar P, Szabó C, Möller P, Gebhard F, Georgieff M, Calzia E, Radermacher P, Wagner K. Cardiopulmonary, histologic, and inflammatory effects of intravenous Na2S after blunt chest trauma-induced lung contusion in mice. J Trauma 2011;71:1659-1667). In brief, the appropriate cytokine standards and samples were added to a filter plate. The samples were incubated with antibodies chemically attached to fluorescent-labeled micro beads. Thereafter, premixed detection antibodies were added to each well, and subsequently, streptavidin-phycoerythrin was added. Beads were then re-suspended, and the cytokines reaction mixture was quantified using the Bio-Plex protein array reader. Data were automatically processed and analyzed by Bio-Plex Manager Software 4.1 using the standard curve produced from recombinant cytokine standards. Levels below the detection limit of the assays were set to zero for statistical purposes.

Plasma levels and kidney tissue concentrations of tumor necrosis factor (TNF)-$\alpha$, interleukin (IL)-6 and IL-10, monocyte chemoattractant protein (MCP)-1, and keratinocyte-dervived chemokine (KC) were determined using a commercially available "Multiplex Cytokine Kit" (Bio-Plex Pro Precision Pro Cytokine Assay, Bio-Rad, Hercules, CA), which allows to collect several parameters out of one single sample. The individual work steps of the assay were performed according to the manufacturer's instructions (see also Wagner F, Wagner K, Weber S, Stahl B, Knöferl MW, Huber-Lang M, Seitz DH, Asfar P, Calzia E, Senftleben U, Gebhard F, Georgieff M, Radermacher P, Hysa V. Inflammatory effects of hypothermia and inhaled H2S during resuscitated, hyperdynamic murine septic shock. Shock 2011;35:396-402; and Wagner F, Scheuerle A, Weber S, Stahl B, McCook O, Knöferl MW, Huber-Lang M, Seitz DH, Thomas J, Asfar P, Szabó C, Möller P, Gebhard F, Georgieff M, Calzia E, Radermacher P, Wagner K. Cardiopulmonary, histologic, and inflammatory effects of intravenous Na2S after blunt chest trauma-induced lung contusion in mice. J Trauma 2011;71:1659-1667).

In brief, the fluorescence-labed microspheres ("beads") were added to a 96-well plate, followed by two washing steps, the addition of internal standards and the addition of plasma- and kidney homogenate samples. During the subsequent incubation the single cytokines bind to the antibodies attached to polystyrene-beads. After the addition of the cytokine-specific biotin-labeled antibodies, which are for the detection of the single cytokines, and an additional incubation time, subsequently phycoerythrin-labeled streptavidine was added. After an additional incubation time, beads were then resuspended, and the plates could be measured with a specific flow cytometer (Bio-Plex suspension array system, Bio-Rad, Hercules, CA). Data were automatically processed and analyzed by Bio-Plex Manager Software 4.1 using the standard curve produced from recombinant cytokine standards. For the plasma levels the concentration was provided in pg * mL$^{-1}$, the concentration of the kidney homogenates were converted to the appropriate protein concentration and provided in pg * mg$^{-1}$ protein.

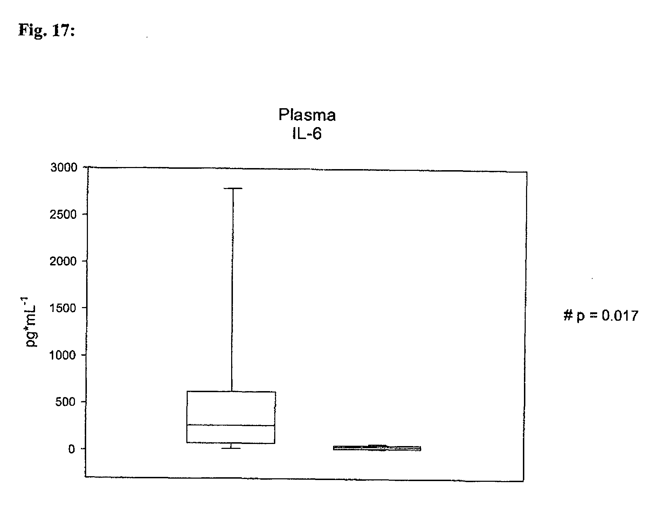

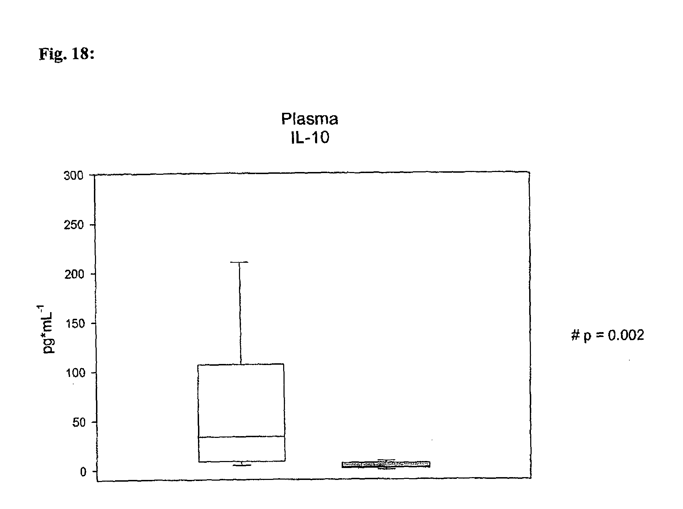

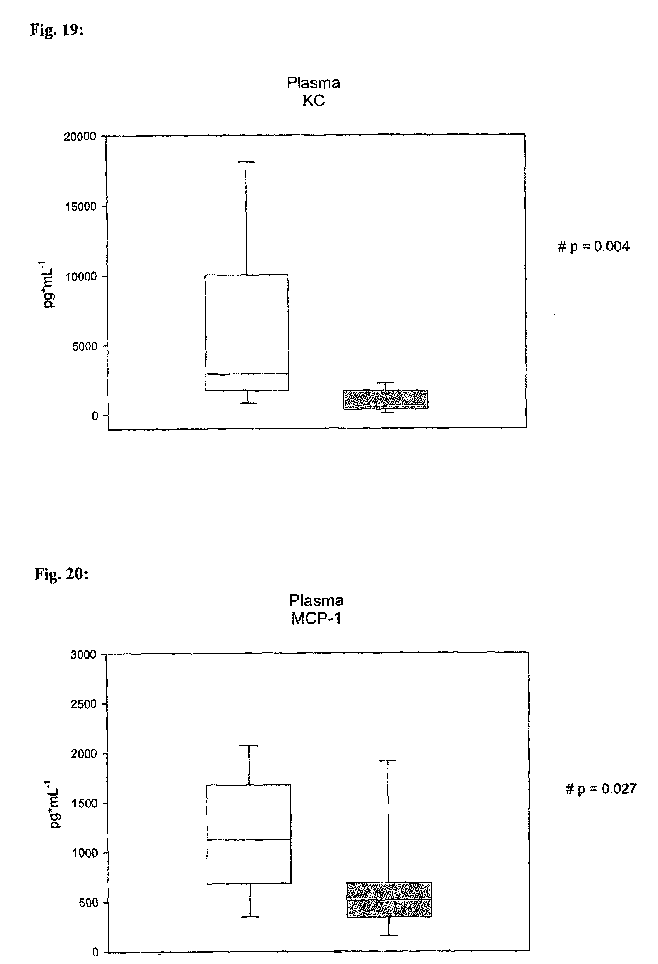

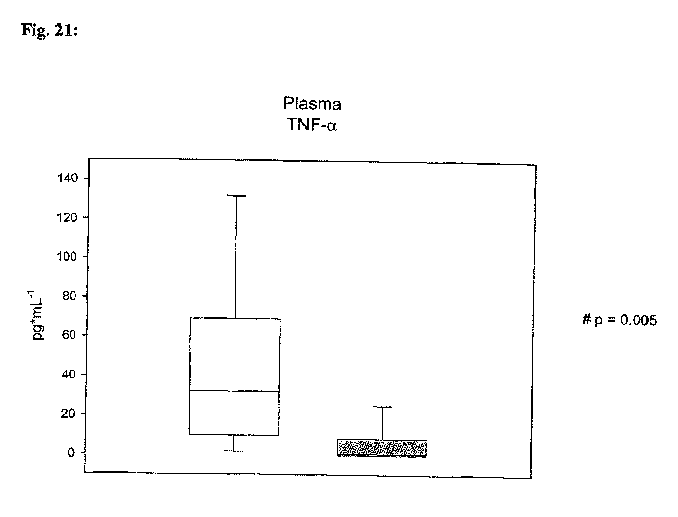

NT-M caused a significant lowering of plasma concentrations of IL-6 (Fig. 17), IL-10 (Fig. 18), keratinocyte-derived chemokine (KC) (Fig. 19), monocyte chemoattractant protein-1 (MCP-1) (Fig. 20), TNF-alpha (Fig. 21).

## Example 11

### Ischemia/Reperfusion-Induced Acute Kidney Injury

Another non-septic acute kidney injury model has been established, where acute kidney injury is induced by ischemia/reperfusion (Nakamoto M, Shapiro JI, Shanley PF, Chan L, and Schrier RW. In vitro and in vivo protective effect of atriopeptin III on ischemic acute renal failure. J ClinInvest 80:698-705, 1987., Chintala MS, Bernardino V, and Chiu PJS. Cyclic GMP but not cyclic AMP prevents renal platelet accumulation following ischemiareperfusion in anesthetized rats. J PharmacolExpTher 271:1203-1208, 1994). This model was used to assess whether treatment with anti-adrenomedullin antibody can improve kidney function.

The experiment was performed as follows:

### Effect of a NT-M on Acute Kidney Injury Induced by Ischemia/Reperfusion in Rats

**Study Design:**

| Group | Test Article | Route | Conc mg/ml | Dosage ml/kg | mg/kg | Rats (Male) |
|---|---|---|---|---|---|---|
| 1 | I-R + vehicle[a] | IV | | 5 | NA x 3 | 8 |
| 2 | I-R + NT-M | IV | | 5 | x 3[b] | 8 |

[a] vehicle; injected intravenously (i.v.) 5 min before reperfusion on day 0, followed by injections on days 1 and 2.

[b]NT-M at 4 mg/kg was injected intravenously (i.v.) 5 min before reperfusion on day 0, followed by 2 mg/kg i.v. each on days 1 and 2.

[c]Urine collection on days -1, 0, 1 and 2, with blood chemistry and urine analysis on days 0, 1, 2 and 3, respectively. Plasma samples were collected in EDTA tubes (Days 0 (immediate before surgery), 1, 2: 100 $\mu$l, before vehicle or TA; Day 3:120 $\mu$l.

Clinical observations: daily before surgery, following surgery and throughout treatment.

Groups of 8 male Sprague-Dawley rats weighing 250 to 280 g were used. The animals were kept on a 12-hr light/dark cycle and receive a standard diet with distilled water ad libitum. The animals receive fluid supplements (0.9% NaCl and 5% dextrose/1:1, 10 ml/kg p.o.) 30 min prior to surgery (day 0). The rats were anaesthetized with pentobarbital (50 mg/kg, i.p.). The abdominal cavity was exposed via a midline incision, followed by intravenous administration of heparin (100 U/kg, i.v.) and both renal arteries were occluded for 45 min by using vascular clamps. Immediately after removal of the renal clips, the kidneys were observed for additional 1 min to ensure color change indicating blood reperfusion. The test compound (NT-M) and vehicle (phosphate buffered saline) were injected intravenously 5 min before reperfusion, followed by daily injection on days 1 and 2.

Urine collection. The 24-h urine collection on ice was initiated at 24h before ischemia/reperfusion on day -1 (-24h to 0h), and day 0 (0-24h), day 1 (24-48h) and day 2 (48-72h) after reperfusion,

Blood collection: 0.4 ml blood was collected through the tail vein into EDTA tubes at 0h (before I RI surgery), 24h (before vehicle or TA), 48h (before vehicle or TA) and 72h for determination of plasma creatinine/Na+/K+, and BUN; 2 ml blood was collected through venal cava terminally.

The animals were placed in individual cages where urine was collected for 24 h day -1 (-24h-0h), day 0 (0-24h), day 1 (24-48h) and day 2 (48-72h) after reperfusion on day 0. Urine volume, urinary Na+, K+, and creatinine were measured.

The creatinine clearance (CCr) was calculated as follows:

$$CCr\ (ml/24\ h) = [UCr\ (mg/ml) \times V\ (ml/24\ h)] / PCr\ (mg/ml)$$

The 24-hr urinary excretion of sodium (Na+) was calculated as follows:

$$UNaV\ (\mu Eq/24\ h) = UNa\ (\mu Eq/ml) \times V\ (ml/24\ h)$$

The fractional excretion of Na+ (FENa), or percentage of the filtered sodium that is excreted into the final urine, is a measure of tubular Na+ reabsorptive function. It was computed as follows:

$$FENa\ (\%) = 100 \times [UNa\ (\mu Eq/ml) \times V\ (ml/24\ h)] / PNa\ (\mu Eq/ml) \times CCr\ (ml/24\ h)$$

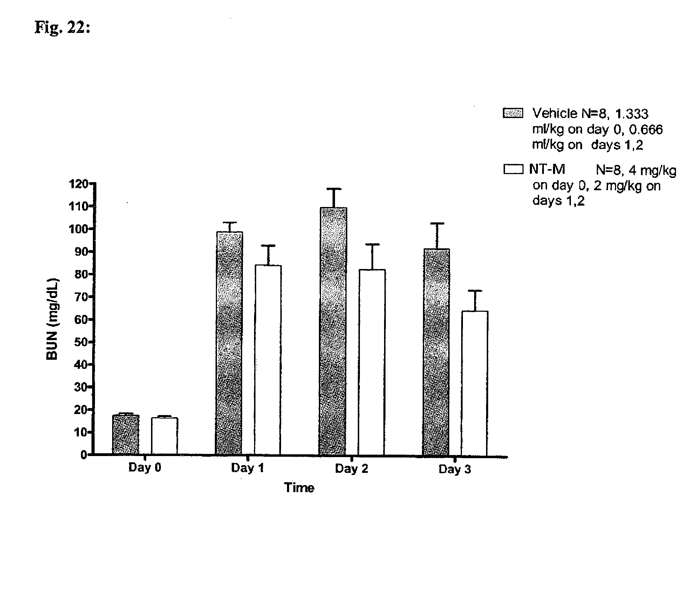

Treatment with anti-Adrenomedullin antibody improved several measures of kidney function: Blood urea nitrogen (BUN) showed a strong increase in the vehicle group (0 h: 17.49 mg/dL, 24 h: 98.85 mg/dL, 48 h: 109.84 mg/dL, 72 h: 91.88 mg/dL), which was less pronounced with NT-M treatment (0 h: 16.33 mg/dL, 24 h: 84.2 mg/dL, 48 h: 82.61 mg/dL, 72 h: 64.54 mg/dL) (Fig. 22).

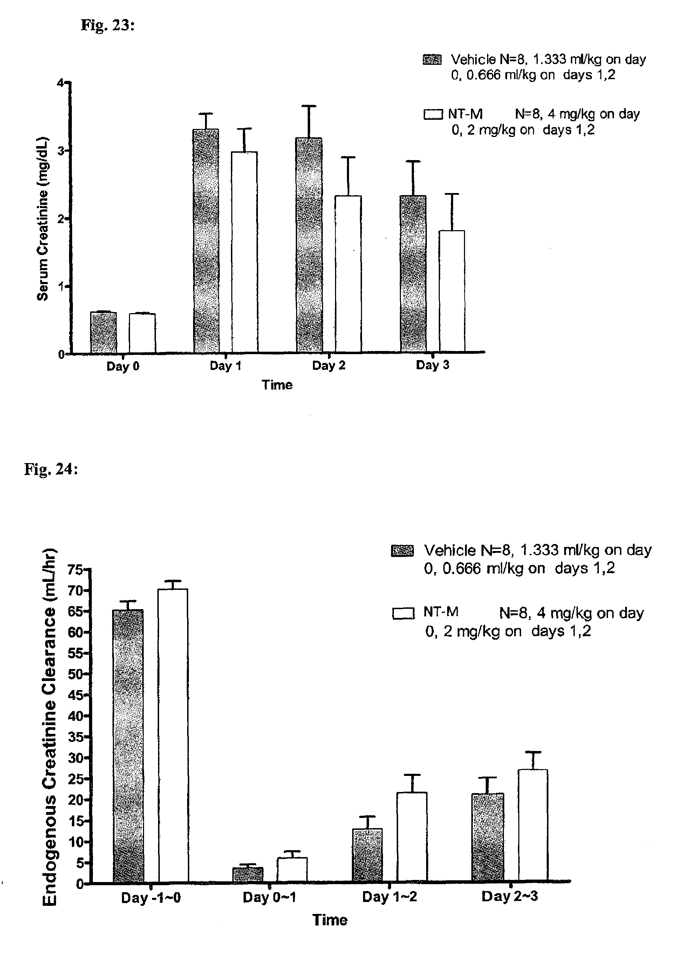

Serum creatinine developed similarily: Vehicle group (0 h: 0.61 mg/dL, 24 h: 3.3 mg/dL, 48 h: 3.16 mg/dL, 72 h: 2.31 mg/dL), NT-M group: (0 h: 0.59 mg/dL, 24 h: 2.96 mg/dL, 48 h: 2.31 mg/dL, 72 h: 1.8 mg/dL) (Fig. 23).

The endogenous creatinine clearance dropped massively on day one and thereafter improved better in the NT-M group than in the vehicle group. Vehicle group: (0 h: 65.17mL/h, 24 h: 3.5mL/h, 48 h: 12.61mL/h, 72 h: 20.88mL/h), NT-M group: (0 h: 70.11mL/h, 24 h: 5.84mL/h, 48 h: 21.23mL/h, 72 h: 26.61mL/h) (Fig. 24).

**FIGURE DESCRIPTION**

**Fig. 1a:**
Illustration of antibody formats - Fv and scFv-Variants

**Fig 1b:**
Illustration of antibody formats - heterologous fusions and bifunctional antibodies

**Fig 1c:**
Illustration of antibody formats - bivalental antibodies and bispecific antibodies

**Fig. 2:**

hADM 1-52 (SEQ ID No. 21)
mADM 1-50 (SEQ ID No. 22)

aa 1-21 of human ADM (SEQ ID No. 23)
aa 1-42 of human ADM (SEQ ID No. 24)
aa 43-52 of human ADM (SEQ ID No. 25)
aa 1-14 of human ADM (SEQ ID NO: 26)
aa 1-10 of human ADM (SEQ ID NO: 27)
aa 1-6 of human ADM (SEQ ID NO: 28)
aa 1-32 of human mature human ADM (SEQ ID NO: 29)
aa 1-40 of mature murine ADM (SEQ ID NO: 30)
aa 1-31 of mature murine ADM (SEQ ID NO: 31)

**Fig. 3:**

a: Dose response curve of human ADM. Maximal cAMP stimulation was adjusted to 140% activation
b: Dose/ inhibition curve of human ADM 22-52 (ADM-receptor antagonist) in the presence of 5.63nM hADM.
c: Dose/ inhibition curve of CT-H in the presence of 5.63 nM hADM.
d: Dose/ inhibition curve of MR-H in the presence of 5.63 nM hADM.
e: Dose/ inhibition curve of NT-H in the presence of 5.63 nM hADM.
f: Dose response curve of mouse ADM. Maximal cAMP stimulation was adjusted to 100% activation
g: Dose/ inhibition curve of human ADM 22-52 (ADM-receptor antagonist) in the presence of 0,67 nM mADM.
h: Dose/ inhibition curve of CT-M in the presence of 0,67 nM mADM.
i: Dose/ inhibition curve of MR-M in the presence of 0,67 nM mADM.
j: Dose/ inhibition curve of NT-M in the presence of 0,67 nM mADM.
k: shows the inhibition of ADM by F(ab)2 NT-M and by Fab NT-M
1: shows the inhibition of ADM by F(ab)2 NT-M and by Fab NT-M

**Fig. 4:**
This figure shows a typical hADM dose/ signal curve. And an hADM dose signal curve in the presence of 100 $\mu$g/mL antibody NT-H.

**Fig. 5:**
This figure shows the stability of hADM in human plasma (citrate) in absence and in the presence of NT-H antibody.

**Fig. 6:**
Alignment of the Fab with homologous human framework sequences

**Fig. 7:**
This figure shows the Noradrenalin requirements for early and late treatment with NT-M

**Fig. 8:**
This figure shows urine production after early and late treatment with NT-M

**Fig. 9:**
This figure shows the fluid balance after early and late treatment with NT-M

**Fig. 10:**
Liver tissue activation of nuclear factor kappa-light-chain gene enhancer in B cells (NF-$\kappa$B) analyzed by electophoretic mobility shift assay (EMSA). # depicts p<0.001 vs. vehicle.

**Fig. 11:**
Development of serum creatinine over time. Mean +/- SEM are shown.

**Fig. 12 :**
Development of blood urea nitrogen (BUN) over time. Mean +/- SEM are shown.

**Fig. 13:**
Development of endogenous creatinine clearance over time. Mean +/- SEM are shown.

**Fig.14:**

Development of fractional secretion of Na$^+$ over time. Mean +/- SEM are shown.

**Fig. 15:**
Keratinocyte-derived chemokine (KC) levels determined in relation to the total kidney protein extracted. The white box-plot shows results obtained with vehicle, the grey box-plot shows results obtained after treatment with NT-M.

**Fig. 16:**
Keratinocyte-derived chemokine (KC) levels determined in relation to the total liver protein extracted. The white box-plot shows results obtained with vehicle, the grey box-plot shows results obtained after treatment with NT-M.

**Fig. 17:**
Plasma IL-6 levels. The white box-plot shows results obtained with vehicle, the grey box-plot shows results obtained after treatment with NT-M.

**Fig.18:**
Plasma IL-10 levels. The white box-plot shows results obtained with vehicle, the grey box-plot shows results obtained after treatment with NT-M.

**Fig.19:**
Plasma keratinocyte-derived chemokine (KC) levels. The white box-plot shows results obtained with vehicle, the grey box-plot shows results obtained after treatment with NT-M.

**Fig. 20:**
Plasma monocyte chemoattractant protein-1 (MCP-1) levels. The white box-plot shows results obtained with vehicle, the grey box-plot shows results obtained after treatment with NT-M.

**Fig. 21:**
Plasma TNF-alpha levels. The white box-plot shows results obtained with vehicle, the grey box-plot shows results obtained after treatment with NT-M.

**Fig. 22:**
Development of blood urea nitrogen (BUN) over time. Mean +/- SEM are shown.

**Fig. 23:**
Development of serum creatinine over time. Mean +/- SEM are shown.

**Fig. 24:**
Development of endogenous creatinine clearance over time. Mean +/- SEM are shown.

**Claims**

1. Anti-adrenomedullin antibody or an anti-ADM antibody fragment binding to adrenomedullin or an anti-ADM non-Ig-protein scaffold binding to adrenomedullin for use as a medicament, wherein said antibody or fragment or scaffold binds to the N-terminal part, amino acids 1 to 21, of adrenomedullin:

   YRQSMNNFQGLRSFGCRFGTC; SEQ ID No. 23.

2. Anti-adrenomedullin antibody or an anti-ADM antibody fragment binding to adrenomedullin or an anti-ADM non-Ig-protein scaffold binding to adrenomedullin for use as a medicament according to claim 1, wherein said antibody or fragment or scaffold is monospecific.

3. Anti-adrenomedullin antibody or an anti-ADM antibody fragment binding to adrenomedullin or an anti-ADM non-Ig-protein scaffold binding to adrenomedullin for use as a medicament according to claim 1 or 2, wherein said antibody or fragment or scaffold exhibits a binding affinity to ADM of at least $10^{-7}$ M.

4. Anti-adrenomedullin antibody or an anti-ADM antibody fragment binding to adrenomedullin or an anti-ADM non-Ig-protein scaffold binding to adrenomedullin for use as a medicament according to any of claim 1 to 3, wherein said antibody or fragment or scaffold is not ADM-binding-Protein-1, complement factor H.

5. Anti-adrenomedullin antibody or an anti-ADM antibody fragment binding to adrenomedullin or an anti-ADM non-Ig-protein scaffold binding to adrenomedullin for use as a medicament, wherein said antibody or said fragment or said scaffold according to any of claims 1 to 4 binds to an epitope containing the N-terminal end ; amino acid 1; of adrenomedullin.

6. Anti-Adrenomedullin (ADM) antibody or an anti-ADM antibody fragment binding to adrenomedullin or anti-ADM non-Ig-protein scaffold binding to adrenomedullin for use as a medicament according to any of the claims 1 to 5, **characterized in that** said antibody, antibody fragment or non-Ig scaffold does not bind to the C-terminal portion of ADM, being it amino acids 43-52 of ADM
PRSKISPQGY-NH2 ; SEQ ID NO: 25.

7. Anti-adrenomedullin antibody or an anti-ADM antibody fragment binding to adrenomedullin or an anti-ADM non-Ig-protein scaffold binding to adrenomedullin for use as a medicament according to any of claims 1 to 6, wherein said antibody or said fragment or said scaffold is an ADM stabilizing antibody or fragment or scaffold that enhances the half life,$t_{1/2}$ half retention time, of adrenomedullin in serum, blood, plasma at least 10 %, or at least 50 %, or >50 %, or 100 % and/or wherein said antibody or fragment or scaffold blocks the circulating ADM bioactivity not more than 50 %, as determined in a human recombinant Adrenomedullin receptor cAMP functional assay.

8. Anti-adrenomedullin antibody or an anti-ADM antibody fragment binding to adrenomedullin or an anti-ADM non-Ig-protein scaffold binding to adrenomedullin for use as a medicament according to any of claims 1 to 7, wherein said antibody or said fragment or said scaffold is a modulating anti-ADM antibody or fragment or scaffold that enhances the half life, $t_{1/2}$ half retention time, of adrenomedullin in serum, blood, plasma at least 10 %, or at least, 50 %, or >50 %, or 100 % and that blocks the bioactivity of ADM to not more than 50 %, as determined in a human recombinant Adrenomedullin receptor cAMP functional assay.

9. Anti-adrenomedullin antibody or an anti-ADM antibody fragment binding to adrenomedullin for use as a medicament according to any of claims 1 to 8, wherein said antibody or fragment is a monospecific antibody or monospecific antibody fragment that binds to ADM or an antibody fragment thereof, binding to ADM wherein the heavy chain comprises the sequences:

SEQ ID NO: 1
GYTFSRYW
SEQ ID NO: 2
ILPGSGST
SEQ ID NO: 3
TEGYEYDGFDY
and wherein the light chain comprises the sequences
SEQ ID NO:4
QSIVYSNGNTY
SEQ ID NO: 5
RVS
SEQ ID NO: 6
FQGSHIPYT.

10. Anti-adrenomedullin antibody or an anti-ADM antibody fragment binding to adrenomedullin for use as a medicament according to claim 9, wherein said antibody or fragment is a monospecific antibody or fragment that binds to ADM or an antibody fragment thereof, wherein said antibody or fragment comprises the sequences:

SEQ ID NO: 7; AM-VH-C

QVQLQQSGAELMKPGASVKISCKATGYTFSRYWIEWVKQRPGHGLEWIGEILP
GSGSTNYNEKFKGKATITADTSSNTAYMQLSSLTSEDSAVYYCTEGYEYDGFD
YWGQGTTLTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID NO: 8; AM-VH1

QVQLVQSGAEVKKPGSSVKVSCKASGYTFSRYWISWVRQAPGQGLEWMGRIL
PGSGSTNYAQKFQGRVTITADESTSTAYMELSSLRSEDTAVYYCTEGYEYDGFD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID NO: 9; AM-VH2-E40

QVQLVQSGAEVKKPGSSVKVSCKASGYTFSRYWIEWVRQAPGQGLEWMGRIL
PGSGSTNYAQKFQGRVTITADESTSTAYMELSSLRSEDTAVYYCTEGYEYDGFD
YWGQGTTVTVSSASTKGPSVFPLAPSSKSTSGGTAALGCLVKDYFPEPVTVSWN

SGALTSGVHTFPAVLQSSGLYSLSSVVTVPSSSLGTQTYICNVNHKPSNTKVDKR
VEPKHHHHHH

SEQ ID NO: 10 ; AM-VH3-T26-E55

QVQLVQSGAEVKKPGSSVKVSCKATGYTFSRYWISWVRQAPGQGLEWMGEIL
PGSGSTNYAQKFQGRVTITADESTSTAYMELSSLRSEDTAVYYCTEGYEYDGFD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID NO: 11 ; AM- VH4- T26-E40-E55

QVQLVQSGAEVKKPGSSVKVSCKATGYTFSRYWIEWVRQAPGQGLEWMGEIL
PGSGSTNYAQKFQGRVTITADESTSTAYMELSSLRSEDTAVYYCTEGYEYDGFD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID NO: 12; AM-VL-C

DVLLSQTPLSLPVSLGDQATISCRSSQSIVYSNGNTYLEWYLQKPGQSPKLLIYR
VSNRFSGVPDRFSGSGSGTDFTLKISRVEAEDLGVYYCFQGSHIPYTFGGGTKLE
IKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGE
C

SEQ ID NO: 13; AM-VL1

DVVMTQSPLSLPVTLGQPASISCRSSQSIVYSNGNTYLNWFQQRPGQSPRRLIYR
VSNRDSGVPDRFSGSGSGTDFTLKISRVEAEDVGVYYCFQGSHIPYTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO: 14 ; AM-VL2-E40

DVVMTQSPLSLPVTLGQPASISCRSSQSIVYSNGNTYLEWFQQRPGQSPRRLIYRVSNRD
SGVPDRFSGSGSGTDFTLKISRVEAEDVGVYYCFQGSHIPYTFGQGTKLEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC

11. Anti-adrenomedullin antibody or an anti-ADM antibody fragment binding to adrenomedullin or an anti-ADM non-Ig-protein scaffold binding to adrenomedullin according to any of the claims 1 to 10 and a vasopressor e.g. catecholamine for use as a medicament.

12. Anti-adrenomedullin antibody or an anti-ADM antibody fragment binding to adrenomedullin or an anti-ADM non-Ig-protein scaffold binding to adrenomedullin according to any of the claims 1 to 10 and an intravenous fluid administration for use as a medicament.

13. Anti-adrenomedullin antibody or an anti-ADM antibody fragment binding to adrenomedullin or an anti-ADM non-Ig-protein scaffold binding to adrenomedullin according to any of the claims 1 to 10 and a TNF-alpha-antibody for use as a medicament.

14. Anti-adrenomedullin antibody or an anti-ADM antibody fragment binding to adrenomedullin or an anti-ADM non-Ig-protein scaffold binding to adrenomedullin according to any of the claims I to 11 and antibiotics for use as a medicament.

15. Pharmaceutical composition comprising an anti-adrenomedullin antibody or an anti-ADM antibody fragment binding to adrenomedullin or an anti-ADM non-Ig-protein scaffold binding to adrenomedullin for use as a medicament according to any of claims 1 to 14.

**Patentansprüche**

1. Anti-Adrenomedullin-Antikörper oder ein anti-ADM-Antikörperfragment bindend an Adrenomedullin oder ein anti-ADM nicht-Ig-Proteingerüst bindend an Adrenomedullin, zur Verwendung als Medikament, wobei der Antikörper oder das Fragment oder das Gerüst an den N-terminalen Teil, Aminosäuren 1-21, von Adrenomedullin bindet:

YRQSMNNFQGLRSFGCRFGTC; SEQ ID Nr. 23.

2. Anti-Adrenomedullin-Antikörper oder ein anti-ADM-Antikörperfragment bindend an Adrenomedullin oder ein Anti-ADM nicht-Ig-Proteingerüst bindend an Adrenomedullin, zur Verwendung als Medikament gemäß Anspruch 1, wobei der Antikörper oder das Fragment oder das Gerüst monospezifisch ist.

3. Anti-Adrenomedullin-Antikörper oder ein anti-ADM-Antikörperfragment bindend an Adrenomedullin oder ein Anti-ADM nicht-Ig-Proteingerüst bindend an Adrenomedullin, zur Verwendung als Medikament gemäß einem der Ansprüche 1 bis 2, wobei der Antikörper oder das Fragment oder das Gerüst eine Bindungsaffinität von mindestens $10^{-7}$ M gegenüber ADM aufweist.

4. Anti-Adrenomedullin-Antikörper oder ein anti-ADM-Antikörperfragment bindend an Adrenomedullin oder ein Anti-ADM nicht-Ig-Proteingerüst bindend an Adrenomedullin, zur Verwendung als Medikament gemäß einem der Ansprüche 1 bis 3, wobei der Antikörper oder das Fragment oder das Gerüst nicht das ADM-bindende Protein-1, Komplementfaktor H, ist.

5. Anti-Adrenomedullin-Antikörper oder ein anti-ADM-Antikörperfragment bindend an Adrenomedullin oder ein Anti-ADM nicht-Ig-Proteingerüst bindend an Adrenomedullin zur Verwendung als Medikament, wobei der Antikörper oder das Fragment oder das Gerüst gemäß einem der Ansprüche 1 bis 4 an ein Epitop bindet, welches das N-terminale Ende, Aminosäure 1, von Adrenomedullin aufweist.

6. Anti-Adrenomedullin (ADM)-Antikörper oder anti-ADM-Antikörperfragment bindend an Adrenomedullin oder ein Anti-ADM nicht-Ig-Proteingerüst bindend an Adrenomedullin, zur Verwendung als Medikament gemäß einem der Ansprüche 1 bis 5, **dadurch gekennzeichnet, dass** der besagte Antikörper, das Antikörperfragment oder das nicht-Ig-Gerüst nicht an den C-terminalen Abschnitt von ADM, bestehend aus den Aminosäuren 43-52 von ADM, bindet. PRSKISPQGY-NH2; SEQ ID NR: 25.

7. Anti-Adrenomedullin-Antikörper oder ein anti-ADM-Antikörperfragment bindend an Adrenomedullin oder ein Anti-ADM Nicht-Ig-Proteingerüst bindend an Adrenomedullin zur Verwendung als Medikament gemäß einem der Ansprüche 1 bis 6, wobei der Antikörper oder das Fragment oder das Gerüst ein ADM-stabilisierender Antikörper, Fragment oder Gerüst ist, das die Halbwertzeit, $t_{1/2}$ Verweilhalbwertszeit, von Adrenomedullin in Serum, Blut, Plasma um mindestens 10% oder um mindestens 50% oder um >50% oder um 100% erhöht und/oder wobei der Antikörper oder das Fragment oder das Gerüst die zirkulierende ADM-Bioaktivität um nicht mehr als 50% blockiert gemäß der Bestimmung in einem humanen, rekombinanten Andrenomedullin-Rezeptor cAMP Funktionsassay.

8. Anti-Adrenomedullin-Antikörper oder ein anti-ADM-Antikörperfragment bindend an Adrenomedullin oder ein Anti-ADM nicht-Ig-Proteingerüst bindend an Adrenomedullin, zur Verwendung als Medikament gemäß einem der Ansprüche 1 bis 7, wobei der Antikörper oder das Fragment oder das Gerüst ein modulierender Anti-ADM-Antikörper oder ein Fragment oder ein Gerüst ist, das die Halbwertzeit, $t_{1/2}$ Verweilhalbwertszeit, von Adrenomedullin, in Serum, Blut, Plasma um mindestens 10% oder um mindestens 50% oder um >50% oder um 100% erhöht und das die Bioaktivität von ADM um nicht mehr als 50% blockiert gemäß der Bestimmung in einem humanen, rekombinanten Andrenomedullin-Rezeptor cAMP Funktionsassay.

9. Anti-Adrenomedullin-Antikörper oder ein anti-ADM-Antikörperfragment bindend an Adrenomedullin, zur Verwendung als Medikament gemäß einem der Ansprüche 1 bis 8, wobei der Antikörper oder das Fragment ein monospezifischer Antikörper oder ein monospezifisches Antikörperfragment ist, bindend an ADM oder ein Antikörperfragment davon, welches an ADM bindet, wobei die schwere Kette folgende Sequenzen umfasst:

    SEQ ID NR: 1
    GYTFSRYW
    SEQ ID NR: 2
    ILPGSGST
    SEQ ID NR: 3
    TEGYEYDGFDY
    und wobei die leichte Kette die folgenden Sequenzen umfasst:
    SEQ ID NR: 4
    QSIVYSNGNTY
    SEQ ID NR: 5
    RVS
    SEQ ID NR: 6
    FQGSHIPYT.

**10.** Anti-Adrenomedullin-Antikörper oder ein anti-ADM-Antikörperfragment bindend an Adrenomedullin, zur Verwendung als Medikament gemäß Anspruch 9, wobei der besagte Antikörper oder das Fragment ein monospezifischer Antikörper oder ein Fragment ist, bindend an ADM oder ein Antikörperfragment davon, wobei der Antikörper oder das Fragment die folgenden Sequenzen umfasst:

SEQ ID NR: 7
AM-VH-C

QVQLQQSGAELMKPGASVKISCKATGYTFSRYWIEWVKQRPGHGLEWIGEILP
GSGSTNYNEKFKGKATITADTSSNTAYMQLSSLTSEDSAVYYCTEGYEYDGFD
YWGQGTTLTVSSASTKGPSVFPLAPSSKSTSGGTAALGCLVKDYFPEPVTVSWN

SGALTSGVHTFPAVLQSSGLYSLSSVVTVPSSSLGTQTYICNVNHKPSNTKVDKR
VEPKHHHHHH

SEQ ID NR: 8

AM-VH1

QVQLVQSGAEVKKPGSSVKVSCKASGYTFSRYWISWVRQAPGQGLEWMGRIL
PGSGSTNYAQKFQGRVTITADESTSTAYMELSSLRSEDTAVYYCTEGYEYDGFD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID NR: 9

AM-VH2-E40

QVQLVQSGAEVKKPGSSVKVSCKASGYTFSRYWIEWVRQAPGQGLEWMGRIL
PGSGSTNYAQKFQGRVTITADESTSTAYMELSSLRSEDTAVYYCTEGYEYDGFD
YWGQGTTVTVSSASTKGPSVFPLAPSSKSTSGGTAALGCLVKDYFPEPVTVSWN
SGALTSGVHTFPAVLQSSG LYSLSSVVTVPSSSLGTQTYICNVNHKPSNTKVDKR
VEPKHHHHHH

SEQ ID NR: 10

AM-VH3-T26-E55

QVQLVQSGAEVKKPGSSVKVSCKATGYTFSRYWISWVRQAPGQGLEWMGEIL
PGSGSTNYAQKFQGRVTITADESTSTAYMELSSLRSEDTAVYYCTEGYE YDGFD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID NR: 11

AM-VH4-T26-E40-E55

QVQLVQSGAEVKKPGSSVKVSCKATGYTFSRYWIEWVRQAPGQGLEWMGEIL
PGSGSTNY AQKFQGRVTITADESTSTAYMELSSLRSEDTAVYYCTEGYEYDGFD
YWGQGTTVTVSSASTKGPSVFPLAPSSKSTSGGTAALGCLVKD YFPEPVTVSWN
SGALTSGVHTFPAVLQSSGLYSLSSVVTVPSSSLGTQTYICNVNHKPSNTKVDKR
VEPKHHHHHH

SEQ ID NR: 12

AM-VL-C

DVLLSQTPLSLPVSLGDQATISCRSSQSIVYSNGNTYLEWYLQKPGQSPKLLIYR

VSNRFSGVPDRFSGSGSGTDFTLKISRVEAEDLGWYCFQGSHIPYTFGGGTKLE

IKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGE
C

SEQ ID NR: 13

AM-VL1

DVVMTQSPLSLPVTLGQPASISCRSSQSIVYSNGNTYLNWFQQRPGQSPRRLIYR

VSNRDSGVPDRFSGSGSGTDFTLKISRVEAEDVGVYYCFQGSHIPYTFGQGTKL

EIKRTVAAPSVFIFPPSDEQLKSGTASVVCLLNNFYPREAKVQWKVDNALQSGN

SQESVTEQDSKDSTYSLSSTLTLSKADYEKH VYACEVTHQGLSSPVTKSFNRG

EC

SEQ ID NR: 14

AM-VL2-E40

DVVMTQSPLSLPVTLGQPASISCRSSQSIVYSNGNTYLEWFQQRPGQSPRRLIYR

VSNRDSGVPDRFSGSGSGTDFTLKISRVEAEDVGVYYCFQGSHIPYTFGQGTKL

EIKRTVAAPSV FIFPPSDEQLKSGTASVVCLLNNFYPREAKVQWVDNALQSGN

SQESVTEQDSKDSTYSLSSTLTLSKADYEKHKVYACEVTHQGLSSPVTKSFNRG

EC

11. Anti-Adrenomedullin-Antikörper oder ein anti-ADM-Antikörperfragment bindend an Adrenomedullin oder ein anti-ADM nicht-Ig-Proteingerüst bindend an Adrenomedullin, gemäß einem der Ansprüche 1 bis 10 und ein Vasopressor, z.B. Catecholamin, zur Verwendung als Medikament.

12. Anti-Adrenomedullin-Antikörper oder ein anti-ADM-Antikörperfragment bindend an Adrenomedullin oder ein anti-ADM nicht-Ig-Proteingerüst bindend an Adrenomedullin, gemäß einem der Ansprüche 1 bis 10 und eine intravenöse Flüssigkeitsverabreichung, zur Verwendung als Medikament.

13. Anti-Adrenomedullin-Antikörper oder ein anti-ADM-Antikörperfragment bindend an Adrenomedullin oder ein anti-ADM nicht-Ig-Proteingerüst bindend an Adrenomedullin, gemäß einem der Ansprüche 1 bis 10 und ein TNF-alpha-Antikörper, zur Verwendung als Medikament.

14. Anti-Adrenomedullin-Antikörper oder ein anti-ADM-Antikörperfragment bindend an Adrenomedullin oder ein anti-ADM nicht-Ig-Proteingerüst bindend an Adrenomedullin, gemäß einem der Ansprüche 1 bis 11 und Antibiotika, zur Verwendung als Medikament.

15. Pharmazeutische Zusammensetzung umfassend einen anti-Adrenomedullin-Antikörper oder ein anti-ADM-Antikörperfragment bindend an Adrenomedullin oder ein anti-ADM nicht-Ig-Proteingerüst bindend an Adrenomedullin, gemäß einem der Ansprüche 1 bis 14, zur Verwendung als Medikament..

## Revendications

1. Anticorps anti-adrénomédulline ou fragment d'anticorps anti-ADM se liant à l'adrénomédulline ou échafaudage protéique non-Ig anti-ADM se liant à l'adrénomédulline, pour utilisation en tant que médicament, ledit anticorps ou fragment ou échafaudage se liant à la partie N-terminal, acides aminés 1-21, de l'adrénomédulline :

   YRQSMNNFQGLRSFGCRFGTC ; SEQ ID N° : 23.

2. L'anticorps anti-adrénomédulline ou fragment d'anticorps anti-ADM se liant à l'adrénomédulline ou échafaudage protéique non-Ig anti-ADM se liant à l'adrénomédulline pour utilisation en tant que médicament selon la revendication 1, ledit anticorps ou fragment ou échafaudage étant monospécifique.

3. L'anticorps anti-adrénomédulline ou fragment d'anticorps anti-ADM se liant à l'adrénomédulline ou échafaudage protéique non-Ig anti-ADM se liant à l'adrénomédulline pour utilisation en tant que médicament selon la revendication 1 ou 2, ledit anticorps ou fragment ou échafaudage présentant une affinité de liaison à l'ADM d'au moins $10^{-7}$ M.

4. L'anticorps anti-adrénomédulline ou fragment d'anticorps anti-ADM se liant à l'adrénomédulline ou échafaudage protéique non-Ig anti-ADM se liant à l'adrénomédulline pour utilisation en tant que médicament selon une quelconque des revendications 1 à 3, ledit anticorps ou fragment ou échafaudage n'étant pas la protéine 1 de liaison à l'ADM, complément du facteur H.

5. L'anticorps anti-adrénomédulline ou fragment d'anticorps anti-ADM se liant à l'adrénomédulline ou échafaudage protéique non-Ig anti-ADM se liant à l'adrénomédulline pour utilisation en tant que médicament, ledit anticorps ou fragment ou échaffaudage selon une quelconque des revendications 1 à 4 se liant à un épitope contenant l'extrémité N-terminal, acide aminé 1, de l'adrénomédulline.

6. L'anticorps anti-adrénomédulline (ADM) ou fragment d'anticorps anti-ADM se liant à l'adrénomédulline ou échafaudage protéique non-Ig anti-ADM se liant à l'adrénomédulline pour utilisation en tant que médicament selon une quelconque des revendications 1 à 5, **caractérisé en ce que** ledit anticorps, fragment ou échaffaudage non-Ig ne se lie pas à la partie C-terminal de l'ADM, même s'il s'agit des acides aminés 43-52 de l'ADM.
   PRSKISPQGY-NH2 ; SEQ ID N° : 25.

7. L'anticorps anti-adrénomédulline ou fragment d'anticorps anti-ADM se liant à l'adrénomédulline ou échafaudage protéique non-Ig anti-ADM se liant à l'adrénomédulline pour utilisation en tant que médicament selon une quelconque des revendications 1 à 6, ledit anticorps ou fragment ou échafaudage étant un anticorps ou fragment ou échafaudage de stabilisation d'ADM qui améliore la demi-vie (demi-temps de rétention, $t_{1/2}$) de l'adrénomédulline dans le sérum, le sang, le plasma d'au moins 10 %, d'au moins 50 %, de plus de 50 % ou à 100 % et/ou ledit anticorps ou fragment ou échafaudage bloquant la bioactivité d'ADM de circulation de pas plus de 50 %, tel que déterminé dans un essai fonctionnel cAMP de récepteur d'adrénomédulline humain recombinant.

8. L'anticorps anti-adrénomédulline ou fragment d'anticorps anti-ADM se liant à l'adrénomédulline ou échafaudage protéique non-Ig anti-ADM se liant à l'adrénomédulline pour utilisation en tant que médicament selon une quelconque des revendications 1 à 7, ledit anticorps ou fragment ou échaffaudage étant un anticorps ou fragment ou échafaudage de modulation d'ADM ou un anticorps ou fragment ou échafaudage qui améliore la demi-vie (demi-temps de rétention, $t_{1/2}$) de l'adrénomédulline dans le sérum, le sang, le plasma d'au moins 10 %, d'au moins 50 %, de plus de 50 % ou à 100 % et bloque la bioactivité de l'ADM de pas plus de 50 %, tel que déterminé dans un essai fonctionnel cAMP de récepteur d'adrénomédulline humain recombinant.

9. L'anticorps anti-adrénomédulline ou fragment d'anticorps anti-ADM se liant à l'adrénomédulline pour utilisation en tant que médicament selon une quelconque des revendications 1 à 8, ledit anticorps ou fragment étant un anticorps monospécifique ou fragment de celui-ci se liant à l'ADM ou au fragment d'anticorps de celle-ci, se liant à l'ADM, la chaîne lourde comprenant les séquences :

SEQ ID N° : 1
GYTFSRYW
SEQ ID N° : 2
ILPGSGST
SEQ ID N° : 3
TEGYEYDGFDY
et la chaîne légère comprenant les séquences :
SEQ ID N° : 4
QSIVYSNGNTY
SEQ ID N° : 5
RVS
SEQ ID N° : 6
FQGSHIPYT.

10. L'anticorps anti-adrénomédulline ou fragment d'anticorps anti-ADM se liant à l'adrénomédulline pour utilisation en tant que médicament selon la revendication 9, ledit anticorps ou fragment étant un anticorps monospécifique ou fragment de celui-ci se liant à l'ADM ou au fragment d'anticorps de celle-ci, ledit anticorps ou fragment de celui-ci comprenant les séquences :

SEQ ID N° : 7

AM-VH-C

QVQLQQSGAELMKPGASVKISCKATGYTFSRYWIEWVKQRPGHGLEWIGEILP
GSGSTNYNEKFKGKATITADTSSNTAYMQLSSLTSEDSAVYYCTEGYEYDGFD
YWGQGTTLTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID N° : 8

AM-VH1

QVQLVQSGAEVKKPGSSVKVSCKASGYTFSRYWISWVRQAPGQGLEWMGRIL
PGSGSTNYAQKFQGRVTITADESTSTAYMELSSLRSEDTAVYYCTEGYEYDGFD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID N° : 9

AM-VH2-E40

QVQLVQSGAEVKKPGSSVKVSCKASGYTFSRYWIEWVRQAPGQGLEWMGRIL
PGSGSTNYAQKFQGRVTITADESTSTAYMELSSLRSEDTAVYYCTEGYEYDGFD
YWGQGTTVTVSSASTKGPSVFPLAPSSKSTSGGTAALGCLVKDYFPEPVTVSWN
SGALTSGVHTFPAVLQSSG LYSLSSVVTVPSSSLGTQTYICNVNHKPSNTKVDKR
VEPKHHHHHH

SEQ ID N° : 10

AM-VH3-T26-E55

QVQLVQSGAEVKKPGSSVKVSCKATGYTFSRYWISWVRQAPGQGLEWMGEIL
PGSGSTNYAQKFQGRVTITADESTSTAYMELSSLRSEDTAVYYCTEGYE YDGFD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKHHHHHH

SEQ ID N° : 11

AM-VH4-T26-E40-E55

QVQLVQSGAEVKKPGSSVKVSCKATGYTFSRYWIEWVRQAPGQGLEWMGEIL
PGSGSTNY AQKFQGRVTITADESTSTAYMELSSLRSEDTAVYYCTEGYEYDGFD
YWGQGTTVTVSSASTKGPSVFPLAPSSKSTSGGTAALGCLVKD YFPEPVTVSWN
SGALTSGVHTFPAVLQSSGLYSLSSVVTVPSSSLGTQTYICNVNHKPSNTKVDKR
VEPKHHHHHH

SEQ ID N°: 12

AM-VL-C

DVLLSQTPLSLPVSLGDQATISCRSSQSIVYSNGNTYLEWYLQKPGQSPKLLIYR
VSNRFSGVPDRFSGSGSGTDFTLKISRVEAEDLGWYCFQGSHIPYTFGGGTKLE
IKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGE C

SEQ ID N° : 13

AM-VL1

DVVMTQSPLSLPVTLGQPASISCRSSQSIVYSNGNTYLNWFQQRPGQSPRRLIYR
VSNRDSGVPDRFSGSGSGTDFTLKISRVEAEDVGVYYCFQGSHIPYTFGQGTKL
EIKRTVAAPSVFIFPPSDEQLKSGTASVVCLLNNFYPREAKVQWKVDNALQSGN
SQESVTEQDSKDSTYSLSSTLTLSKADYEKH VYACEVTHQGLSSPVTKSFNRG
EC

SEQ ID N° : 14

AM-VL2-E40

DVVMTQSPLSLPVTLGQPASISCRSSQSIVYSNGNTYLEWFQQRPGQSPRRLIYR
VSNRDSGVPDRFSGSGSGTDFTLKISRVEAEDVGVYYCFQGSHIPYTFGQGTKL
EIKRTVAAPSV FIFPPSDEQLKSGTASVVCLLNNFYPREAKVQWVDNALQSGN
SQESVTEQDSKDSTYSLSSTLTLSKADYEKHKVYACEVTHQGLSSPVTKSFNRG

EC

**11.** L'anticorps anti-adrénomédulline ou fragment d'anticorps anti-ADM se liant à l'adrénomédulline ou échafaudage protéique non-Ig anti-ADM se liant à l'adrénomédulline selon une quelconque des revendications 1 à 10 et vasopresseur, par exemple, la catécholamine pour utilisation en tant que médicament.

**12.** L'anticorps anti-adrénomédulline ou fragment d'anticorps anti-ADM se liant à l'adrénomédulline ou échafaudage protéique non-Ig anti-ADM se liant à l'adrénomédulline selon une quelconque des revendications 1 à 10 et administration de fluide par voie intraveineuse pour utilisation en tant que médicament.

**13.** L'anticorps anti-adrénomédulline ou fragment d'anticorps anti-ADM se liant à l'adrénomédulline ou échafaudage protéique non-Ig anti-ADM se liant à l'adrénomédulline selon une quelconque des revendications 1 à 10 et anticorps TNF-alpha pour utilisation en tant que médicament.

**14.** L'anticorps anti-adrénomédulline ou fragment d'anticorps anti-ADM se liant à l'adrénomédulline ou échafaudage protéique non-Ig anti-ADM se liant à l'adrénomédulline selon une quelconque des revendications 1 à 11 et antibiotique pour utilisation en tant que médicament.

**15.** Composition pharmaceutique comprenant un anticorps anti-adrénomédulline ou un fragment d'anticorps anti-ADM se liant à l'adrénomédulline ou un échafaudage protéique non-Ig anti-ADM se liant à l'adrénomédulline pour utilisation en tant que médicament selon une quelconque des revendications 1 à 14.

**Fig. 1a:**

| Fv and scFV-variants |
|---|

dsFv-fragment     Fv-fragment

Disulfidbonds stabilized scFv-fragment     scFv-fragment     triabody

bivalent tandem scFv-fragment     bivalent diabody     tetrabody

bispecific tandem-scFv$_2$-fragment     bispecific diabody

bispecific *knob-into-hole* stabilized diabody     bispecific single chain diabody     bispecific disulfidbonds stabilized diabody     bispecific tandem-diabody

**Fig 1b:**

bivalent helix-
stabilized scFv-
dimer

bispecific leucine-zipper- sta-
bilized scFv-dimer

four-helix-bundel
stabilized scFv-
tetramer

**Heterologous
fusions**

bispecific four-helix-
bundel stabilized scFv-
tetramer

**Bifunctional
antibodies**

scFv-streptavidin-
fusion protein

Fab-fusion proteins
(e.g. to alkanine
phosphatase)

scFv-fusion proteins
(e.g. to RNase, GFP,
among others)

**Fig. 1c:**

**Fig. 2:**

SEQ ID NO: 21

**YRQSMNNFQGLRSFGCRFGTCTVQKLAHQIYQFTDKDKDNVAPRSKISPQGY-NH$_2$**

SEQ ID NO: 22

**YRQSMNQGSRSNGCRFGTCTFQKLAHQIYQLTDKDKDGMAPRNKISPQGY-NH$_2$**

SEQ ID NO: 23

**YRQSMNNFQGLRSFGCRFGTC**

SEQ ID NO: 24

**YRQSMNNFQGLRSFGCRFGTCTVQKLAHQIYQFTDKDKDNVA**

SEQ ID NO: 25

**PRSKISPQGY-NH2**

SEQ ID NO: 26

**YRQSMNNFQGLRSF**

SEQ ID NO: 27

**YRQSMNNFQG**

SEQ ID NO: 28

**YRQSMN**

SEQ ID NO: 29

**YRQSMNNFQGLRSFGCRFGTCTVQKLAHQIYQ**

SEQ ID NO: 30

**YRQSMNQGSRSNGCRFGTCTFQKLAHQIYQLTDKDKDGMA**

SEQ ID NO: 31

YRQSMNQGSRSNGCRFGTCTFQKLAHQIYQL

Fig. 3 a:

Fig. 3 b:

Fig. 3 c:

Fig. 3 d:

Fig. 3 e:

**Fig. 3 f:**

**Fig. 3 g:**

Fig. 3 h:

Fig. 3 i:

Fig. 3 j:

Fig. 3 k:

**Fig. 3 I:**

Fig. 4:

**Standard curve hADM**

**Fig. 5:**

Stability hADM

**Fig 6:**

**IGHV1-69*11:**
QVQLVQSGAEVKKPGSSVKVSCKASGGTFSSYAISWVRQAPGQGLEWMGRIIPILGT
ANYAQKFQGRVTITADESTSTAYMELSSLRSEDTAVYYCARYYYYYGMDVWGQGT
TVTVSS

**HB3:**
QVQLQQSGAELMKPGASVKISCKATGYTFSRYWIEWVKQRPGHGLEWIGEILPGSGS
TNYNEKFKGKATITADTSSNTAYMQLSSLTSEDSAVYYCTEGYEYDGFDYWGQGTT
LTVSS

**Alignment (ClustalW2):** Identical amino acids are illustrated by stars; points indicate conservative changes.

IGHV1:

```
QVQLVQSGAEVKKPGSSVKVSCKASGGTFSSYAISWVRQAPGQGLEWMGRIIPILGTANYAQKFQGR
VTITADESTSTAYMELSSLRSEDTAVYYCARYYYYYGMDVWGQGTTVTVSS
```

HB3:

```
QVQLQQSGAELMKPGASVKISCKATGYTFSRYWIEWVKQRPGHGLEWIGEILPGSGSTNYNEKFKGK
ATITADTSSNTAYMQLSSLTSEDSAVYYCTEGYEYDGFDYWGQGTTLTVSS
```

```
**** *****: ***:***:****:* *** * *.**:* **:****:*.*:*  *::** :**:*:
.***** *:.****:**** ***:*****:. * * *:* ******:****
```

**Fig. 7**

## Noradrenaline requirements

**Fig. 8**

## Urine production

**Fig. 9**

Fluid balance

**Fig. 10**

Liver
NF-kB

**Fig. 11:**

Vehicle N=8, 1.333mL/kg on Day 0, 0.666mL/kg on Day 2, 4, 6

NT-M N=8, 4mg/kg on Day 0, 2mg/kg on Day 2, 4, 6

**Fig. 12:**

Vehicle N=8, 1.333mL/kg on Day 0, 0.666mL/kg on Day 2, 4, 6

NT-M N=8, 4mg/kg on Day 0, 2mg/kg on Day 2, 4, 6

**Fig. 13:**

Fig. 14:

**Fig. 15:**

Kidney
KC

# p = 0.001

**Fig. 16:**

Liver
KC

# p = 0.001

**Fig. 17:**

Plasma
IL-6

# p = 0.017

**Fig. 18:**

Plasma
IL-10

# p = 0.002

**Fig. 19:**

Plasma
KC

# p = 0.004

**Fig. 20:**

Plasma
MCP-1

# p = 0.027

Fig. 21:

Plasma
TNF-α

# p = 0.005

**Fig. 22:**

Vehicle N=8, 1.333 ml/kg on day 0, 0.666 ml/kg on days 1,2

NT-M    N=8, 4 mg/kg on day 0, 2 mg/kg on days 1,2

**Fig. 23:**

Vehicle N=8, 1.333 ml/kg on day 0, 0.666 ml/kg on days 1,2

NT-M    N=8, 4 mg/kg on day 0, 2 mg/kg on days 1,2

**Fig. 24:**

Vehicle N=8, 1.333 ml/kg on day 0, 0.666 ml/kg on days 1,2

NT-M    N=8, 4 mg/kg on day 0, 2 mg/kg on days 1,2

## REFERENCES CITED IN THE DESCRIPTION

### Patent documents cited in the description

- EP 0622458 A2
- WO 2004090546 A
- WO 2004097423 A1
- WO 2006027147 A1
- WO 2007062676 A
- WO 2007062676 A1
- US 5807715 A
- US 5585089 A
- WO 9117271 A, Dower
- WO 92001047 A, McCafferty
- WO 9220791 A, Winter
- WO 9312227 A, Lonberg
- WO 9110741 A, Kucherlapati

- US 20100028995 A
- EP 1266025 A
- WO 2011154420 A
- WO 2011073214 A
- US 20040023334 A
- EP 2231860 A
- WO 2010060748 A
- EP 2314308 A
- WO 2011023685 A
- WO 2005040229 A
- EP 1941867 A
- EP 0353971 A

### Non-patent literature cited in the description

- **KITAMURA, K. et al.** Adrenomedullin: A Novel Hypotensive Peptide Isolated From Human Pheochromocytoma. *Biochemical and Biophysical Research Communications,* 1993, vol. 192 (2), 553-560
- **EDITORIAL, TAKAHASHI, K.** Adrenomedullin: from a pheochromocytoma to the eyes. *Peptides,* 2001, vol. 22, 1691
- **ETO, T.** A review of the biological properties and clinical implications of adrenomedullin and proadrenomedullin N-terminal 20 peptide (PAMP), hypotensive and vasodilating peptides. *Peptides,* 2001, vol. 22, 1693-1711
- **HINSON et al.** Adrenomedullin, a Multifunctional Regulatory Peptide. *Endocrine Reviews,* 2000, vol. 21 (2), 138-167
- **KITAMURA, K. et al.** The intermediate form of glycine-extended adrenomedullin is the major circulating molecular form in human plasma. *Biochem. Biophys. Res. Commun.,* 1998, vol. 244 (2), 551-555
- **PIO, R. et al.** Complement Factor H is a Serum-binding Protein for adrenomedullin, and the Resulting Complex Modulates the Bioactivities of Both Partners. *The Journal of Biological Chemistry,* 2001, vol. 276 (15), 12292-12300
- **HINSON et al.** Adrenomedullin, a Mul-tifunctional Regulatory Peptide. *Endocrine Reviews,* 2000, vol. 21 (2), 138-167
- **KUWASAKO, K. et al.** Purification and characterization of PAMP-12 (PAMP-20) in porcine adrenal medulla as a major endogenous biologically active peptide. *FEBS Lett,* 1997, vol. 414 (1), 105-110

- **KUWASAKI, K. et al.** Increased plasma proadrenomedullin N-terminal 20 peptide in patients with essential hypertension. *Ann. Clin. Biochem.,* 1999, vol. 36, 622-628
- **TSURUDA, T. et al.** Secretion of proadrenomedullin N-terminal20 peptide from cultured neonatal rat cardiac cells. *Life Sci.,* 2001, vol. 69 (2), 239-245
- **HIRATA et al.** Increased Circulating Adreno-medullin, a Novel Vasodilatory Peptide, in Sepsis. *Journal of Clinical Endocrinology and Metabolism,* 1996, vol. 81 (4), 1449-1453
- **EHLENZ, K. et al.** High levels of circulating adrenomedullin in severe illness: Correlation with C-reactive protein and evidence against the adrenal medulla as site of origin. *Exp Clin Endocrinol Diabetes,* 1997, vol. 105, 156-162
- **TOMODA, Y. et al.** Regulation of adrenomedullin secretion from cultured cells. *Peptides,* 2001, vol. 22, 1783-1794
- **UEDA, S. et al.** Increased Plasma Levels of Adrenomedullin in Patients with Systemic Inflammatory Response Syndrome. *Am. J. Respir. Crit. Care Med.,* 1999, vol. 160, 132-136
- **WANG, P.** Adrenomedullin and cardiovascular responses in sepsis. *Peptides,* 2001, vol. 22, 1835-1840
- **SCHÜTZ et al.** Circulating Precursor levels of endothelin-1 and adrenomedullin, two endothelium-derived, counteracting substances, in sepsis. *Endothelium,* 2007, vol. 14, 345-351

- **PIO et al.** Identification, characterization, and physiological actions of factor H as an Adrenomedullin binding Protein present in Human Plasma. *Microscopy Res. and Technique,* 2002, vol. 55, 23-27
- **MARTINEZ et al.** Mapping of the Adrenomedullin-Binding domains in Human Complement factor H. *Hypertens Res,* 2003, vol. 26, 56-59
- **LANZAVECCHIA et al.** *Eur. J. Immunol.,* 1987, vol. 17, 105
- **HUSTON et al.** *Proc. Natl. Acad. Sci. U.S.A.,* 1988, vol. 85, 5879-5883
- **BIRD et al.** *Science,* 1988, vol. 242, 423-426
- **HOOD et al.** Immunology. 1984
- **HUNKAPILLER ; HOOD.** *Nature,* 1986, vol. 323, 15-16
- **E. KABAT et al.** Sequences of Proteins of Immunological Interest. U.S. Department of Health and Human Services, 1983
- **LANE, R.D.** A short-duration polyethylene glycol fusion technique for increasing production of monoclonal antibody-secreting hybridomas. *J. Immunol. Meth.,* 1985, vol. 81, 223-228
- **ZIEGLER, B. et al.** Glutamate decarboxylase (GAD) is not detectable on the surface of rat islet cells examined by cytofluorometry and complement-dependent antibody-mediated cytotoxicity of monoclonal GAD antibodies. *Horm. Metab. Res.,* 1996, vol. 28, 11-15
- **HUST, M. ; MEYER, T. ; VOEDISCH, B ; RÜLKER, T. ; THIE, H. ; EL-GHEZAL, A. ; KIRSCH, M.I. ; SCHÜTTE, M. ; HELMSING, S ; MEIER, D.** A human scFv antibody generation pipeline for proteome research. *Journal of Biotechnology,* 2011, vol. 152, 159-170
- **SCHÜTTE, M. ; THULLIER, P ; PELAT, T. ; WEZLER, X. ; ROSENSTOCK, P. ; HINZ, D. ; KIRSCH, M.I. ; HASENBERG, M. ; FRANK, R. ; SCHIRRMANN, T.** Identification of a putative Crf splice variant and generation of recombinant antibodies for the specific detection of Aspergillus fumigatus. *PLoS One,* 2009, vol. 4, 6625
- **ALMAGRO JC ; FRANSSON J.** Humanization of antibodies. *Front Biosci.,* 01 January 2008, vol. 13, 1619-33
- **NISHIO et al.** Increased plasma concentrations of adrenomedullin correlate with relaxation of vascular tone in patients with septic shock. *Crit Care Med.,* 1997, vol. 25 (6), 953-7
- **MARX et al.** Monoclonal Antibody Production. *ATLA,* 1997, vol. 25, 121
- **HUST, M. ; MEYER, T. ; VOEDISCH, B. ; RÜLKER, T. ; THIE, H. ; EL-GHEZAL, A. ; KIRSCH, M.I. ; SCHÜTTE, M. ; HELMSING, S. ; MEIER, D.** A human scFv antibody generation pipeline for proteome research. *Journal of Biotechnology,* 2011, vol. 152, 159-170
- **SCHÜTTE, M. ; THULLIER, P. ; PELAT, T. ; WEZLER, X. ; ROSENSTOCK, P. ; HINZ, D. ; KIRSCH, M.I. ; HASENBERG, M. ; FRANK, R. ; SCHIRRMANN, T.** Identification of a putative Crf splice variant and generation of recombinant antibodies for the specific detection of Aspergillus fumigatus. *PLoS One,* 2009, vol. 4, 6625
- **LORENZ et al.** Functional Antibodies Targeting IsaA of Staphylococcus aureus Augment Host Immune Response and Open New Perspectives for Antibacterial Therapy. *Antimicrob Agents Chemother,* January 2011, vol. 55 (1), 165-173
- **COULTER, A. ; HARRIS, R.** *J. Immunol. Meth.,* 1983, vol. 59, 199-203
- **LINDNER I. et al.** alpha}2-Macroglobulin inhibits the malignant properties of astrocytoma cells by impeding {beta}-catenin signaling. *Cancer Res.,* 2010, vol. 70, 277-87
- **KAUFMANN B. et al.** Neutralization of West Nile virus by cross-linking of its surface proteins with Fab fragments of the human monoclonal antibody CR4354. *PNAS,* 2010, vol. 107, 18950-5
- **CHEN X. et al.** Requirement of open headpiece conformation for activation of leukocyte integrin $\alpha$x$\beta$2. *PNAS,* 2010, vol. 107, 14727-32
- **UYSAL H. et al.** Structure and pathogenicity of antibodies specific for citrullinated collagen type II in experimental arthritis. *J. Exp. Med.,* 2009, vol. 206, 449-62
- **THOMAS G. M. et al.** Cancer cell-derived microparticles bearing P-selectin glycoprotein ligand 1 accelerate thrombus formation in vivo. *J. Exp. Med.,* 2009, vol. 206, 1913-27
- **KONG F. et al.** Demonstration of catch bonds between an integrin and its ligand. *J. Cell Biol.,* 2009, vol. 185, 1275-84
- **MARIANI, M. et al.** A new enzymatic method to obtain high-yield F(ab')2 suitable for clinical use from mouse IgG1. *Mol.Immunol.,* 1991, vol. 28, 69-77
- **BEALE, D.** Molecular fragmentation: Some applications in immunology. *Exp Comp Immunol,* 1987, vol. 11, 287-96
- **ELLERSON, J.R. et al.** A fragment corresponding to the CH2 region of immunoglobulin G (IgG) with complement fixing activity. *FEBS Letters,* 1972, vol. 24 (3), 318-22
- **KERBEL, R.S. ; EL-LIOT, B.E.** Detection of Fc receptors. *Meth Enzymol,* 1983, vol. 93, 113-147
- **KULKARNI, P.N. et al.** Conjugation of methotrexate to IgG antibodies and their F(ab')2 fragments and the effect of conjugated methotrexate on tumor growth in vivo. *Cancer Immunol Immunotherapy,* 1985, vol. 19, 211-4
- **LAMOYI, E.** Preparation of F(ab')2 Fragments from mouse IgG of various subclasses. *Meth Enzymol,* 1986, vol. 121, 652-663

- **PARHAM, P. et al.** Monoclonal antibodies: purification, fragmentation and application to structural and functional studies of class I MHC antigens. *J Immunol Meth,* 1982, vol. 53, 133-73
- **RAYCHAUDHURI, G. et al.** Human IgG1 and its Fc fragment bind with different affinities to the Fc receptors on the human U937, HL-60 and ML-1 cell lines. *Mol Immunol,* 1985, vol. 22 (9), 1009-19
- **ROUSSEAUX, J. et al.** The differential enzyme sensitivity of rat immunoglobulin G subclasses to papain an pepsin. *Mol Immunol,* 1980, vol. 17, 469-82
- **ROUSSEAUX, J. et al.** Optimal condition for the preparation of Fab and F(ab')2 fragments from monoclonal IgG of different rat IgG subclasses. *J Immunol Meth,* 1983, vol. 64, 141-6
- **WILSON, K.M. et al.** Rapid whole blood assay for HIV-1 seropositivity using an Fab-peptide conjugate. *J Immunol Meth,* 1991, vol. 138, 111-9
- **JONES, P. T. ; DEAR, P. H ; FOOTE, J. ; NEUBERGER, M. S. ; WINTER, G.** Replacing the complementarity-determining regions in a human antibody with those from a mouse. *Nature,* 1986, vol. 321, 522-525
- **PADLAN, E. A.** A possible procedure for reducing the immunogenicity of antibody variable domains while preserving their ligand-binding properties. *Mol. Immunol.,* 1991, vol. 28, 489-498
- **HARRIS, L. ; BAJORATH, J.** Profiles for the analysis of immunoglobulin sequences: Comparison of V gene subgroups. *Protein Sci.,* 1995, vol. 4, 306-310
- **ALBUSZIES G et al.** Effect of increased cardiac output on hepatic and intestinal microcirculatory blood flow, oxygenation, and metabolism in hyperdynamic murine septic shock. *Crit Care Med,* 2005, vol. 33, 2332-8
- **ALBUSZIES G et al.** The effect of iNOS deletion on hepatic gluconeogenesis in hyperdynamic murine septic shock. *Intensive Care Med,* 2007, vol. 33, 1094-101
- **BARTH E et al.** Role of iNOS in the reduced responsiveness of the myocardium to catecholamines in a hyperdynamic, murine model of septic shock. *Crit Care Med,* 2006, vol. 34, 307-13
- **BAUMGART K et al.** Effect of SOD-1 over-expression on myocardial function during resuscitated murine septic shock. *Intensive Care Med,* 2009, vol. 35, 344-9
- **BAUMGART K et al.** Cardiac and metabolic effects of hypothermia and inhaled H2S in anesthetized and ventilated mice. *Crit Care Med,* 2010, vol. 38, 588-95
- **SIMKOVA V et al.** The effect of SOD-1 over-expression on hepatic gluconeogenesis and whole-body glucose oxidation during resuscitated, normotensive murine septic shock. *Shock,* vol. 30, 578-84

- **WAGNER F et al.** Effects of intravenous H2S after murine blunt chest trauma: a prospective, randomized controlled trial. *Crit Care,* 2011
- **SCHRIER ; WANG.** Mechanisms of Disease Acute Renal Failure and Sepsis. *The New England Journal of Medicine,* 2004, vol. 351, 159-69
- **JACOB.** Acute Renal Failure. *Indian J. Anaesth.,* 2003, vol. 47 (5), 367-372
- **WAGNER F ; WAGNER K ; WEBER S ; STAHL B ; KNÖFERL MW ; HUBER-LANG M ; SEITZ DH ; ASFAR P ; CALZIA E ; SENFTLEBEN U.** Inflammatory effects of hypothermia and inhaled H2S during resuscitated, hyperdynamic murine septic shock. *Shock,* 2011, vol. 35 (4), 396-402
- **WAGNER F ; SCHEUERLE A ; WEBER S ; STAHL B ; MCCOOK O ; KNÖFERL MW ; HUBER-LANG M ; SEITZ DH ; THOMAS J ; ASFAR P.** Cardiopulmonary, histologic, and inflammatory effects of intravenous Na2S after blunt chest trauma-induced lung contusion in mice. *J Trauma,* 2011, vol. 71 (6), 1659-67
- **CHIU PJS.** Models used to assess renal functions. *Drug Develop Res,* 1994, vol. 32, 247-255
- **WAGNER F ; WAGNER K ; WEBER S ; STAHL B ; KNÖFERL MW ; HUBER-LANG M ; SEITZ DH ; ASFAR P ; CALZIA E ; SENFTLEBEN U.** Inflammatory effects of hypothermia and inhaled H2S during resuscitated, hyperdynamic murine septic shock. *Shock,* 2011, vol. 35, 396-402
- **WAGNER F ; SCHEUERLE A ; WEBER S ; STAHL B ; MCCOOK O ; KNÖFERL MW ; HUBER-LANG M ; SEITZ DH ; THOMAS J ; ASFAR P.** Cardiopulmonary, histologic, and inflammatory effects of intravenous Na2S after blunt chest trauma-induced lung contusion in mice. *J Trauma,* 2011, vol. 71, 1659-1667
- **WAGNER F ; WAGNER K ; WEBER S ; STAHL B ; KNÖFERL MW ; HUBER-LANG M ; SEITZ DH ; ASFAR P ; CALZIA E ; SENFTLEBEN U.** Inflammatory effects of hypothermia and inhaled H2S during resuscitated, hyperdynamic murine septic shock. *Shock,* 2011, vol. 35, 396-402
- **WAGNER F ; SCHEUERLE A ; WEBER S ; STAHL B ; MCCOOK O ; KNÖFERL MW ; HUBER-LANG M ; SEITZ DH ; THOMAS J ; ASFAR P.** Cardiopulmonary, histologic, and inflammatory effects of intravenous Na2S after blunt chest trauma-induced lung contusion in mice. *J Trauma,* 2011, vol. 71, 1659-1667
- **NAKAMOTO M ; SHAPIRO JI ; SHANLEY PF ; CHAN L ; SCHRIER RW.** In vitro and in vivo protective effect of atriopeptin III on ischemic acute renal failure. *J ClinInvest,* 1987, vol. 80, 698-705
- **CHINTALA MS ; BERNARDINO V ; CHIU PJS.** Cyclic GMP but not cyclic AMP prevents renal platelet accumulation following ischemiareperfusion in anesthetized rats. *J PharmacolExpTher,* 1994, vol. 271, 1203-1208